# EUROPEAN PATENT SPECIFICATION

(11) **EP 1 257 544 B1**
(45) Date of publication and mention of the grant of the patent: **25.07.2012**
(21) Application number: 01912714.1
(22) Date of filing: 09.02.2001
(51) Int. Cl.: C07D 401/04, C07D 487/08, C07D 207/16, C07D 401/12, C07D 405/12, C07D 409/12, C07D 277/06, C07D 211/62, C07D 205/04, A61K 31/496, A61P 25/28

(54) **PIPERAZINE AND PIPERIDINE DERIVATIVES FOR TREATMENT OR PREVENTION OF NEURONAL DAMAGE**
PIPERAZIN- UND PIPERIDINVERBINDUNGEN ZUR BEHANDLUNG ODER PRÄVENTION NEURONALER SCHÄDEN
DERIVES DE PIPERAZINE ET DE PIPERIDINE POUR LE TRAITEMENT OU LA PREVENTION DE DOMMAGES NEUROLOGIQUES

(30) Priority: 11.02.2000 US 181944 P; 10.11.2000 US 247330 P
(43) Date of publication of application: 20.11.2002
(73) Proprietor: VERTEX PHARMACEUTICALS INCORPORATED, Cambridge, MA 02139-4242 (US)
(72) Inventor: TOMLINSON, Ronald, Marlborough, MA 01752 (US); LAUFFER, David, Stow, MA 01775 (US); MULLICAN, Michael, Needham, MA 02194 (US)
(74) Representative: Vossius & Partner
(86) International application number: PCT/US2001/004210
(87) International publication number: WO 2001/058891

(56) References cited:
- EP-A- 0 333 522
- US-A- 4 374 990
- US-A- 5 232 923
- US-A- 5 290 793
- US-A- 5 811 434

## Description

### TECHNICAL FIELD OF THE INVENTION

The present invention relates to piperazine and piperidine derivatives, which are especially useful for treating or preventing neuronal damage, particularly damage associated with neurological diseases. These compounds are also useful for stimulating nerve growth. The invention also provides compositions comprising the compounds of the present invention and methods of utilizing those compositions for treating or preventing neuronal damage or for stimulating nerve growth.

### BACKGROUND OF THE INVENTION

Neurological diseases are associated with the death of or injury to neuronal cells. Typical treatment of neurological diseases involves drugs capable of inhibiting neuronal cell death. A more recent approach involves the promotion of nerve regeneration by promoting neuronal growth.

Neuronal growth, which is critical for the survival of neurons, is stimulated *in vitro* by nerve growth factors (NGF). For example, Glial Cell Line-Derived Neurotrophic Factor (GDNF) demonstrates neurotrophic activity both, *in vivo* and *in vitro,* and is currently being investigated for the treatment of Parkinson's disease. Insulin and insulin-like growth factors have been shown to stimulate growth of neurites in rat pheochromocytoma PC12 cells and in cultured sympathetic and sensory neurons [Recio-Pinto et al., J. Neurosci., 6, pp. 1211-1219 (1986)]. Insulin and insulin-like growth factors also stimulate the regeneration of injured motor nerves *in vivo* and *in vitro* [Near et al., Proc. Natl. Acad. Sci., pp. 89, 11716-11720 (1992); and Edbladh et al., Brain Res., 641, pp. 76-82 (1994)]. Similarly, fibroblast growth factor (FGF) stimulates neural proliferation [D. Gospodarowicz et al., Cell Differ., 19, p. 1 (1986)] and growth [M. A. Walter et al., Lymphokine Cytokine Res., 12, p. 135 (1993)].

There are, however, several disadvantages associated with the use of nerve growth factors for treating neurological diseases. They do not readily cross the blood-brain barrier. They are unstable in plasma and they have poor drug delivery properties.

Recently, small molecules have been shown to stimulate neurite outgrowth *in vivo.* In individuals suffering from a neurological disease, this stimulation of neuronal growth protects neurons from further degeneration, and accelerates the regeneration of nerve cells. For example, estrogen has been shown to promote the growth of axons and dendrites, which are neurites sent out by nerve cells to communicate with each other in a developing or injured adult brain [(C. Dominique Toran-Allerand et al., J. Steroid Biochem. Mol. Biol., 56, pp. 169-78 (1996); and B. S. McEwen et al., Brain Res. Dev. Brain. Res., 87, pp. 91-95 (1995)]. The progress of Alzheimer's disease is slowed in women who take estrogen. Estrogen is hypothesized to complement NGF and other neurotrophins and thereby help neurons differentiate and survive.

Other target sites for the treatment of neurodegenerative disease are the immunophilin class of proteins. Immunophilins are a family of soluble proteins that mediate the actions of immunosuppressant drugs such as cyclosporin A, FK506 and rapamycin. Of particular interest is the 12 kDa immunophilin, FK-506 binding protein (FKBP12). FKBP12 binds FK-506 and rapamycin, leading to an inhibition of T-cell activation and proliferation. Interestingly, the mechanism of action of FK-506 and rapamycin are different. For a review, see, S. H. Solomon et al., Nature Med., 1, pp. 32-37 (1995). It has been reported that compounds with an affinity for FKBP12 that inhibit that protein's rotomase activity possess nerve growth stimulatory activity. [Lyons et al., Proc. Natl. Acad. Sci. USA, 91, pp. 3191-3195 (1994)]. Many of these such compounds also have immunosuppressive activity.

FK506 (Tacrolimus) has been demonstrated to act synergistically with NGF in stimulating neurite outgrowth in PC12 cells as well as sensory ganglia [Lyons et al. (1994)]. This compound has also been shown to be neuroprotective in focal cerebral ischemia [J. Sharkey and S. P. Butcher, Nature, 371, pp. 336-339 (1994)] and to increase the rate of axonal regeneration in injured sciatic nerves [B. Gold et al., J. Neurosci., 15, pp. 7509-16 (1995)].

The use of immunosuppressive compounds, however, has drawbacks in that prolonged treatment with these compounds can cause nephrotoxicity [Kopp et al., J. Am. Soc. Nephrol., 1, p. 162 (1991)], neurological deficits [P.C. DeGroen et al., N. Eng. J. Med., 317, p. 861 (1987)] and vascular hypertension [Kahan et al., N. Eng. J. Med., 321, p. 1725 (1989)].

Sub-classes of FKBP binding compounds which inhibit rotomase activity, but which purportedly lack immunosuppressive function have been disclosed for use in stimulating nerve growth and for neuroprotection [see, United States patent 5,614,547; WO 96/40633; WO 96/40140; WO 97/16190; WO 98/13343; WO 98/13355; WO 98/29116; WO 98/29117; WO 98/35675; WO 98/37882; WO 98/37885; J. P. Steiner et al., Proc. Natl. Acad. Sci. USA, 94, pp. 2019-23 (1997); and G. S. Hamilton et al., Bioorg. Med. Chem. Lett., 7, pp. 1785-90 (1997)].

Stimulation of neural axons in nerve cells by piperidine derivatives is described in WO 96/41609. Clinical use of the piperidine and pyrrolidine derivatives known so far for stimulating axonal growth has not been promising, as the compounds are unstable in plasma and do not pass the blood-brain barrier in adequate amounts.

More recently, classes of compounds which lack the ability to bind FKBP and lack immunosuppressive function have been described for use in stimulating nerve growth and preventing neurodegeneration [see, WO 98/20891; WO 98/20892; WO 98/20893 and WO 99/10340]

Though a wide variety of compounds for treating or preventing neurological degenerative diseases have been described, only two of these are currently in clinical trials and none have been approved for commercialization. And while compounds which share certain structural similarities to the compounds disclosed herein have been described in United States patent Nos. 5,102,882, 4,115,569 and 4,374,990, neither of those patents specifically teach or suggest the compounds of the present invention, nor is there any teaching that such compounds would have utility in stimulating nerve growth or preventing neurodegeneration.

Thus, there remains a need for the discovery and design of new compounds and compositions that have the ability to prevent and/or treat neuronal damage associated with neuropathologic conditions.

### SUMMARY OF THE INVENTION

The present invention discloses compounds having formula (I):
A compound of the formula: 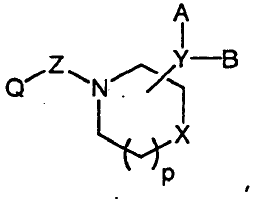 wherein:
   each Q is a monocyclic, bicyclic or tricyclic ring system wherein in said ring system:
      a. each ring is independently partially unsaturated or fully saturated;
      b. each ring comprises 3 to 7 ring atoms independently selected from C, N, O or S;
      c. no more than 4 ring atoms in Q are selected from N, O or S;
      d. any S is optionally replaced with S(O) or S(O)₂;
      e. at least one ring comprises a N ring atom that is substituted with R¹;
      f. one to five hydrogen atoms in Q are optionally and independently replaced with halo, -OH, =O, =N-OR¹, (C₁-C₆) -straight or branched alkyl, Ar-substituted- (C₁-C₆) -straight or branched alkyl, (C₂-C₆) - straight or branched alkenyl or alkynyl, Ar-substituted-(C₂-C₆) -straight or branched alkenyl or alkynyl, O-(C₁-C₆) - straight or branched alkyl, O-[(C₁-C₆)-straight or branched alkyl]-Ar, O-(C₂-C₆)-straight or branched alkenyl or alkynyl, O-[(C₂-C₆)-straight or branched alkenyl or alkynyl] -Ar, or O-Ar; and
      g. Q is not a pyroglutamic moiety, wherein
   each R¹ is independently selected from (C₁-C₁₀)-straight or branched alkyl, Ar-substituted-(C₁-C₁₀)-straight or branched alkyl, (C₂-C₁₀)-straight or branched alkenyl or alkynyl, or Ar-substituted-(C₂-C₁₀)-straight or branched alkenyl or alkynyl; wherein
   one to two CH₂ groups of said alkyl, alkenyl, or alkynyl chains in R¹ are optionally and independently replaced with O, S, S(O), S(O)₂, C(O) or N(R²), wherein when R¹ is bound to nitrogen, the CH₂ group of R¹ bound directly to said nitrogen cannot be replaced with C(O);
   Ar is selected from phenyl, 1-naphthyl, 2-naphthyl, 2-pyridyl, 3-pyridyl, 4-pyridyl, pyridazinyl, 2-pyrimidinyl, 4-pyrimidinyl, 5-pyrimidinyl, pyrazinyl, 1,3,5-triazinyl,
   each Ar is optionally and independently substituted with one to three substituents selected from halo, hydroxy, nitro, =O, -SO₃H, trifluoromethyl, trifluoromethoxy, (C₁-C₆)-straight or branched alkyl, (C₁-C₆)-straight or branched alkenyl, O-[(C₁-C₆)-straight or branched alkyl], O-[(C₁-C₆) -straight or branched alkenyl], O-benzyl, O-phenyl, 1,2-methylenedioxy, - N(R³) (R⁴), carboxyl, N- (C₁-C₆-straight or branched alkyl or C₂-C₆-straight or branched alkenyl) carboxamides, N,N-di-(C₁-C₆-straight or branched alkyl or C₂-C₆-straight or branched alkenyl) carboxamides, N-(C₁-C₆-straight or branched alkyl or C₂-C₆-straight or branched alkenyl) sulfonamides, or N,N-di-(C₁-C₆-straight or branched alkyl or C2-C6-straight or branched alkenyl) sulfonamides;
   each of R³ and R⁴ are independently selected from (C₁-C₆) -straight or branched alkyl, (C₂-C₆) -straight or branched alkenyl or alkynyl, hydrogen, phenyl or benzyl; or wherein R³ and R⁴ are taken together with the nitrogen atom to which they are bound to form a 5-7 membered heterocyclic ring;
   each R² is independently selected from hydrogen, (C₁-C₆) -straight or branched alkyl, or (C₂-C₆)-straight or branched alkenyl or alkynyl;
   X is selected from C(R²)₂, N(R²), H, O, S, S(O), or S(O)₂
   Y is selected from a bond, -O-, (C₁-C₆)-straight or branched) alkyl, or (C₂-C₆) -straight or branched) alkenyl or alkynyl; wherein Y is bonded to the depicted ring via a single bond or a double bond; and wherein one to two of the CH₂ groups of said alkyl, alkenyl, or alkynyl is optionally and independently replaced with O, S, S(O), S(O)₂, C(O) or N(R²);
   Z is -C(O)- or -CH₂-
   p is 0, 1 or 2;
   each of A and B is independently selected from hydrogen or Ar; or one of A or B is absent; and
   wherein two carbon ring atoms in the depicted ring structure are optionally linked to one another via a C₁-C₄ straight alkyl or a C₂-C₄ straight alkenyl to create a bicyclic moiety.

The compounds according to the present invention are as claimed in claim 1.

In another embodiment, the invention provides pharmaceutical compositions comprising the compounds of the present invention. These compositions may be utilized for promoting neuronal repair or preventing neuronal damage in a patient or in an *ex vivo* nerve cell. More particularly, the compositions and compounds of this invention are useful in treating various neurological diseases. Examples of such diseases include peripheral nerve destruction due to physical injury or diseases such as diabetes; physical injuries to the central nervous system (e.g., brain or spinal cord); stroke; neurological disturbances due to nerve degeneration, such as Parkinson's disease, Alzheimer's disease, and amylotrophic lateral sclerosis.

### DETAILED DESCRIPTION OF THE INVENTION

The present invention provides compounds having the formulae:
1. A compound selected from any one of compounds:

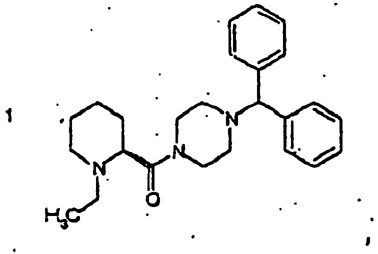

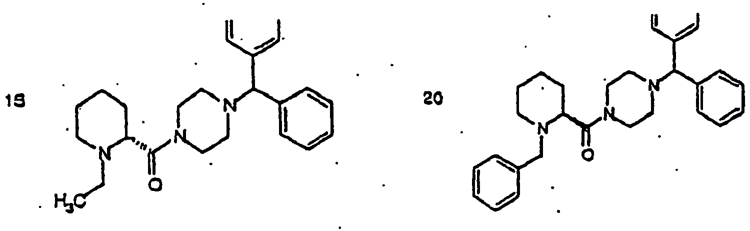

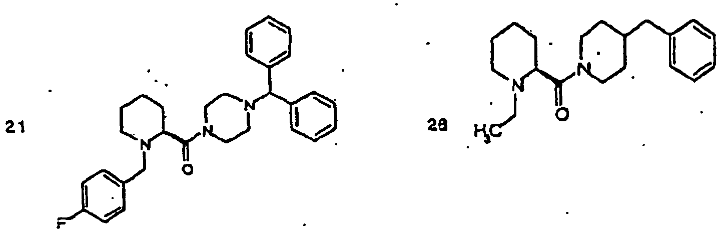

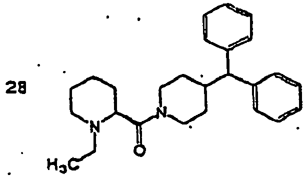

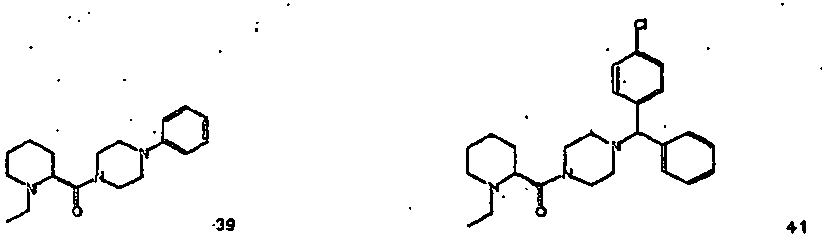

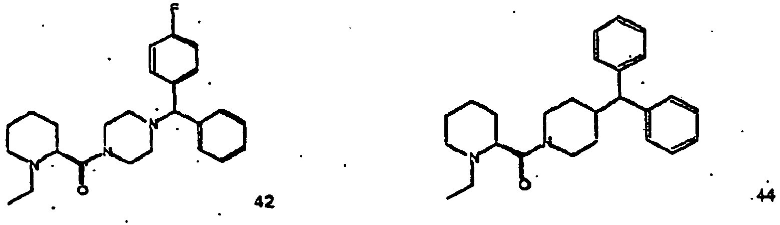

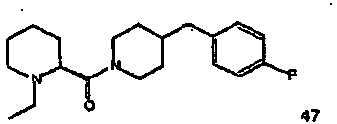

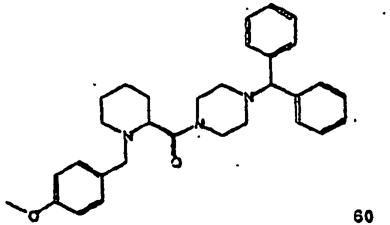

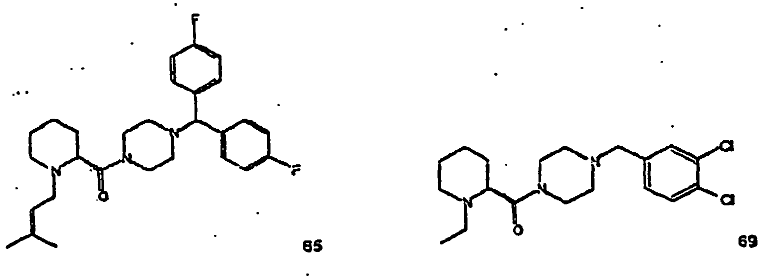

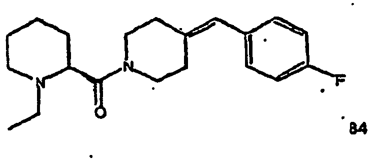

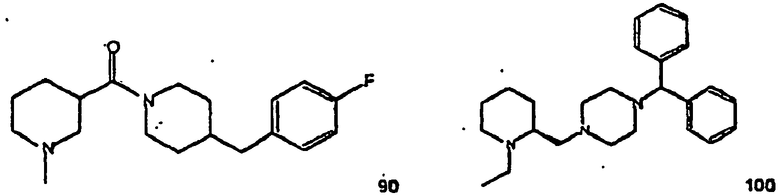

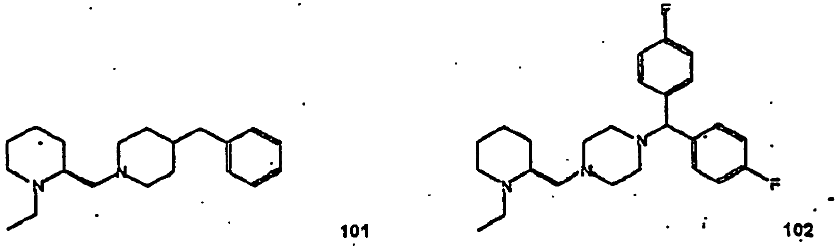

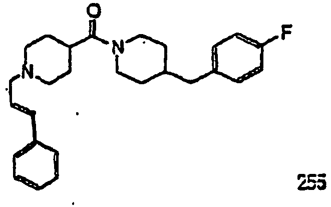

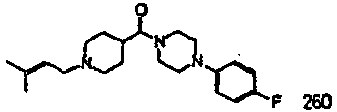

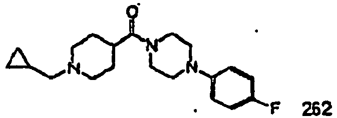

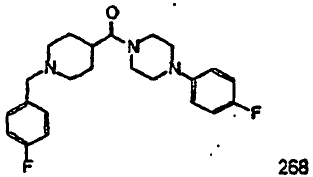

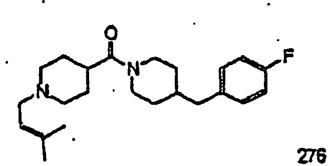

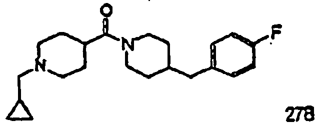

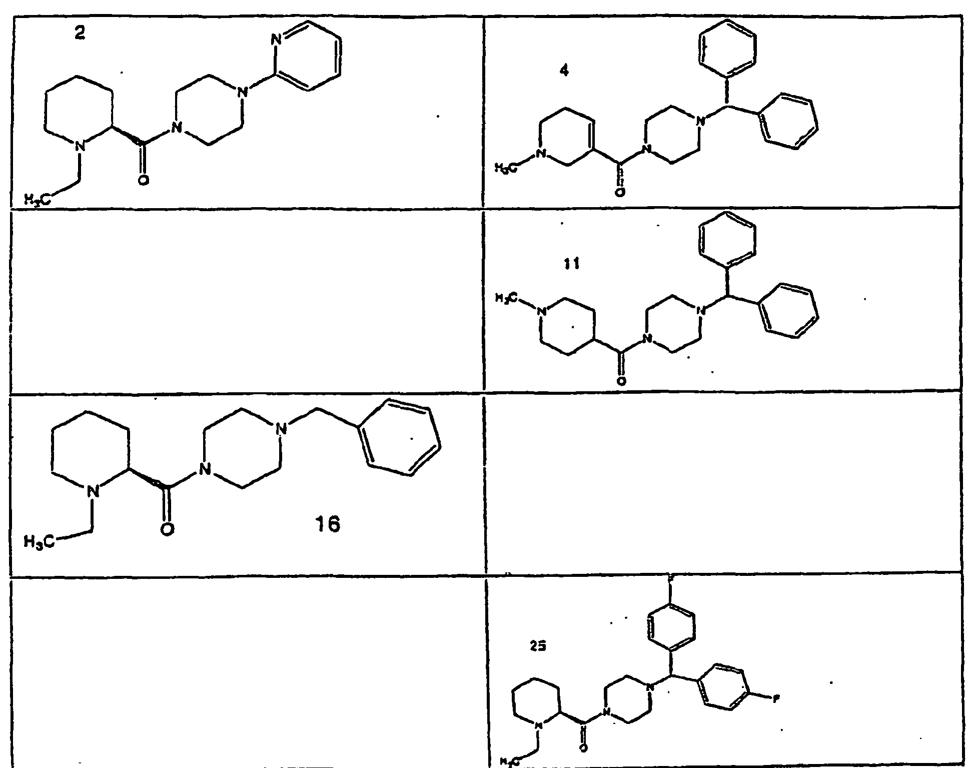

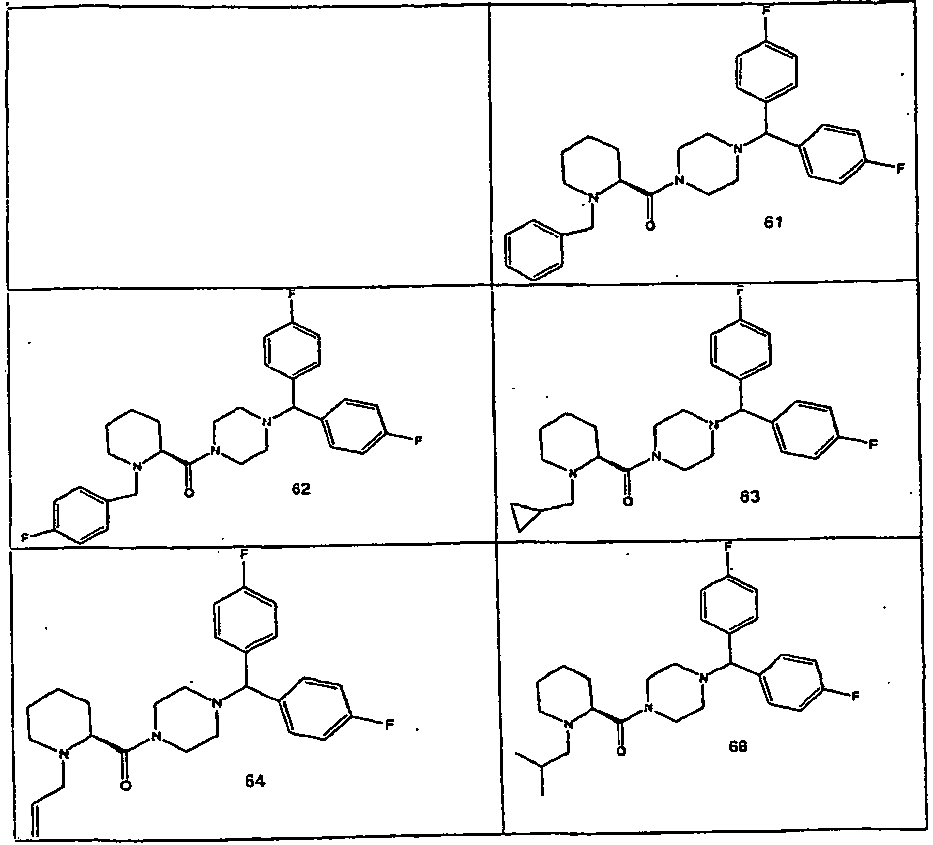

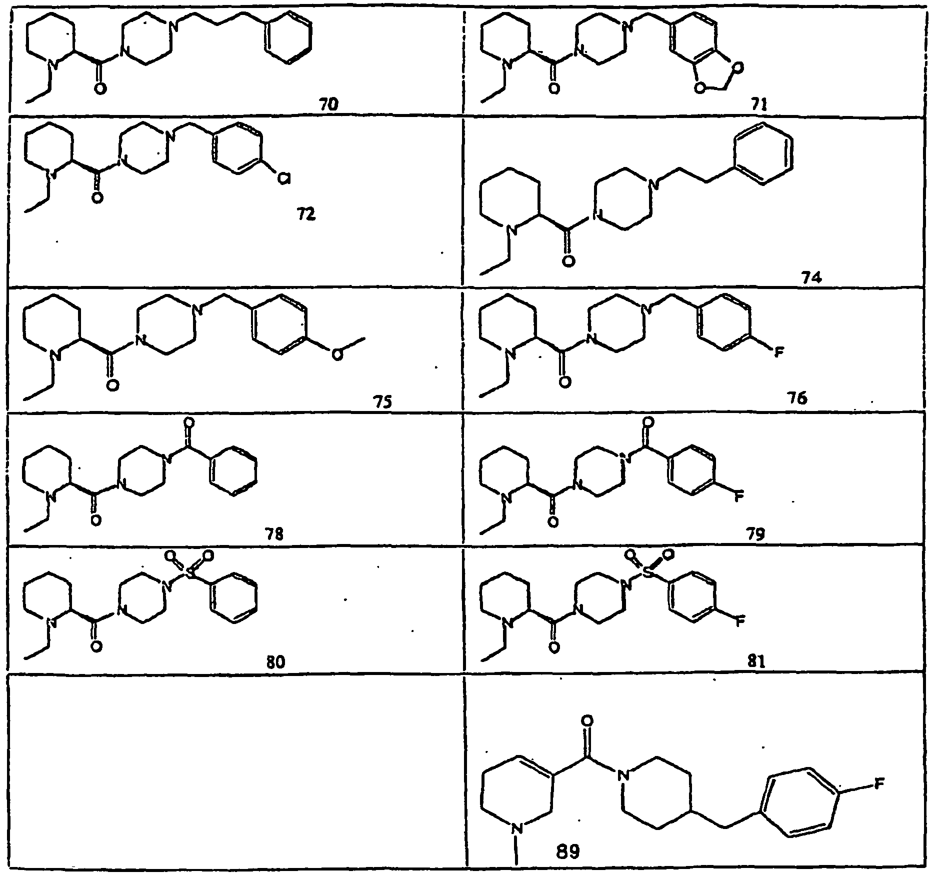

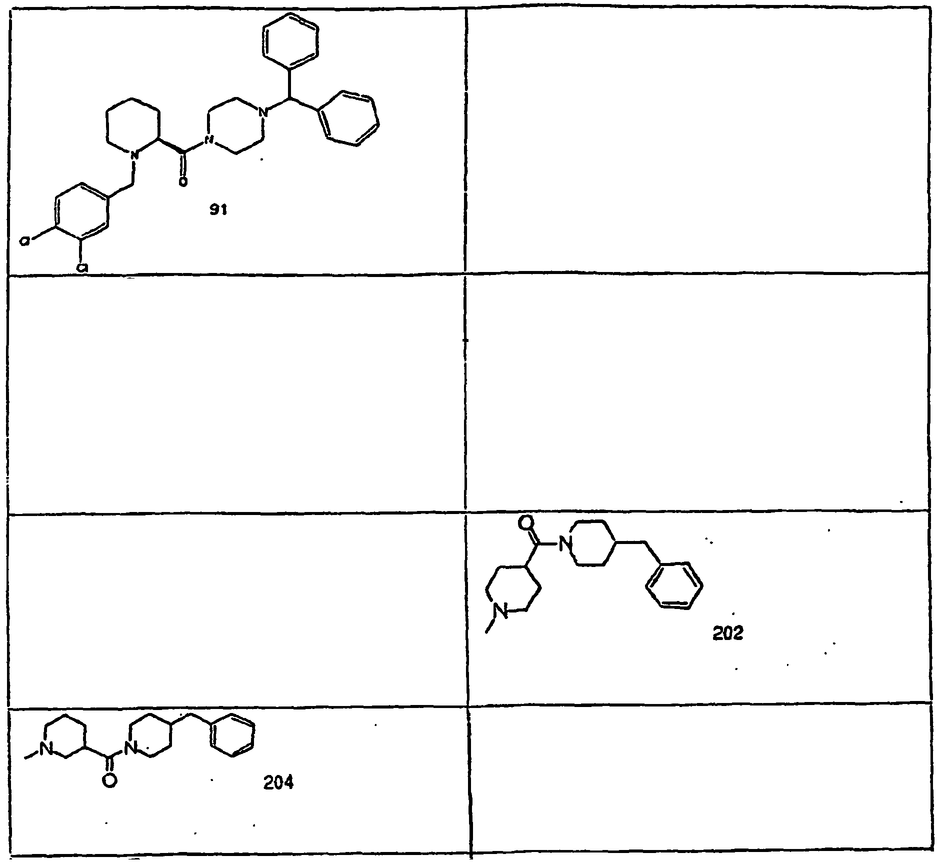

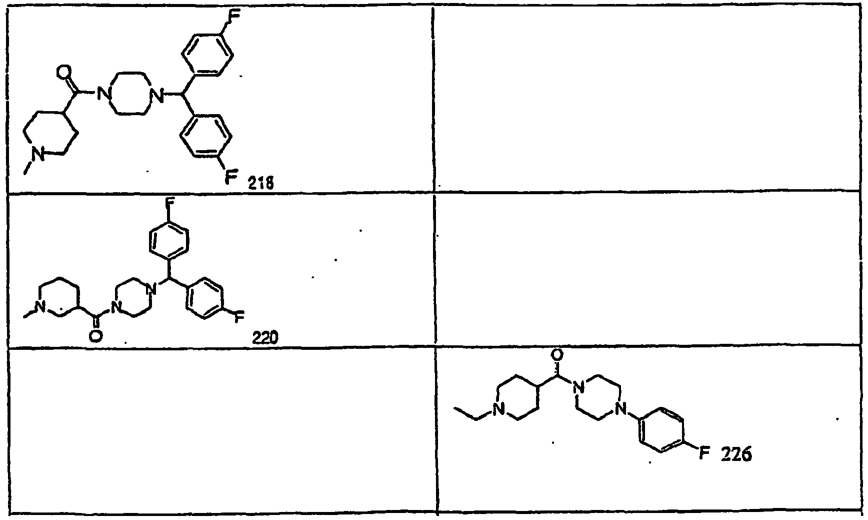

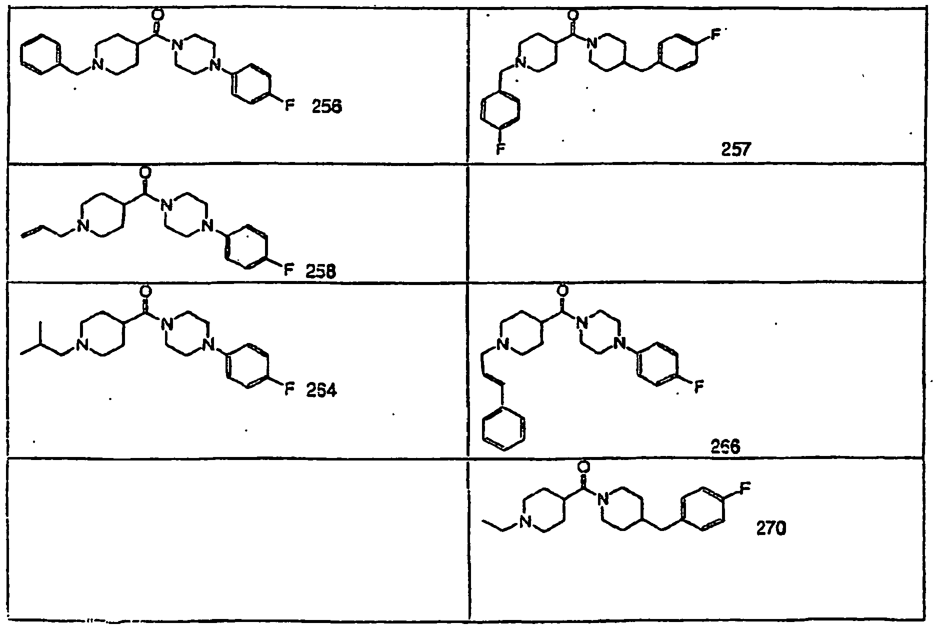

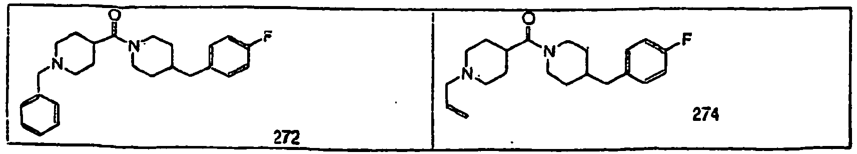

Compounds of formula (I) as disclosed above containing an indole at position Q are described in European Patent Publication 0 624.575 and in C. Kuehm-Caubere et al., J. Med. Chem., 40, pp. 1201-10 (1997), and are said to be inhibitors of low density lipoproteins (LDL) and membrane lipid oxidation. Compounds containing a pyroglutamide at Q are described in United States Patent 5,102,882 and are said to useful as nootropics.

Applicants believe that compounds in which Q is an indole or a pyroglutamide will possess nerve growth stimulatory activity and/or protect against neurodegeneration. Such activity is not disclosed or suggested in the prior art.

The compounds listed in Table 1 and Table 2, below and the compounds set forth in the Examples are within the scope of the present invention (unless stated otherwise).

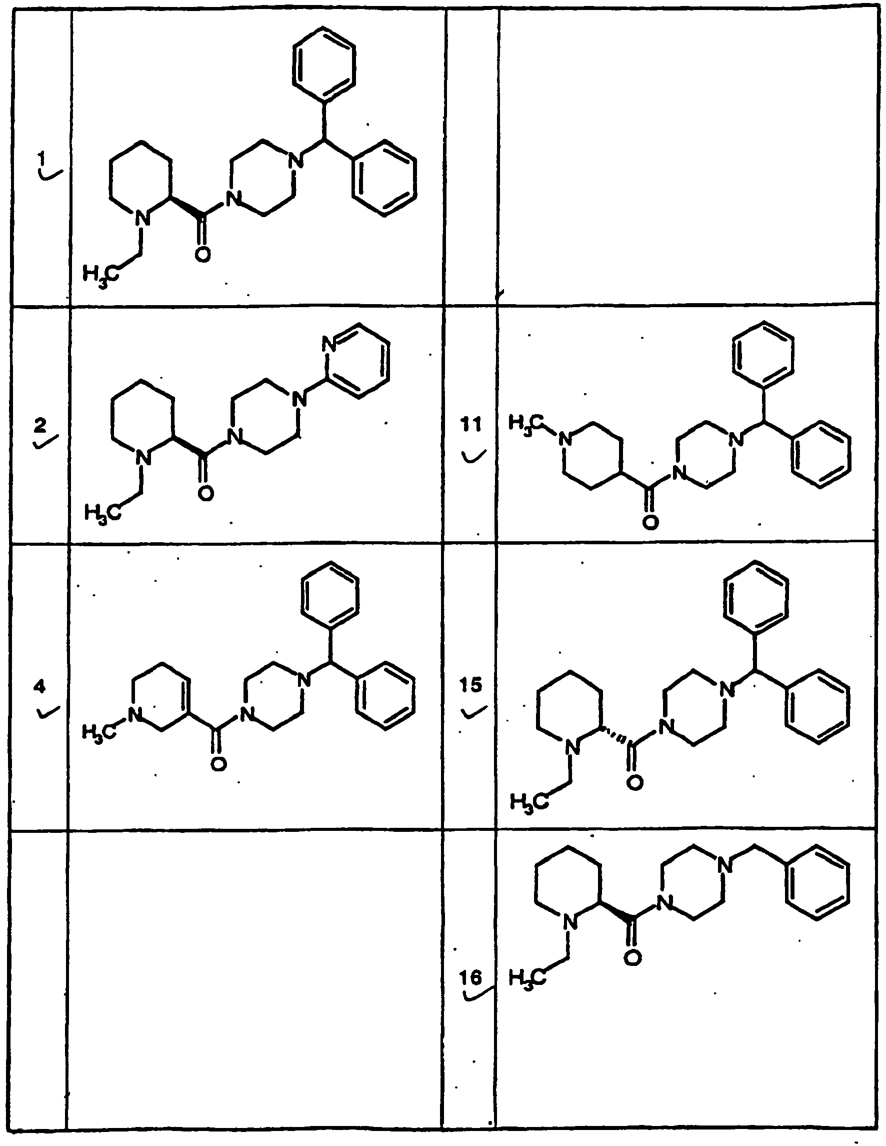

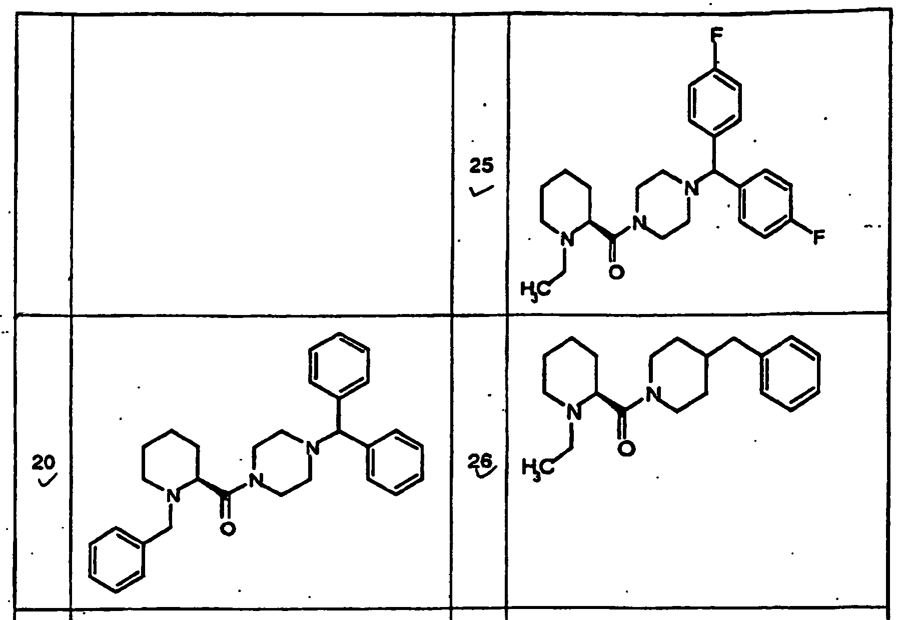

Preferred are compounds 1, 15, 20, 21, 26, 28, 39, 41, 42, 44, 47, 60, 65, 69, 84, 90, 100, 101, 102, 255, 260, 262, 268, 276, 278,

The compounds may be stereoisomers, geometric isomers or stable tautomers. The invention envisions all possible isomers, such as E and Z isomers, S and R enantiomers, diastereoisomers, racemates, and mixtures of those.

The compounds of the present invention may be readily prepared using known synthetic methods. For example, the compounds may be prepared as shown below in any of Schemes 1 through 7:

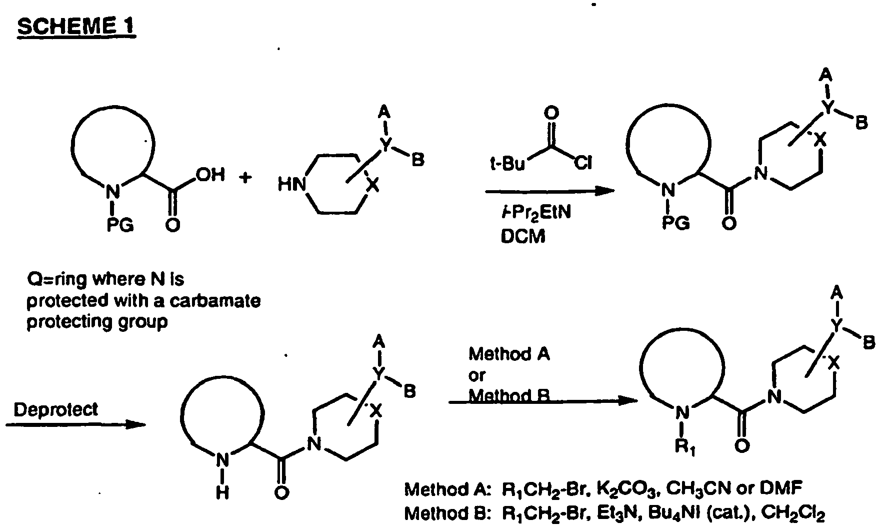

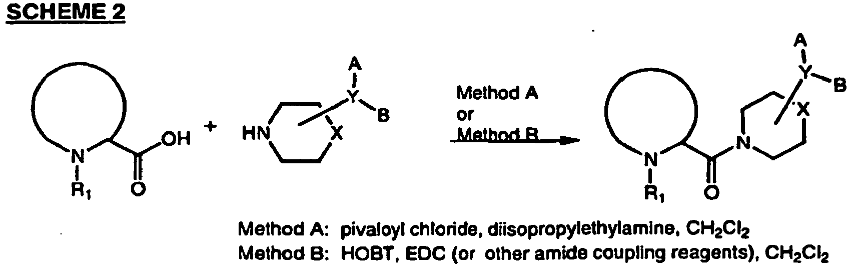

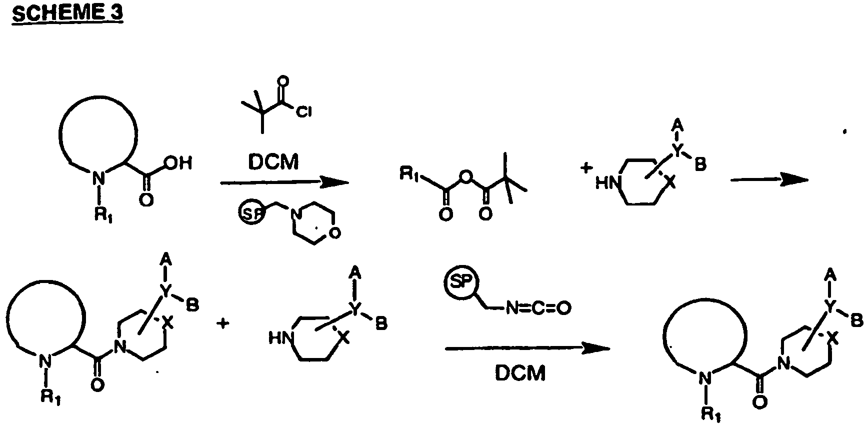

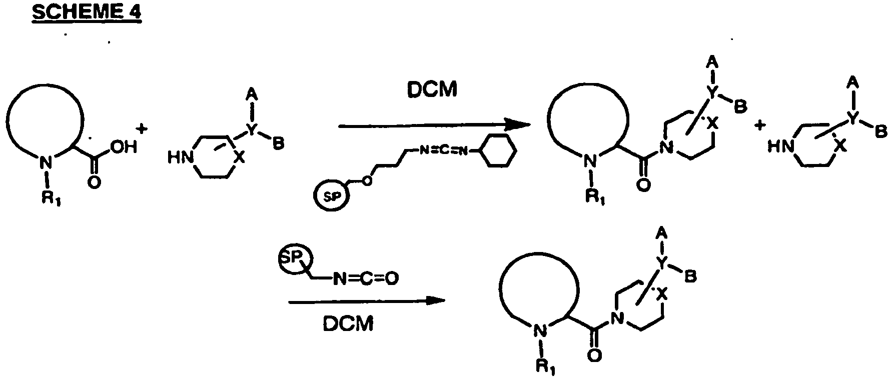

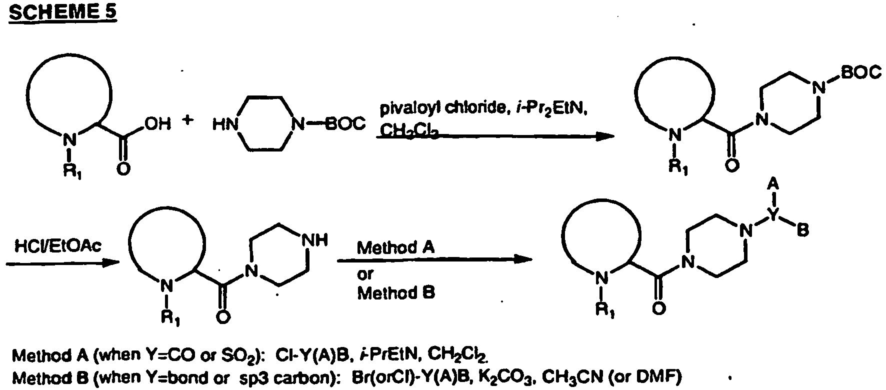

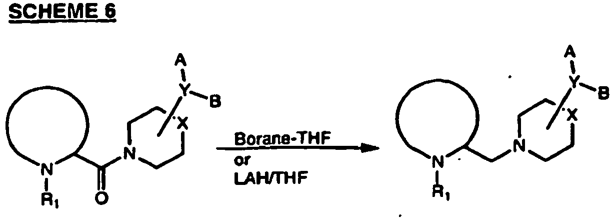

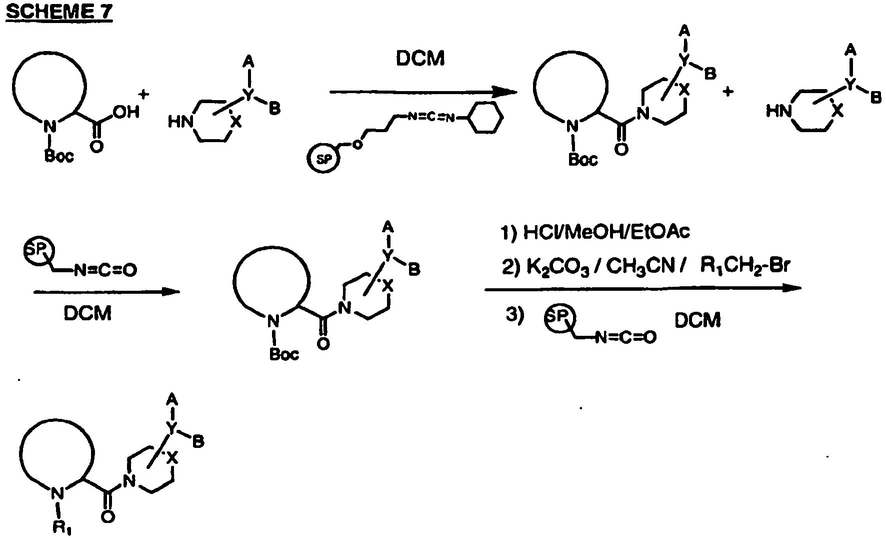

In the 7 schemes depicted above, the following abbreviation are used: tBu-C(O)-Cl = pivaloyl chloride; iPr₂EtN = diisopropylethylamine; DCM = dichloromethane; HCl = hydrogen chloride gas; EtOAc = ethyl acetate; Et₃N = triethylamine; DMF = dimethylformamide; THF = tetrahydrofuran; MeOH = methanol; Bu₄NI = tetrabutylammonium iodide; HOBT = N-hydroxybenzotriazole; EDC = 1-(3-Dimethylaminopropyl)-3-ethylcarbodiimide hydrochloride; LAH = Lithium aluminum hydride. Schemes 3, 4 and 7 are combinatorial chemistry type wherein reactants linked to a polystyrene solid support ("SP") are used.

Each of these schemes are described in more detail in the Example section.

One of skill in the art will be well aware of analogous synthetic methods for preparing compounds of formula (I).

The nerve growth stimulatory activity of the compounds of this invention may be initially assayed using several cell culture assays known in the art. For example, the compounds of this invention may be tested in a neurite outgrowth assay using pheochromocytoma PC12 cells as described by Lyons et al., PNAS, 91, pp. 3191-3195 (1994). A similar assay may be carried out in SH-SY5Y human neuroblastoma cells. Alternatively, the chick dorsal root ganglia assay described in United States patent 5,614,547 or in G. S. Hamilton et al., Bioorg. Med. Chem. Lett., (1997) and references cited therein, may be utilized.

The compounds of this invention may also be assayed for nerve growth stimulatory activity in *vivo* using a mouse model of Parkinson's disease [J. P. Steiner et al., Proc. Natl. Acad. Sci. USA, 94, pp. 2019-23 (1997), United States patent 5,721,256] or following surgical sciatic nerve crush in rats.

The neuroprotective activity of the compounds of this invention may be assayed using rat embryo ventral mesencephalic cells in culture which are subsequently exposed to the glutamate receptor agonist NMDA. This assay is described in detail in the example section.

According to another embodiment, this invention provides compositions comprising a compound of formula (I) and a pharmaceutically acceptable carrier.

Pharmaceutically acceptable carriers that may be used in these pharmaceutical compositions include, but are not limited to, ion exchangers, alumina, aluminum stearate, lecithin, serum proteins, such as human serum albumin, buffer substances such as phosphates, glycine, sorbic acid, potassium sorbate, partial glyceride mixtures of saturated vegetable fatty acids, water, salts or electrolytes, such as protamine sulfate, disodium hydrogen phosphate potassium hydrogen phosphate, sodium chloride, zinc salts, colloidal silica, magnesium trisilicate, polyvinyl pyrrolidone, cellulose-based substances, polyethylene glycol, sodium carboxy methylcellulose, polyacrylates, waxes, polyethylene-polyoxypropylene-block polymers, polyethylene glycol and wool fat.

In another embodiment, the pharmaceutical composition of the present invention is comprised of a compound the invention , a pharmaceutically acceptable carrier, and a neurotrophic factor.

The term "neurotrophic factor," as used herein, refers to compounds which are capable of stimulating growth or proliferation of nervous tissue. Numerous neurotrophic factors have been identified in the art and any of those factors may be utilized in the compositions of this invention. These neurotrophic factors include, but are not limited to, nerve growth factor (NGF), insulin-like growth factor (IGF-1) and its active truncated derivatives such as gIGF-1 and Des(1-3)IGF-I, acidic and basic fibroblast growth factor (aFGF and bFGF, respectively), platelet-derived growth factors (PDGF), brain-derived neurotrophic factor (BDNF), ciliary neurotrophic factors (CNTF), glial cell line-derived neurotrophic factor (GDNF), neurotrophin-3 (NT-3)and neurotrophin 4/5 (NT-4/5). The most preferred neurotrophic factor in the compositions of this invention is NGF.

As used herein, the described compounds used in the pharmaceutical compositions this invention, are defined to include pharmaceutically acceptable derivatives thereof. A "pharmaceutically acceptable derivative" denotes any pharmaceutically acceptable salt, ester, or salt of such ester, of a compound of this invention or any other compound which, upon administration to a patient, is capable of providing (directly or indirectly) a compound of this invention, or a metabolite or residue thereof, characterized by the ability to promote repair or prevent damage of neurons from disease or physical trauma.

If pharmaceutically acceptable salts of the described compounds are used, those salts are preferably derived from inorganic or organic acids and bases. Included among such acid salts are the following: acetate, adipate, alginate, aspartate, benzoate, benzenesulfonate, bisulfate, butyrate, citrate, camphorate, camphorsulfonate, cyclopentanepropionate, digluconate, dodecylsulfate, ethanesulfonate, fumarate, glucoheptanoate, glycerophosphate, hemisulfate, heptanoate, hexanoate hydrochloride, hydrobromide, hydroiodide, 2-hydroxyethanesulfonate, lactate, maleate, methanesulfonate, 2-naphthalenesulfonate, nicotinate, oxalate, palmoate, pectinate, persulfate, 3-phenyl-propionate, picrate, pivalate, propionate, succinate, tartrate, thiocyanate, tosylate and undecanoate. Base salts include ammonium salts, alkali metal salts, such as sodium and potassium salts, alkaline earth metal salts, such as calcium and magnesium salts, salts with organic bases, such as dicyclohexylamine salts, N-methyl-D-glucamine, and salts with amino acids such as arginine, lysine, and so forth. Also, the basic nitrogen-containing groups can be quaternized with such agents as lower alkyl halides, such as methyl, ethyl, propyl, and butyl chloride, bromides and iodides; dialkyl sulfates, such as dimethyl, diethyl, dibutyl and diamyl sulfates, long chain halides such as decyl, lauryl, myristyl and stearyl chlorides, bromides and iodides, aralkyl halides, such as benzyl and phenethyl bromides and others. Water or oil-soluble or dispersible products are thereby obtained.

The described compounds utilized in the compositions of this invention may also be modified by appending appropriate functionalities to enhance selective biological properties. Such modifications are known in the art and include those which increase biological penetration into a given biological system (e.g., blood, lymphatic system, central nervous system), increase oral availability, increase solubility to allow administration by injection, alter metabolism and alter rate of excretion.

The compositions of the present invention may be administered orally, parenterally, by inhalation spray, topically, rectally, nasally, buccally, vaginally or via an implanted reservoir. The term "parenteral" as used herein includes subcutaneous, intravenous, intramuscular, intra-articular, intra-synovial, intrasternal, intrathecal, intrahepatic, intralesional and intracranial injection or infusion techniques. Preferably, the compositions are administered orally, intraperitoneally or intravenously.

Sterile injectable forms of the compositions of this invention may be aqueous or oleaginous suspension. These suspensions may be formulated according to techniques known in the art using suitable dispersing or wetting agents and suspending agents. The sterile injectable preparation may also be a sterile injectable solution or suspension in a non-toxic parenterally acceptable diluent or solvent, for example as a solution in 1,3-butanediol. Among the acceptable vehicles and solvents that may be employed are water, Ringer's solution and isotonic sodium chloride solution. In addition, sterile, fixed oils are conventionally employed as a solvent or suspending medium. For this purpose, any bland fixed oil may be employed including synthetic mono- or di-glycerides. Fatty acids, such as oleic acid and its glyceride derivatives are useful in the preparation of injectables, as are natural pharmaceutically-acceptable oils, such as olive oil or castor oil, especially in their polyoxyethylated versions. These oil solutions or suspensions may also contain a long-chain alcohol diluent or dispersant, such as Ph. Helv or similar alcohol.

The pharmaceutical compositions of this invention may be orally administered in any orally acceptable dosage form including, but not limited to, capsules, tablets, aqueous suspensions or solutions. In the case of tablets for oral use, carriers which are commonly used include lactose and corn starch. Lubricating agents, such as magnesium stearate, are also typically added. For oral administration in a capsule form, useful diluents include lactose and dried corn starch. When aqueous suspensions are required for oral use, the active ingredient is combined with emulsifying and suspending agents. If desired, certain sweetening, flavoring or coloring agents may also be added.

Alternatively, the pharmaceutical compositions of this invention may be administered in the form of suppositories for rectal administration. These can be prepared by mixing the agent with a suitable non-irritating excipient which is solid at room temperature but liquid at rectal temperature and therefore will melt in the rectum to release the drug. Such materials include cocoa butter, beeswax and polyethylene glycols.

The pharmaceutical compositions of this invention may also be administered topically, especially when the target of treatment includes areas or organs readily accessible by topical application, including diseases of the eye, the skin, or the lower intestinal tract. Suitable topical formulations are readily prepared for each of these areas or organs.

Topical application for the lower intestinal tract can be effected in a rectal suppository formulation (see above) or in a suitable enema formulation. Topically-transdermal patches may also be used.

For topical applications, the pharmaceutical compositions may be formulated in a suitable ointment containing the active component suspended or dissolved in one or more carriers. Carriers for topical administration of the compounds of this invention include, but are not limited to, mineral oil, liquid petrolatum, white petrolatum, propylene glycol, polyoxyethylene, polyoxypropylene compound, emulsifying wax and water. Alternatively, the pharmaceutical compositions can be formulated in a suitable lotion or cream containing the active components suspended or dissolved in one or more pharmaceutically acceptable carriers. Suitable carriers include, but are not limited to, mineral oil, sorbitan monostearate, polysorbate 60, cetyl esters wax, cetearyl alcohol, 2-octyldodecanol, benzyl alcohol and water.

For ophthalmic use, the pharmaceutical compositions may be formulated as micronized suspensions in isotonic, pH adjusted sterile saline, or, preferably, as solutions in isotonic, pH adjusted sterile saline, either with or without a preservative such as benzylalkonium chloride. Alternatively, for ophthalmic uses, the pharmaceutical compositions may be formulated in an ointment such as petrolatum.

The pharmaceutical compositions of this invention may also be administered by nasal aerosol or inhalation. Such compositions are prepared according to techniques well-known in the art of pharmaceutical formulation and may be prepared as solutions in saline, employing benzyl alcohol or other suitable preservatives, absorption promoters to enhance bioavailability, fluorocarbons, and/or other conventional solubilizing or dispersing agents.

The amount of both a described compound and the optional neurotrophic factor that may be combined with the carrier materials to produce a single dosage form will vary depending upon the host treated and the particular mode of administration. Preferably, the compositions should be formulated so that a dosage of between 0.01 - 100 mg/kg body weight/day of the described compound can be administered. If a neurotrophic factor is present in the composition, then a dosage of between 0.01 µg - 100 mg/kg body weight/day of the neurotrophic factor can be administered to a patient receiving these compositions.

It should also be understood that a specific dosage and treatment regimen for any particular patient will depend upon a variety of factors, including the activity of the specific compound employed, the age, body weight, general health, sex, diet, time of administration, rate of excretion, drug combination, and the judgment of the treating physician and the severity of the particular disease being treated. The amount of active ingredients will also depend upon the particular described compound and neurotrophic factor in the composition.

According to another embodiment, this invention provides methods for promoting repair or preventing neuronal damage in an ex vivo nerve cell. Such methods comprise the step of treating nerve cells, glial cells, chromafin cells or stem cells with any of the compounds described above. When used to promote repair or prevent neuronal damage in a patient, the compound is formulated into a composition additionally comprising a pharmaceutically acceptable carrier. The amount of the compound utilized in these methods is between about 0.01 and 100 mg/kg body weight/day.

According to an alternate embodiment, the method of promoting repair or preventing neuronal damage comprises the additional step of treating nerve cells with a neurotrophic factor, such as those contained in the pharmaceutical compositions of this invention. This embodiment includes administering the compound and the neurotrophic agent in a single dosage form or in separate, multiple dosage forms. If separate dosage forms are utilized, they may be administered concurrently, consecutively or within less than about 5 hours of one another.

According to another embodiment, the compounds and compositions of this invention are used to stimulate axonal growth in nerve cells. The compounds are, therefore, suitable for treating or preventing neuronal damage caused by a wide variety of diseases or physical traumas. These include, but are not limited to, Alzheimer's disease, Parkinson's disease, ALS, Huntington's disease, Tourette's syndrome, multiple sclerosis, stroke and ischemia associated with stroke, neural paropathy, other neural degenerative diseases, motor neuron diseases, peripheral neuropathies including chemoneuropathies, sciatic injury, spinal cord or brain injuries, facial nerve damage, nerve damage associated with surgery or chemotherapy, retinopathy, macular degeneration, depression or schizophrenia.

The compounds and compositions of this invention used to stimulate axonal growth in nerve cells are also useful in increasing nerve graft survival and differentiation, increasing stem cell transplant survival and differentiation, and in increasing glial cell transplant survival and differentiation.

In a particularly preferred embodiment of the invention, the compounds and compositions are used to treat a patient suffering from trigeminal neuralgia, glosspharyngeal neuralgia, Bell's palsy, myasthenia gravis, muscular dystrophy, muscle injury, progressive muscular atrophy, progressive bulbar inherited muscular atrophy, herniated, ruptured, or prolapsed invertebrae disk syndrome's, cervical spondylosis, plexus disorders, thoracic outlet destruction syndromes, peripheral neuropathies, such as those caused by lead, dapsone, ticks, or porphyria, other peripheral myelin disorders, Alzheimer's disease, Gullain-Barre syndrome, Parkinson's disease and other Parkinsonian disorders, ALS, Tourette's syndrome, multiple sclerosis, other central myelin disorders, stroke and ischemia associated with stroke, neural paropathy, other neural degenerative diseases, motor neuron diseases, sciatic injury, neuropathy associated with diabetes, spinal cord injuries, facial nerve injury and other trauma, chemotherapy- and other medication-induced neuropathies, Huntington's disease, and protein fibrillization diseases, such as Diffuse Lewy Body disease, Alzheimer's disease-Lewy Body variant, Famillal British Dementia, and Frontotemporal Dementia.

More preferably, the compositions of the present invention are used for treating Parkinson's disease, amylotrophic lateral sclerosis, Alzheimer's disease, stroke, neuralgias, muscular atrophies, and Guillain-Barre syndrome.

For use of the compounds according to the invention as medications, they are administered in the form of a pharmaceutical preparation containing not only the active ingredient but also carriers, auxiliary substances, and/or additives suitable for enteric or parenteral administration. Administration can be oral or sublingual as a solid in the form of capsules or tablets, as a liquid in the form of solutions, suspensions, elixirs, aerosols or emulsions, or rectal in the form of suppositories, or in the form of solutions for injection which can be given subcutaneously, intramuscularly, or intravenously, or which can be given topically or intrathecally. Auxiliary substances for the desired medicinal formulation include the inert organic and inorganic carriers known to those skilled in the art, such as water, gelatin, gum arabic, lactose, starches, magnesium stearate, talc, vegetable oils, polyalkylene glycol, etc. The medicinal formulations may also contain preservatives, stabilizers, wetting agents, emulsifiers, or salts to change the osmotic pressure or as buffers.

Solutions or suspensions for injection are suitable for parenteral administration, and especially aqueous solutions of the active compounds in polyhydroxy-ethoxylated castor oil.

Surface-active auxiliary substances such as salts of gallic acid, animal or vegetable phospholipids, or mixtures of them, and liposomes or their components, can be used as carrier systems.

The neurotrophic effect of the compounds of formula (I) of the present invention and their physiologically acceptable salts can be determined using several cell culture assays known in the art or the assay described in Example 66. For example, the compounds of this invention may be tested in a neurite outgrowth using pheochromocytoma PC12 cells as described by W. E. Lyons et al., Proc. Natl. Acad. Sci. USA, 91, pp. 3191-3195 (1994). A similar assay may be carried out in SH-SY5Y human neuroblastoma cells. Alternatively, the chick dorsal root ganglia assay described in United States patent 5,614,547 or in G. S. Hamilton et al., Bioorg. Med. Chem. Lett., (1997) and references cited therein, may be utilized.

The compounds of this invention may also be assayed for nerve growth activity in vivo using a mouse model of Parkinson's disease [J. P. Steiner et al., Proc. Natl. Acad. Sci. USA, 94, pp. 2019-23 (1997)]

In order that this invention be more fully understood, the following examples are set forth. These examples are for the purpose of illustration only and are not to be construed as limiting the scope of the invention in any way.

### Reference Example 1

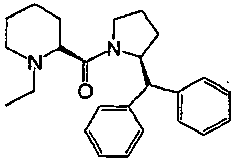

### 1-[(S)-2-(1,1-Diphenylmethyl)-pyrrolidin-1-yl]-1-((S)-1-ethyl-piperidin-2-yl)-methanone (Compound 27)

To a solution of 1-ethyl-(2S)-piperidine-2-carboxylic acid (158mg, 1.0mmols, 1.2eq.) in 5mL anhydrous DCM was added N,N-diisopropyl-ethylamine (585µL, 3.4mmols, 4.0 eq.) The reaction was stirred under N₂ for 10 min. then treated with pivaloyl chloride (124µL, 1.0mmols, 1.2eq.) drop-wise via syringe. The reaction was stirred 1.5h, then treated with a solution of (S)-2-(1,1-diphenyl-methyl)-pyrrolindine (199mg, 0.84mmols, 1.0 eq.) in 2mL anhydrous DCM drop-wise, and stirred at room temperature ("RT") for 96h. The reaction was diluted with 20mL DCM and washed with 20mL saturated NaHCO₃. The aqueous layer was extracted twice with 20mL DCM, then the combined organics were washed with water and brine, dried over sodium sulfate, filtered, and evaporated. The residue was purified via flash chromatography (98/2 dichloromethane/methanol) yielding 261mg product. The product was then dissolved in 20mL DCM and washed twice with saturated NaHCO₃. The basic layer was extracted once with 20mL DCM, and the combined organics were washed once with water and once with brine, dried over sodium sulfate, filtered, and evaporated in vacuo to afford 172 mg (58%) of the title compound. ¹H NMR (Bruker, 500MHz,CD₃OD): d 7.50-7.10 (m, 10H), 5.25 (m, 1H), 4.50-4.10 (dd, rotomers, 1H), 4.00-3.65 (m, 1H), 3.60-3.30 (m, 1H), 3.20-2.80 (m, 2H), 2.70-2.50 & 2.30-2.15 (m, rotomers, 1H); 2.10-1.20 (m, 12H). 1.10 & 0.90 (t, rotomers, 3H) ppm. MS (M+H) 377.

Using the procedure described in Example 1 the compounds set forth in Examples 2 through 8 were prepared:

### Example 2

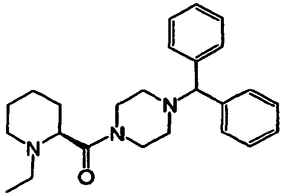

### 1-[4-(1,1-Diphenylmethyl)piperazin-1-yl]-1-((S)-1-ethylpiperidin-2-yl)methanone (Compound 1)

1H NMR (CDCl3, 500 MHz) δ 7.35 (m, 4H), 7.18 (m, 4H), 7.11 (m, 2H), 4.16 (s, 1H, Ph2CH), 3.99 (br s, 1H), 3.78 (br. s, 1H), 3.61 (br. s, 1H), 3.51 (br. s, 1H), 2.98 (m, 2H), 2.55 (m, 1H), 2.32-2.22 (m, 4H), 2.10 (m, 1H), 1.78 (m, 1H), 1.62-1.38 (m, 5H), 1.18 (m, 1H), 0.95 (t, 3H) ppm. MS (M+H): 392.5

### Example 3

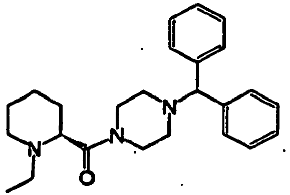

### 1-[4-(1,1-Diphenylmethyl)piperazin-1-yl]-1-((R)-1-ethylpiperidin-2-yl)methanone (Compounds 15)

1H NMR (CDC13, 500 MHz) δ 7.35 (m, 4H), 7.2 (m, 4H), 7.08 (m, 2H), 4.12 (s, 1H) , 3.98 (br. s, 1H), 3.78 (br. s, 1H), 3.59 (br. s, 1H), 3.51 (br. s, 1H), 2.98 (m, 2H, 2.58 (m, 1H), 2.35-2.25 (m, 4H), 2.10 (m, 1H), 1.81 (m, 1H), 1.65-1.40 (m, 5H), 1.14 (m, 1H), 0.95 (t, 3H) ppm. MS (M+H): 392.5

### Reference Example 4

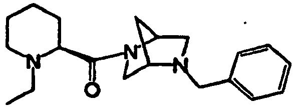

### 1-(5-benzyl-2,5-diaza-bicyclo[2.2.1]-hept-2-yl)-1-((S)-1-ethyl-piperidino-2-yl)-methanone (Compound 24)

82mg (33%) crystalline product. 1H NMR (Bruker 500MHz, CD₃OD): d 1.1 (m, 3H); 1.2-1.5 (m, 2H); 1.5-1.9 (m, 6H); 2.0-2.4 (m, 3H); 2.6-2.8 (m, 2H) 2.9 (m, 1H); 3.1-3.3 (m, 2H); 3.4-3.65 (m, 2H); 3.7-3.9 (m, 2H); 4.6-4.8. (dd, 1H); 7.2 (t, 1H) ; 7.3-7.4 (m, 4H) ppm. MS (M+H): 377

### Example 5

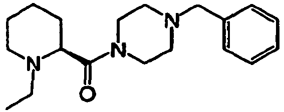

### 1-(4-Benzylpiperazin-1-yl)-1-((S)-1-ethylpiperidin-2-yl)methanone (Compound 16).

1H NMR (CDCl3, 500 MHz) δ 7.25 (m, 4H), 7.21 (m, 1H), 3.95 (br.s, 1H), 3.74 (br. s, 1H), 3.61 (br. s, 1H), 3.52 (br. s, 1H), 3.41 (s, 2H, PhCH₂), 3.04 (m, 2H), 2.58 (m, 2H), 2.36-2.28 (m, 4H), 2.12 (m, 1H), 1.80 (m, 1H), 1.71-1.42 (m, 4H), 1.18 (m, 1H), 0.95 (t, 3H) ppm. MS (M+H): 316.4

### Example 6

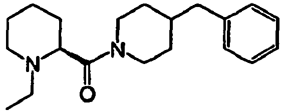

### 1-(4-Benzylpiperidin-1-yl)-1-((S)-1-ethylpiperidin-2-yl)methanone (Compound 26).

1H NMR (CDCl3, 500 MHz) δ 7.25-7.05 (m, 5H), 4.65 (br.s, 1H), 4.54 (d, 2H), 3.07 (m, 2H), 2.82 (m, 1H), 2.58-2.38 (m, 4H), 2.12 (m, 1H), 1.82 (m, 1H), 1.7-1.38 (m, 7H), 1.22 (m, 1H), 1.06 (m, 2H), 0.96 (t, 3H) ppm. MS (M+H): 315.4

### Example 7

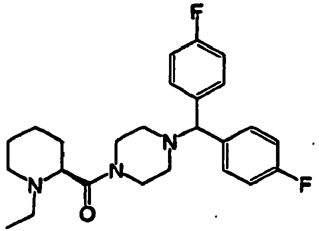

### 1-{4-[1,1-Bis-(4-fluorophenyl)methyl]-piperazin-1-yl}-1-((S)-1-ethylpiperidin-2-yl)methanone (Compound 25).

H NMR (CDCl3, 500 MHz)) δ 7.53 (m, 4H), 7.14 (m, 4H), 4.39 (s, 1H), 4.22 (br. s, 1H), 3.98 (br. s, 1H), 3.84 (br. s, 1H), 3.74 (br. s, 1H), 3.25 (m, 2H), 2.81 (m, 1H), 2.54 (m, 2H), 2.48 (m, 2H), 2.36 (m, 1H), 2.04 (m, 1H), 1.90 (m, 2H), 1.84-1.62 (m, 3H), 1.41 (m, 1H), 1.18 (t, 3H) ppm.

### Reference Example 8

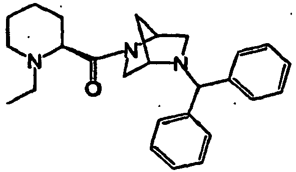

### 1-[(1S, 4S)-5-(1,1-Diphenylmethyl)-5-diazabicyclo[2.2.1]-hept-2-yl]-1-((S)-1-ethylpiperidin-2-yl)methanone (Compound 17).

1H NMR (CDCl3, 500 MHz) δ 7.38 (m, 4H), 7.18 (m, 4H), 7.12 (m, 2H), 4.76 (s, 0.5H), 4.52 (s, 1H), 4.43 (s, 0.5H), 3.65 (m, 1H), 3.38 (m, 1H), 3.22-2.98 (m, 2H), 2.85-2.46 (m, 3H), 2.33 (m, 1H), 2.08 (m, 1H), 1.92-1.10 (m, 9H), 1.02 & 0.97 (two t, 3H) ppm.

### Example 9

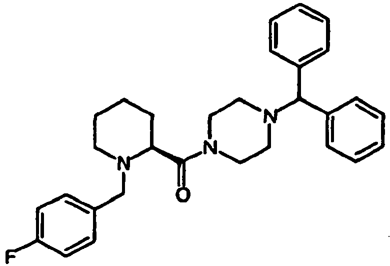

### 1-[4-(1,1-Diphenyl-methyl)-piperazin-1-yl]-1-[(S)-1-(4-fluoro-benzyl)-piperidin-2-yl]-methanone (Compound 21).

To a solution of 120 mg of 1-[4-(1,1-Diphenylmethyl)-piperazin-1-yl]-1-(S)-piperidin-2-yl-methanone dihydrochloride (0.28 mmol, 1 equiv.) in 10 mL of acetonitrile was added 300 mg of potassium carbonate (2.17 mmol, 8 equiv.) and 200 µL of 4-flourobenzyl bromide (1.6 mmol, 6 equiv.). The reaction was allowed to stir at 25 °C for 1 hr and then concentrated to a white solid which was extracted with dichloromethane and concentrated to a pale yellow oil. The crude product was purified by silica gel chromatography (20:1 methylene chloride:methanol, R_{f} = 0.2), yielding 56 mg (0.118 mmol, 42% yield) of 1-[4-(1,1-Diphenyl-methyl)-piperazin-1-yl]-1-[(S)-1-(4-fluoro-benzyl)-piperidin-2-yl]-methanone as a clear oil. ¹H NMR (CDCl₃, 500 MHz) d 7.35-7.05 (10 H, m, Ar), 6.90-6.75 (4 H, m, Ar), 4.05 (1 H, s, Ph₂CH), 3.7 (1 H, d, m, ArCH₂), 3.5 (1 H, br s), 3.1 (1H, m), 2.2 (4 H, br s), 1.5 (4 H, br s), 1.35 (3 H, br s), 1.1 (2 H, br s) ppm. MS: 472.44(M+H) found.

### Example 10

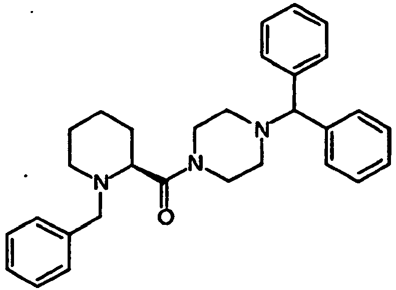

### 1-((S)-1-Benzyl-piperidin-2-yl)-1-[4-(1,1-diphenyl-methyl)-piperazin-1-yl]-methanone (Compound 20).

Compound 20 was prepared similarly to Compound 21, above, in Example 9.

¹H NMR (CDCl₃, 500 MHz) d 7.35-7.05 (15 H, m, Ar), 4.10 (1 H, s, PH₂CH), 3.8 (1 H, d, m, ArCH₂), 3.5 (3 H, br s), 3.1 (1H, m), 2.85 (1 H, br s), 2.2 (4 H, br s), 1.5 (4 H, br s), 1.35 (3 H, br s), 1.1 (2 H, br s) ppm. MS: 454.47 (M+H) found.

### Example 11

### Combinatorial Synthesis of Compounds Via Scheme 3

To N-ethylpipecolinic acid (0.157 g , 1.0 mmol) in 14 mL of dry CH₂Cl₂ was added pivaloyl chloride (0.121 g, 1.01 mmol) neat. After 1 hr, 1 mL of the resulting reaction solution was added to 14 wells of a reaction block containing morpholinomethyl polystyrene HL resin (100 mg, 0.4 mmol) and the appropriate amine derivative (0.2 mmol) in 2 mL of dry CH₂Cl₂. After shaking for 12 hrs, polystyrene methyl isocyanate (80 mg, 0.1 mmol) was added and the reaction solution was shaken an additional 12 hrs. Filtration and evaporation afforded the crude amide derivatives. Purification was accomplished with solid phase extraction (SPE-C) with methanol and methanol/ammonia to give the desired product.

Compounds 1 and 2 were synthesized in this manner.

### Example 12

### Combinatorial Synthesis of Compounds Via Scheme 4

To N-cyclohexanecarbodiimide-N'-propyloxymethyl polystyrene resin (150 mg, 0.15 mmol) in the wells of a reaction block was added the appropriate carboxylic acid derivative (0.075 mmol) neat. To each well was added 3 ml of 1-benzhydrylpiperazine (0.05 mmol) in dry CH₂Cl₂. After shaking for 12 hrs, polystyrene methyl isocyanate (80 mg, 0.1 mmol) was added and the reaction solution was shaken an additional 12 hrs. Filtration and evaporation afforded the crude amide derivatives. Purification was accomplished with reverse phase HPLC with H2O/acetonitril (0.1% TFA) to give the desired product as a trifluoroacetate salt.

Compounds 4, 7*, 8*, and 11 were synthesized in this manner. (*not encompassed in the present invention)

### Reference Example 13

### Synthesis of ((2S,4R)-1-Benzyl-4-hydroxypyrrolidin-2-yl)-(4-benzylpiperidin-1-yl)-methanone (Compound 30) Step A.

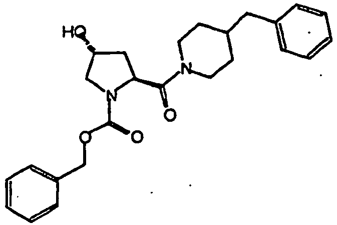

### (2S,4R)-2-(4-Benzylpiperidine-1-carbonyl)-4-hydroxypyrrolidine-1-carboxylic acid benzyl ester (Compound 32).

(2S,4R)-4-Hydroxy-pyrrolidine-1,2-dicarboxylic acid 1-benzyl ester (5.00g, 19 mmol) was dissolved in 50 mL anhydrous dichloromethane and 11 mL (63 mmol) of N,N-diisopropylethylamine. Pivaloyl chloride (2.32 mL, 19mmol) was added dropwise and the solution was stirred for 1 hour. Next, 4-benzylpiperidine (2.76 mL, 16 mmol) was added, and the solution was stirred for 16 hours. The reaction was diluted with dichloromethane, and then washed with saturated sodium bicarbonate, water, and brine. The organic layer was dried over sodium sulfate, filtered, and evaporated in vacuo to give a yellow oil that was purified by flash column chromatography (SiO₂) eluting with a gradient from ethyl acetate to dichloromethane to 2.0% methanol in dichloromethane. ¹H NMR (CDCl₃, 500MHz): δ 0.8-1.3(m, 2H); 1.3-1.9 (m, 4H); 2.0-2.15 (m, 2H); 2.2 (m, 1H); 2.3-2.5 (m, 1H); 2.6 (m, 2H); 2.7-3.1 (4t, 1H); 3.6 (d, 0.5H); 3.7 (m, 0.5H) ; 3.8 (m, 1.5H) ; 4.0 (t, 0.5H); 4.4-4.7 (m, 2H); 5.0-5.3 (m, 2H); 7.0-7.4 (m, 10H) ppm. MS: m/z 423 (M+1).

### Step B.

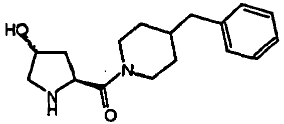

### (4-Benzylpiperidin-1-yl)-((2S,4R)-4-hydroxypyrrolidin-2-yl)-methanone (Compound 33) not encompassed in the present invention.

We dissolved (2S,4R)-2-(4-Benzyl-piperidine-1-carbonyl)-4-hydroxy-pyrrolidine-1-carboxylic acid benzyl ester (2.77g, 6.5 mmol) in 50 mL anhydrous EtOH, and degassed with N₂. Add Pd(OH)₂ (1.7 g, cat.) and stir under H₂ (1atm.). The reaction was filtered through Celite and evaporated to afford an orange foam (1.96g, 100%). ¹H NMR (CDCl₃, 500MHz): δ 0. 9-1.2 (m, 2H); 1.5-1.8 (m, 4H); 2.2 (m, 1H); 2.4 (m, 3H); 2.8 (q, 1H); 3.0 (dd, 1H) ; 3.1 (dd, 1H); 3.8 (d, 1H); 4.2 (t, 3H); 4.4 (d, 2H); 7.0 (d, 2H); 7.1 (t, 1H); 7.2 (m, 2H) ppm. MS: m/z 289 (M+1)

### Step C.

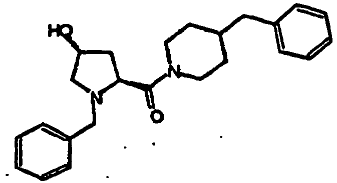

### ((2S,4R) -1-Benzyl-4-hydroxypyrrolidin-2-yl)- (4-benzylpiperidin-1-yl)-methanone (Compound 30) not encompassed in the present invention

(4-Benzylpiperidin-1-yl)-((2S,4R)-4-hydroxy-pyrrolidin-2-yl)-methanone (1.74g, 6.0mmol) was dissolved in 100mL acetonitrile. Potassium carbonate (3.34g, 24 mmol) was added to the solution followed by the addition of benzyl bromide (0.450 ml, 6.0 mmol). The mixture was stirred for 1 hour, filtered, and evaporated in vacuo to afford a viscous oil. The crude product was purified by flash chromatography (SiO₂) eluting with a gradient of EtOAc to 9:1 EtOAc/Methanol to give 1.13 g (50%) of the desired product. ¹H NMR (CDCl₃, 500 MHz): δ 1.0 (m, 2H) ; 1.45 (d, 0.5 H); 1.5-1.7 (broad d, 2.5 H) ; 1.7-2.0 (m, 2H); 2.0-2.1 (m, 0.5H); 2.1-2.2 (m, 0.5H); 2.3-2.6 (m, 4H); 2.6-2.8 (m, 1H); 3.4 (broad s, 1H); 3.5-3.7 (m, 1H); 3.8 (broad s, 2H) ; 3.9 (d, 1H) ; 4.4 (broad s, 1H) ; 4.5 (broad t, 1H) ; 7.0 (d, 2H); 7.1-7.3 (m, 8H) ppm. MS: *m*/*z* 379 (M+1).

### Reference Example 14

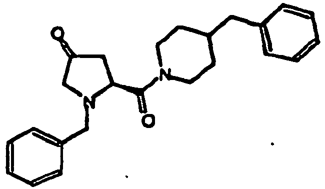

### (2S)-1-Benzyl-5-(4-benzylpiperidine-1-carbonyl)-pyrrolidin-3-one (Compound 29).

Oxalyl chloride (0.065 ml, 0.72 mmol) was added dropwise to a cooled (-78°C) solution of DMSO (0.10 ml, 1.37 mmol) in 10 mL of anhydrous dichloromethane. The mixture was stirred at -65°C for 2 hours. ((2S, 4R)-1-Benzyl-4-hydroxy-pyrrolidin-2-yl)-(4-benzyl-piperidin-1-yl)-methanone (140 mg, 0.37 mmol) in 5mL anhydrous dichloromethane was added to the solution dropwise. After stirring for 2.5 hours at -45°C, N,N-diisopropylethylamine (0.35 ml, 2 mmol) was added dropwise. The reaction was warmed to 0°C and diluted with dichloromethane. The reaction was washed with saturated sodium bicarbonate, water and brine. The organic layer was dried over sodium sulfate, filtered, and evaporated. The crude residue was purified by flash chromatography (SiO₂) using a gradient from dichloromethane to 2%MeOH in dichloromethane, yielding 101 mg (79%) of the desired product. ¹H NMR (CDCl₃, 500MHz) : δ 1.0 (m, 2H) ; 1.5-1.6 (m, 1H); 1.6-1.7 (m, 2H) ; 2.3-2.5 (m, 4H) ; 2.6 (d, 1H) ; 2.7-2.8 (m, 1H) ; 3.0 (d, 1H); 3.5 (t, 1H); 3.6-3.8 (m, 2H); 3.9 (br. s, 1H); 4.1 (br. s, 1H); 4.5 (br. s, 1H); 7.05 (t, 2H); 7.15 (m, 1H); 7.25 (m, 7 H) ppm. MS: m/z 377 (M+1).

### Reference Example 15

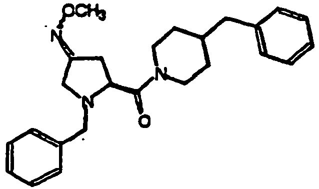

### (2S)-1-Benzyl-5-(4-benzylpiperidine-1-carbonyl) - pyrrolidin-3-one O-methyl-oxime (Compound 31).

(S)-1-Benzyl-5-(4-benzyl-piperidine-1-carbonyl)-pyrrolidin-3-one (Compound 29) (70 mg, 0.19 mmol) and methoxylamine hydrochloride (20 mg, 0.25 mmol) were taken into 5mL anhydrous methanol and heated to 40°C for 2 hours. The reaction was evaporated and then partitioned between dichloromethane and saturated sodium bicarbonate. The aqueous layer was extracted with dichloromethane and the combined organic extracts were washed with brine, dried over sodium sulfate, filtered, and evaporated. The crude residue was purified by flash chromatography (SiO₂) using a gradient from 0%-2% methanol in dichloromethane to yield 25 mg (33%) of the desired product. ¹H NMR (CDCl₃, 500MHz) : δ 0.8-1.2 (m, 3H) ; 1.4-1.8 (m, 3H); 2.3-2.5 (m, 3H); 2.5-2.7 (m, 1H); 2.7-2.9 (m, 2H); 3.1-3.3 (m, 1H); 3.45 (t, 0.5H); 3.6 (d, 0. 5H) ; 3.6-3.8(m, 4H); 3.8-4.0 (m, 2H); 4.5 (br. s, 1H); 7.0 (d, 2H); 7.15 (m, 1H); 7.2-7.4 (m, 7H) ppm. MS: m/z 406 (M+1).

### Example 16

The compounds described in Examples 16-32 were prepared by the procedure described in Example 1 (Scheme 2).

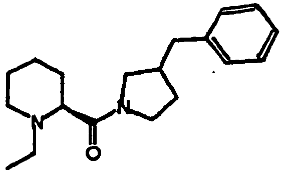

### (3-Benzylpyrrolidin-1-yl)-((2S)-1-ethylpiperidin-2-yl)-methanone (Compound 35) not encompassed in the present invention

Compound 35 was prepared from (2S)-1-ethylpiperidin-2-yl carboxylic acid and 3-benzylpyrrolidine an described in Example 1 to yield 62 mg (17%). ¹H NMR (CDCl₃, 500 MHz): δ 1.35 (m, 3H); 1.4-1.7 (m, 3H) ; 1.8 (m, 2H); 2.0-2.2 (m, 2H); 2.3-2.7 (m, 4H); 2.9-3.25 (m, 4H); 3.25-3.7 (m, 3H) ; 4.0 (bs, 1H); 4.1-4.2 (m, 1H); 7.1 (m, 2H); 7.1-7.3 (m, 3H) ppm.

### Reference Example 17

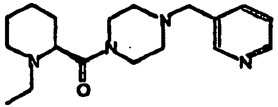

### ((2S)-1-Ethylpiperidin-2-yl)-(4-pyridin-3-ylmethylpiperazin-1-yl)-methanone trihydrochloride (Compound 36).

Compound **36** was prepared from (2S)-1-ethylpiperidin-2-yl carboxylic acid and 3-pyridinylmethylpiperazine as described in Example 1 to afford 229 mg (72%) as the trihydrochloride salt. ¹H NMR (CDCl₃, 500MHz): δ1.0 (t, 3H); 1.2 (m, 1H); 1.4-1.8 (m, 5H); 1.85 (bs, 1H); 2.15 (bs, 1H); 2.15 (bs, 1H); 2.4 (m, 4H); 2.6 (bs, 1H); 3.1 (bs, 2H); 3.4 (s, 2H); 3.5 (bs, 1H); 3.6 (s, 1H); 3.8 (bs, 1H) ; 4.0 (bs, 1H) ; 7.2 (d, 1H); 7.6 (d, 1H); 8.5 (m, 2H) ppm. MS: m/z 317 (M+1).

### Reference Example 18

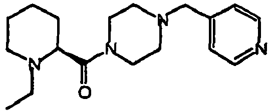

### ((2S)-1-Ethylpiperidin-2-yl)-(4-pyridin-4-ylmethylpiperazin-1-yl)-methanone trihydrochloride (Compound 37).

Compound **37** was prepared from (2S)-1-ethylpiperidin-2-yl carboxylic acid and 4-pyridinylmethylpiperazine as described in Example 1 to yield 236 mg (75%) as the trihydrochloride salt. **¹H** NMR (CDCl3, 500MHz) : δ 1.0 (t, 3H); 1.2 (m, 1H); 1.4-1.8 (m, 5H) ; 1.85 (bs, 1H); 2.15 (bs, 1H); 2.15 (bs, 1H); 2.4 (m, 4H) ; 2.6 (bs, 1H) ; 3.1 (bs, 2H) ; 3.4 (s, 2H) ; 3.5 (bs, 1H); 3.6 (s, 1H); 3.8 (bs, 1H); 4.0 (bs, 1H); 7.2 (d, 2H) ; 8.5 (d, 2H) ppm. MS: *m*/*z* 317 (M+1).

### Reference Example 19

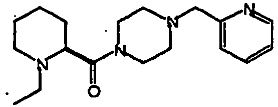

### ((2S)-1-Ethylpiperidin-2-yl)-(4-pyridin-2-ylmethylpiperazin-1-yl)-methanone trihydrochloride (Compound 38).

Compound **38** was prepared from (2S)-1-ethylpiperidin-2-yl carboxylic acid and 2-pyridinylmethylpiperazine as described in Example 1 to yield 42 mg (13%) as the trihydrochloride salt. ¹H NMR (CDCl₃, 500MHz) : δ 1.0 (t, 3H) ; 1.2 (s, 3H); 1.4-1.8 (m, 2H); 1.85 (m, 1H); 2,1 (m, 1H) ; 2.4 (m, 4H); 2.6 (bs, 1H) ; 3.0 (bs, 2H); 3.6 (s, 5H) ; 3.8 (bs, 1H) ; 4.0 (bs, 1H) 7.1 (t, 1H) ; 7.3 (d, 1H) ; 7.6 (t, 1H) ; 8.5 (d, 1H) ppm. MS: *m*/*z* 317 (M+1).

### Example 20

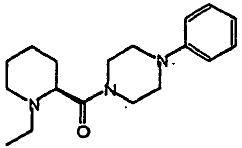

### ((2S)-1-Ethylpiperidin-2-yl)-(4-phenylpiperazin-1-yl)-methanone dihydrochloride (Compound 39).

compound **39** was prepared from (2S)-1-ethylpiperidin-2-yl carboxylic acid and N-phenylpiperazine as described in Example 1 to yield 277 - mg (74%) as the dihydrochloride salt. ¹H NMR (CDCl₃, 500 MHz) : δ 1.3 (t, 3H); 1.6 (m, 1H) ; 1.7 (q, 2H) ; 1.9 (m, 2H); 2.1 (d, 1H) ; 3.0 (2H); 3.2 (m, 1H) ; 3.5 (m, 4H) ; 3.7 (d, 1H); 3.9 (m, 4H); 4.4 (m, 1H) ; 7.3 (m, 3H).; 7.5 (m, 2H). MS: *m*/*z* 406 (M+1) ppm.

### Reference Example 21

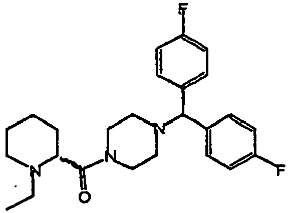

### {4-[Bis-(4-fluorophenyl)methyl]-piperazin-1-yl}-((2R)-1-ethylpiperidin-2-yl)-methanone (Compound 40).

Compound **40** was prepared from (2R)-1-ethylpiperidin-2-yl carboxylic acid and N-Bis-(4-fluorophenyl)methylpiperazine as described in Example 1 to yield 590 mg (46% yield) after chromatography. ¹H NMR (500 MHz, CDCl₃), δ 7.40-7.35 (m, 4H), 7.05-6.95 (m, 4H), 4.20 (s, 1H), 4.05-3.50 (m, 4H), 3.10-3.00 (m, 2H), 2.40-2.25 (m, 4H), 1.85-1.40 (m, 8H), 1.35-1.00 (m, 4H) ppm. MS: *m*/*z* 428.5 (M+1).

### Example 22

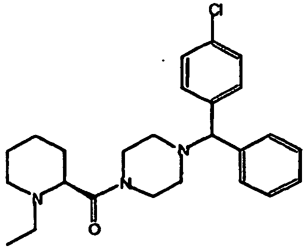

### {4-[(4-Chlorophenyl)phenylmethyl]-piperazin-1-yl}-((2S)-1-ethylpiperidin-2-yl)-methanone dihydrochloride (Compound 41).

Compound **41** was prepared from (2S)-1-ethylpiperidin-2-yl carboxylic acid and N-(4-chlorophenyl) phenylmethylpiperazine as described in Example 1 to yield 170 mg (67%) as the dihydrochloride salt. ¹H NMR (CDCl₃, 500 MHz) δ 7.30 (m, 4H), 7.18 (m, 4H), 7.12 (m, 1H), 4.14 (s, 1H), 3.98 (m, 1H), 3.76 (m, 1H), 3.58 (m, 1H), 3.52 (m, 1H), 3.0 (m, 2H), 2.55 (m, 1H) , 2.26 (m, 3H), 2.14 (m, 1H), 1.85 (m, 1H), 1.7 (m, 2H), 1.52 (m, 3H), 1.14 (m, 2H), 0.95 (m, 3H) ppm. MS: *m*/*z* 426.5 (M+1)

### Example 23

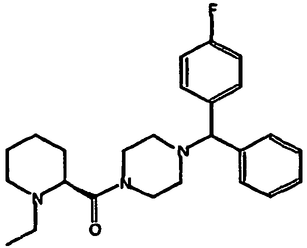

### ((2S)-1-Ethylpiperidin-2-yl)-{4-[(4-fluorophenyl)phenylmethy]-piperazin-1-yl}-methanone dihydrochloride (Compound 42).

Compound **42** was prepared from (2S)-1-ethylpiperidin-2-yl carboxylic acid and N-(4-fluorophenyl)phenylmethylpiperazine as described in Example 1 to yield 282 mg (60%) as the dihydrochloride salt. ¹H NMR (DMSO-d6, 500MHz) δ 7.98 (m, 4H), 7.46 (m, 2H), 7.41 (m, 1H), 7.33 (m, 2H), 5.75 (m, 1H), 4.52-3.88 (m, 5H) , 3.55 (m, 2H), 3.3-2.8 (m, 6H), 2.05 (m, 1H), 1.85 (m, 3H), 1.56 (m, 2H), 1.22 (t, 3H) ppm. MS m/z 410.5 (M+1).

### Reference Example 24

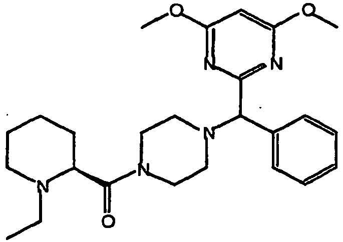

### {4-[4,6-Dimethoxypyrimidin-2-yl)-phenylmethyl]-piperazin-1-yl}-((2S)-1-ethyl-piperidin-2-yl)-methanone (Compound 43).

Compound **43** was prepared from (2S)-1-ethylpiperidin-2-yl carboxylic acid and N-(4,6-dimethoxypyrimidin-2-yl)phenylmethylpiperazine as described in Example 1 to yield 184 mg (40%). ¹H NMR (CDCl₃, 500MHz) δ 7.6 (m, 2H), 7.28 (m, 3H), 5.88 (s, 1H), 4.48 (m, 1H), 4.04 (m, 1H), 3.94 (s, 6H), 3.85 (m, 1H), 3.65 (m, 2H), 3.09 (m, 2H), 2.55 (m, 3H), 2.4 (m, 2H), 2.2 (m, 1H), 1.9 (m, 1H, 1.8-1.5 (m, 4H), 1.26 (m, 2H), 1.04 (m, 3H) ppm. MS *m*/*z* 454.4 (M+1) .

### Example 25

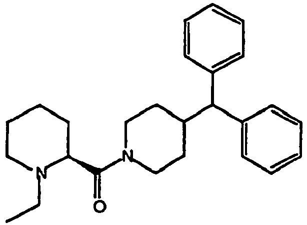

### (4-Benzhydrylpiperidin-1-yl)-((2S)-1-ethylpiperidin-2-yl)-methanone hydrochloride (Compound 44).

Compound **44** was prepared from (2S)-1-ethylpiperidin-2-yl carboxylic acid and 4-benzhydrylpiperidine as described in Example 1 to yield 174mg (57%) as the hydrochloride salt. ¹H NMR (CDCl₃, 500MHz) δ 7.24 (m, 8H), 7.12 (m, 2H), 4.28 (m, 1H), 4.38 (s, 1H) , 3.99 (m, 1H), 3.63 (m, 1H), 3.42 (d, 1H), 3.20 (m, 3H), 3.00 (m, 1H), 2.53 (m, 2H), 2.32 (m, 1H), 2.15 (m, 1H), 1.82-1.60 (m, 5H), 1.50 (m, 1H), 1.35 (m, 3H), 1.03 (m, 2H) ppm. MS: *m*/*z* 391.5 (M+1).

### Reference Example 26

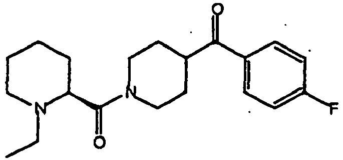

### ((2S)-1-Ethylpiperidin-2-yl)-[4-(4-fluorobenzoyl)-piperidin-1-yl]-methanone (Compound 45).

Compound **45** was prepared from (2S)-1-ethylpiperidin-2-yl carboxylic acid and 4-(4-fluorobenzoyl)piperidine as described in Example 1 to yield 830 mg (80%). ¹H NMR (CDCl₃, 500 MHz) δ .7.88 (m, 2H), 7.06 (m, 2H), 4.55 (m, 1H), 3.37 (m, 1H), 3.08 (m, 3H), 2.72 (m, 1H), 2.54 (m, 1H), 2.1 (m, 1H), 1.88-1.4 (m, 10H), 1.16 (m, 2H), 0.94 (m, 3H) ppm. MS *m*/*z* 347.3 (M+1).

### Reference Example 27

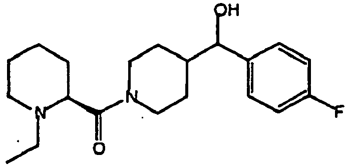

### ((2S)-1-Ethylpiperidin-2-yl)-{4-[(4-fluorophenyl)hydroxymethyl]-piperidin-1-yl}-methanone (Compound 46).

(2S)-1-Ethyl-piperidin-2-yl)-[4-(4-fluorobenzoyl)-piperidin-1-yl]-methanone (Compound 45) (157mg) was dissolved in 5 ml of ethanol. To the solution was added 50mg of 10% palladium on carbon and the flask was charged with hydrogen (1 atm.). After stirring overnight, the reaction was filtered through Celite and the reaction evaporated in vacuo. The reaction was purified by flash chromatography (SiO₂) eluting with 95:5 dichloromethane/ methanol to afford 95 mg of compound 46. ¹H NMR (DMSO-d6, 500 MHz) δ 7.2 (m, 2H), 7.03 (m, 2H), 5.2 (br s, 1H), 4.43 (m, 1H), 4.17 (m, 2H), 3.78 (m, 1H), 3.37 (m, 1H), 3.81 (m, 4H), 2.42 (m, 1H), 1.8-1.5 (m, 6H), 1.42 (m, 2H), 1.06-0.92 (m, 5H) ppm. MS m/z 349.3 (M+1).

### Example 28

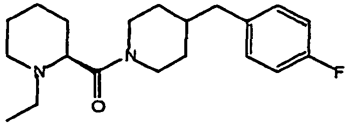

### ((2S)-1-Ethylpiperidin-2-yl)-[4-(4-fluorobenzyl)-piperidin-1-yl]-methanone hydrochloride (Compound 47).

Compound **47** was prepared from (2S)-1-ethylpiperidin-2-yl carboxylic acid and 4-(4-fluorobenzyl)piperidine as described in Example 1 to yield 379mg (67%) as the HCl salt. ¹H NMR (CDCl₃, 500 MHz) δ 6.93 (m, 2H), 6.8 (m, 2H), 4.6 (m, 1H), 4.48 (m, 2H), 2.98 (m, 2H), 2.72 (m, 1H), 2.5 (m, 1H), 2.32 (m, 3H), 2.03 (m, 1H), 1.75 (m, 1H), 1.55 (m, 8H), 1.12 (m, 1H), 0.95 (m, 4H) ppm. MS *m*/*z* 333.4 (M+1).

### Reference Example 29

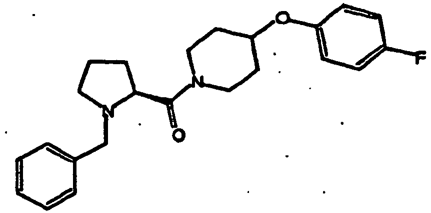

### ((2S)-1-Benzylpyrrolidin-2-yl)-[4-(4-fluorophenoxy)-piperidin-1-yl] -methanone hydrochloride (Compound 48).

Compound **48** was prepared from (2S)-1-benzylpyrrolidin-2-yl carboxylic acid and 4-(4-fluorophenoxy)piperidine as described in Example 1 to yield 516mg (65%) as the hydrochloride salt. ¹H NMR (CDCl₃, 500 MHz) δ 7.36 (m, 5H), 6.98 (m, 2H), 6.87 (m, 2H), 4.4 (m, 1H), 3.98 (m, 1H), 3.80 (m, 2H), 3.52 (m, 4H), 3.10 (m, 1H), 2.32 (m, 1H), 2.15 (m, 1H), 1.84 (m, 4H), 1.75 (m, 3H) ppm. MS: *m*/*z* 383.5 (M+1)

### Reference Example 30

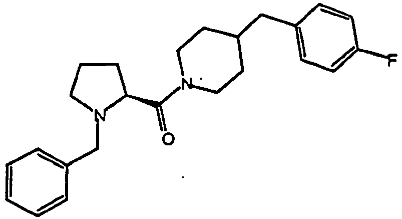

### ((2S)-1-Benzylpyrrolidin-2-yl)-[4-(4-fluorobenzyl)-piperidin-1-yl]-methanone hydrochloride (Compound 49).

Compound **49** was prepared from (2S)-1-benzylpyrrolidin-2-yl carboxylic acid and 4-(4-fluorobenzyl)piperidine as described in Example 1 to yield 674mg (81%) as the hydrochloride salt. ¹H NMR (CDCl₃, 500 MHz) δ 7.83 (m, 1H), 7.71 (m, 1H), 7.42 (m, 3H), 7.07 (m, 4H), 4.58 (m, 2H), 4.38 (m, 0.5H), 4.28 (m, 0.5H), 3.87-3.58 (m, 2H), 3.35 (m, 1H), 2.80 (m, 0.5H), 2.70 (m, 0.5H), 2.58-2.17 (m, 5H), 1.95 (m, 1H), 1.68 (m, 4H), 1.41 (m, 1H), 1.03 (m, 2H) ppm. MS: *m*/*z* 381.5 (M+1)

### Reference Example 31

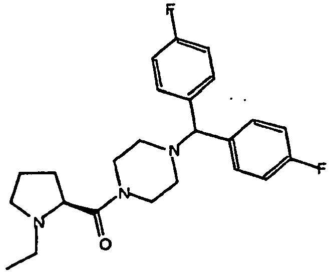

### 4-[Bis-(4-fluorophenyl)methyl]piperazin-1-yl}-((2S)-1-ethylpyrrolidin-2-yl)-methanone dihydrochloride (Compound 50)

Compound **50** was prepared from (2S)-1-ethylpyrrolidin-2-yl carboxylic acid and N-Bis-(4-fluorophenyl)methylpiperazine as described in Example 1 o yield 1.58g (52%) as the dihydrochloride salt. ¹H NMR (CDCl₃, 500 MHz) δ 7.41 (m, 4H), 7.06 (m, 6H), 4.28 (s, 1H), 3.79 (m, 1H), 3.72 (m, 1H), 3.58 (m, 2H), 3.38 (m, 1H), 3.26 (m, 1H), 2.80 (m, 1H), 2.5-2.25 (m, 6H), 2.14 (m, 1H), 1.94 (m, 1H), 1.84 (m, 2H), 1.13 (t, 3H) ppm. MS : *m*/*z* 414.5 (M+1).

### Reference Example 32

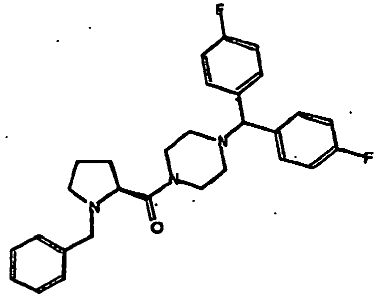

### ((2S)-1-Benzylpyrrolidin-2-yl)-{4-bis-(4-fluorophenyl)methyl)-piperazin-1-yl}-methanone dihydrochloride (Compound 51)

Compound **51** was prepared from (2S)-1-benzylpyrrolidin-2-yl carboxylic acid and N-Bis-(4-fluorophenyl)methylpiperazine as described in Example 1 to yield 1.59g (66%) as the dihydrochloride salt. ¹H NMR (CDCl₃, 500 MHz) δ 7.4 (m, 2H), 7.02 (m, 2H), 4.26 (s, 1H), 3.92 (m, 1H), 3.69-3.4 (m, 3H), 3.32 (m, 1H), 2.39 (m, 3H), 1.65 (m, 3H), 1.45 (m, 6H) ppm. MS: *m*/*z* 476.5 (M+1) .

The compounds described in Examples 33-34 were prepared by Scheme 2 (Method B).

### Reference Example 33

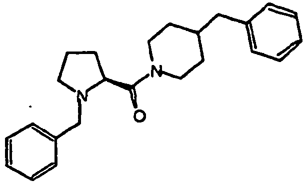

### (4-Benzylpiperidin-1-yl)-((2S)-1-benzylpyrrolidin-2-yl)-methanone hydrochloride (Compound 52).

1-Benzyl-L-proline (3.12 g, 15 mmol) was taken into 60 mL anhydrous dichloromethane. To this solution was added HOBT (2.06 g, 15 mmol), 4-benzylpiperidine (1.77 ml, 10.1 mmol), and EDC (3.84 g, 20 mmol). The reaction was stirred for 16 hours at room temperature. The reaction was diluted with dichloromethane, washed with saturated sodium bicarbonate, water and brine. The organic layer was dried over anhydrous sodium sulfate, filtered, and evaporated in vacuo. The crude residue was purified by flash chromatography (SiO₂) using 4% MeOH in dichloromethane yielding 3.60 g (98%) of compound 52 which was converted to the hydrochloride salt (3.41g; 95%). ¹H NMR (D₂O, 500MHz) : δ 0.4-1.0 (m, 2H) ; 1.4-1.6 (m, 2H); 1.7 (m, 1H) ; 1.8 (m, 1H) ; 1.9 (m, 1H) ; 2.1 (m, 1H) ; 2.4 (m, 4H); 2.8 (m, 1H) ; 3.3 (m, 1H) ; 3.5 (1H) ; 3.7-3.9 (m, 2H); 4.1 (dd, 1H) ; 4.5 (m, 1H) ; 4.4, 4.6 (dd, 1H) ; 7.1-7.4 (m, 10H) ppm. MS *m*/*z* 363 (M+1).

### Reference Example 34

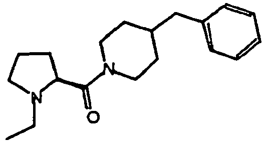

### (4-Benzylpiperidin-1-yl)-((2S)-1-ethylpyrrolidin-2-yl)-methanone hydrochloride (Compound 53).

Compound **53** was prepared from (2S)-1-ethylpyrrolidin-2-yl carboxylic acid and 4-benzylpiperidine as described in Example 33 to yield 233 mg (53%) of as the HCl salt. ¹H NMR (CDCl₃, 500MHz): δ 1.0 (q, 2H); 1.3 (m, 2H); 1.7 (m, 3H); 1.9 (m, 1H) ; 2.0-2.2 (m, 2H) ; 2.4 (m, 3H); 2.5 (m, 1H) ; 2.8 (t, 0.5H) ; 2.9 (t, 0.5H) ; 3.2-3.4 (m, 3H) ; 3.5 (m, 1H) ; 3.7 (t, 1H); 4.4 (m, 1H) ; 4.6 (m, 1H) ; 7.0 (d, 2H); 7.1 (t, 1H) ; 7.2 (t, 2H) ppm. MS *m*/*z* 301 (M+1).

The compounds described in Examples 35-45 were prepared by Scheme 1.

### Reference Example 35

### Preparation of (4-Benzylpiperidin-1-yl)-((2R)-1-benzylpyrrolidin-2-yl)-methanone (Compound 55)

### Step A.

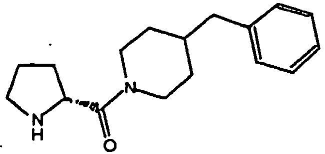

### (4-Benzylpiperidin-1-yl-(2R)-pyrrolidin-2-yl-methanone, hydrochloride (Compound 54).

BOC-D-Proline (3.345g, 15.5 mmol) was dissolved in 25 ml of dichloromethane. To the solution was added 4-benzylpiperidine (1.28 ml, 10.3 mmol), HOBT (2.1g, 15.5 mmol), and EDC (3.96g, 20.6 mmol). The reaction was stirred at room temperature for 16 hours. The reaction was diluted with 50ml of dichloromethane and washed with saturated sodium bicarbonate, water, and brine. The organic layer was dried over anhydrous sodium sulfate, filtered, and evaporated to give a yellow oil that was purified by flash chromatography (Si02) eluting with 95:5 dichloromethane/methanol to afford 3.22g (58% yield) of (2R)-2-(4-Benzylpiperidine-1-carbonyl)-pyrrolidine-1-carboxylic acid *tert*-butyl ester. MS *m*/*z* 373 (M+1).

(2R)-2-(4-Benzylpiperidine-1-carbonyl)-pyrrolidine-1-carboxylic acid *tert*-butyl ester (3.22g, 8.6 mmol) was dissolved in 50 ml of ethyl acetate. The solution was treated with anhydrous HCl and stirred at room temperature for 1 hour. The reaction was evaporated in vacuo and dried to afford 2.5g (94 % yield) of compound 54. ¹H NMR (CDCl₃, 500 MHz) δ 7.35 (m, 2H), 7.2 (m, 1H), 7.1 (d, 2H), 4.7 (br. s, 1H), 4.5 ((t, 1H), 3.7 (t, 1H), 3.6 (m, 1H), 3.4 (br. s, 1H), 3.1 (m, 1H), 2.7 (m, 1H), 2.6 (m, 2H), 2.5 (m, 1H), 2.2 (m, 1H), 2.1-2.0 (m, 1H), 1.9 (m, 1H), 1.85-1.70 (m, 3H), 1.6 (m, 1H), 1.4-1.1 (m, 2H) ppm. MS *m*/*z* 309 (M+1).

### Step B.

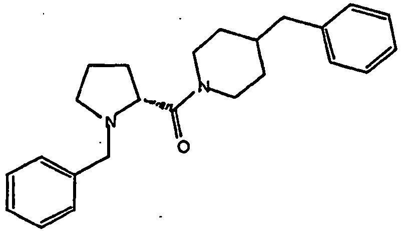

### (4-Benzylpiperidin-1-yl)-((2R)-1-benzylpyrrolidin-2-yl)methanone hydrochloride (Compound 55) not encompassed in the present invention)

(4-Benzylpiperidin-1-yl-(2R)-pyrrolidin-2-yl-methanone hydrochloride (67mg, 0.22 mmol) was dissolved in 5ml of dichloromethane. To the solution was added benzyl bromide (25 µl, 0.22 mmol), triethylamine (60 µl, 0.44mmol), and 5mg of tetrabutylammonium iodide. The solution was stirred at room temperature for 16 hours. The reaction was diluted with 25ml of dichloromethane and washed with saturated sodium bicarbonate, water, and brine. The organic layer was dried over anhydrous sodium sulfate, filtered, and evaporated in vacuo to afford a yellow oil. This was purified by flash chromatography (SiO₂) eluting with 100:2 dichloromethane/methanol to afford compound **55** which was converted to its hydrochloride salt, 43 mg (51% yield). 1H NMR ((D2O, 500 MHz) δ 7.3-7.1 (m, 8H), 7.05 (t, 2H), 4.50 (t, 1H), 4.10 (m, 1H), 3.90 (m, 1H), 3.40 (d, 0.5H), 3.30 (m, 1.5H), 3.0 (m, 1H), 2.75 (q, 1H), 2.5-2.3 (m, 3H), 2.2 (m, 1H), 2.0 (m, 1H), 1.85-1.45 (m, 7H), 1.1-0.85 (m, 2H) ppm.

### Reference Example 36

### Preparation of (4-Benzylpiperidin-1-yl)-((2S)-1-phenethyloyrrolidin-2-yl)-methanone (Compound 57)

### Step A.

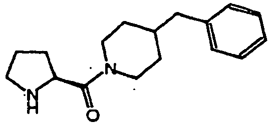

### (4-Benzylpiperidin-1-yl)-(2S)-pyrrolidin-2-yl-methanone hydrochloride (Compound 56).

2-(4-Benzylpiperidine-1-carbonyl)-pyrrolidine-1-carboxylic acid tert-butyl ester (10.4 g, 48 mmol) was subjected to identical conditions as the D isomer in Example 35, Step A (Compound 54) to yield 14.98g (100%) of (2S)-2-(4-Benzylpiperidine-1-carbonyl)-pyrrolidine-1-carboxylic acid *tert*-butyl ester. MS *m*/*z* 373 (M+1).

The product, (2S)-2-(4-Benzylpiperidine-1-carbonyl)-pyrrolidine-1-carboxylic acid tert-butyl ester (14.98g, 48 mmol, 1.2 equivalents) was dissolved in 150mL EtOAc, and HCl(g) was bubbled through for 15 min, then the reaction was stirred for 1 hour. The reaction was evaporated to afford 12.64g (100%) of compound 56 as a white foam. ¹H NMR (CDCl₃, 500MHz) : δ 1.1-1.4 (m, 2H); 1.6 (m, 1H); 1.7-1.85 (m, 3H); 1.9 (m, 1H); 2.0-2.1 (m, 1H) ; 2.2 (m, 1H) ; 2.5 (m, 1H) ; 2.6 (m, 2H) ; 2.7 (m, 1H) , 3.1 (q, 1H) ; 3.4 (bs, 1H); 3.6 (m, 1H); 3.7 (t, 1H); 4.5 (t, 1H) ; 4.7 (bs, 1H); 7.1 (d, 2H); 7.2 (m, 1H); 7.35 (m, 2H) ppm. MS *m*/*z* 273 (M+1).

### Step B.

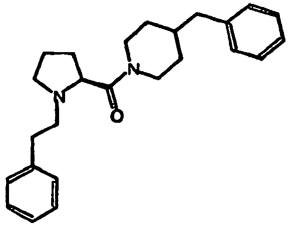

### (4-Benzylpiperidin-1-yl)-((2S)-1-phenethylpyrrolidin-2-yl)-methanone (Compound 57).

We added 174 mg (0.56 mmol, 1.0 equivalent) (4-Benzyl-piperidin-1-yl)-(S)-pyrrolidin-2-yl-methanone, 0.085mL (0.62 mmol, 1.1 equivalents) 2-bromoethylbenzene, and 270 mg (1.96 mmol, 3.5 equivalents) potassium carbonate to 10 mL acetonitrile. The solution was refluxed for 12 hours, filtered, and evaporated. The residue was dissolved in DCM, washed with saturated sodium bicarbonate, and the aqueous layer was extracted with DCM. We washed the combined organic phases with water and brine and then dried the organic phase over sodium sulfate. The solution was then filtered, and evaporated. The residue was purified via flash chromatography using a gradient from DCM to 4%MeOH in DCM. The fractions were evaporated, suspended in 5mL Et₂O and dissolved by the dropwise addition of HCl/Et₂O. The ether was evaporated, the solid residue stirred in 10 mL diethyl ether for 30 min, decanted, and the ether wash was repeated. The solid was filtered and dried under reduced pressure to afford 96 mg (42%) of compound **57** as the HCl salt. ¹H NMR (CDCl₃, 500 MHz) : δ 1.0 (m, 2H) ; 1.7 (m, 3H) ; 1.9 (q, 1H) ; 2.1 (m, 1H) ; 2.2 (m, 1H) ; 2.3 (t, 0.5H) ; 2.5 (t, 2.5H) ; 2.6 (m, 1H) ; 2.7 (t, 0.5H) ; 2.9 (t, 0.5H) ; 3.1 (m, 1H) ; 3.2 (m, 1H) ; 3.3-3.8 (m, 5H) ; 4.4 (t, 1H) ; 4.6 (dd, 1H) ; 7.0 (d, 2H); 7.1-7.35 (m, 8H) ppm. MS m/z 377 (M+1).

### Reference Example 37

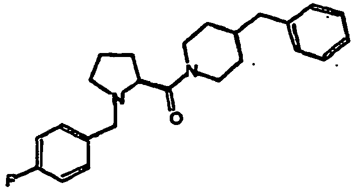

### (4-Benzylpiperidin-1-yl)-[(2S)-1-(4-fluorobenzyl)-Pyrrolidin-2-yl]-methanone hydrochloride (Compound 58).

Compound **58** was prepared as described above except without heating and employing 4-flouro-benzyl-bromide instead of 2-bromoethyl-benzene, yielding 146 mg (70%) as the HCl salt. ¹H NMR (CDCl₃, 500MHz): 80.5-0.8 (m, 1.33H); 1.1 (m, 0.67H) ; 1.6-1.8 (m, 2H); 1.9 (m, 1H) ; 2.0 (m, 1H) ; 2.1 (m, 1H) ; 2.3 (m, 1H) ; 2.6 (m, 4H) ; 3.0 (q, 1H) ; 3.4 (m, 1H) ; 3.7 (dd, 1H) ; 3.8-4.1 (m, 2H) ; 4.3 (dd, 1H); 4.6 & 4.8 (dd, 1H) ; 4.7 (t, 1H) ; 7.2-7.4 (m, 5H) ; 7.45 (m, 2H) ; 7.6 (m, 2H) ppm. MS m/z 381 (M+1).

### Reference Example 38

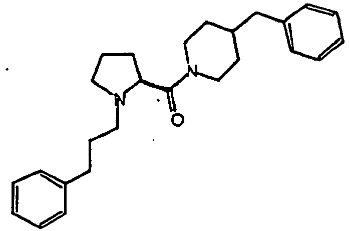

### (4-Benzylpiperidin-1-yl)-[(2S)-1-(3-phenylpropyl) - pyrrolidin-2-yl]-methanone hydrochloride (Compound 59).

Compound **59** was prepared as in Example 37, above, except heating only at 60°C for 12 hours, and employing 3-phenylpropyl bromide instead of 2-bromoethylbenzene, yielding 190 mg (89%) as the HCl salt. ¹H NMR (CDCl₃, 500MHz) : δ 1.0 (m, 2H) ; 1.7 (m, 2H) ; 1.9-2.3 (m, 5H); 2.4-2.2.7 (m, 6H); 2.9 (m, 1H) ; 3.1-3.25 (m, 2H); 3.3 (m, 1H) ; 3.6 (bs, 1H) ; 3.7 (bs, 1H) ; 4.4 (d, 1H) ; 4.6 (bs, 1H) ; 7.0-7.3 (m, 10H) ppm. MS m/z 391 (M+1).

### Example 39

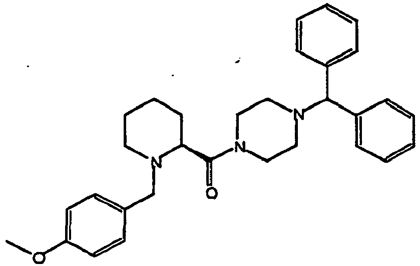

### (4-Benzhydrylpiperazin-1-yl)-[(2S)-1-(4-methoxybenzyl)-piperidin-2-yl]-methanone dihydrochloride (Compound 60).

Compound **60** was prepared from [4-(1,1-diphenylmethyl]-piperazin-1-yl]-(2S)-piperidin-2-yl-methanone and 4-methoxybenzyl bromide as described for Compound **21** in Example 9 to afford 141mg (55%) as the dihydrochloride salt. ¹H NMR (DMSO-d6, 500 MHz) δ 8.2 (m, 4H), 7.71 (m, 6H), 7.63 (dd, 2H), 7.28 (dd, 2H), 5.95 (m, 1H), 4.95-4.32 (m, 3H), 4.28 (m, 2H), 4.12 (m, 3H), 4.03 (s, 3H), 3.86 (m, 1H) , 3.6-3.1 (m, 4H), 2.33 (m, 1H), 1.96 (m, 3H), 1.85 (m, 1H), 1.76 (m, 1H) ppm. MS m/z 484.5 (M+1).

### Example 40

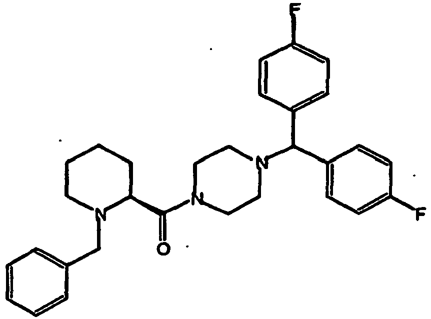

### ((2S)-1-Benzylpiperidin-2-yl)-{4-[bis-(4-fluorophenyl) methyl]-piperazin-1-yl}-methanone (Compound 61).

Compound **61** was prepared from {4-[Bis-(4-fluoro-phenyl)-methyl]-piperazin-1-yl}-(2S)-piperidin-2-yl-methanone and benzyl bromide as described for Compound **21** in Example 9 to afford 448mg (75%) as the dihydrochloride salt. ¹H NMR (CDCl₃, 500 MHz) δ 7.16 (m, 9H), 6.81 (m, 4H), 4.02 (s, 1H), 3.68 (m, 1H), 3.46 (m, 2H), 3.00 (m, 1H), 2.73 (m, 1H), 2.14 (m, 4H), 1.8-1.04 (m, 6H) ppm. MS m/z 490.5 (M+1).

### Example 41

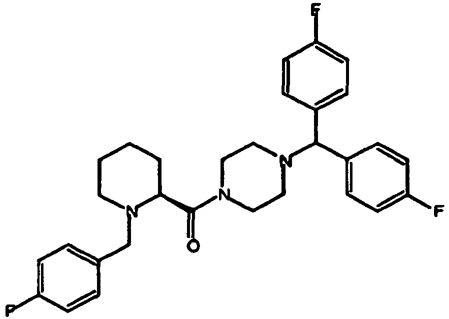

### {4-[Bis-(4-fluorophenyl)methyl]-pipezazin-1-yl}-[(2S)-1-(4-fluorobenzyl)-piperidin-2-yl]-methanone (Compound 62).

Compound **62** was prepared from {4-[Bis-(4-fluoro-phenyl)-methyl]-piperazin-1-yl}-(2S)-piperidin-2-yl-methanone and 4-fluorobenzyl bromide as described for Compound **21** in Example 9 to afford 510mg (83%) as the dihydrochloride salt. ¹H NMR (CDCl₃, 500 MHz) δ 7.24 (m, 6H), 6.90 (m, 6H), 4.09 (s, 1H), 3.71 (m, 1H), 3.54 (m, 2H), 3.11 (m, 1H), 2.80 (m, 1H), 2.19 (m, 4H), 1.80-1.06 (m, 10H) ppm. MS *m*/*z* 508.5 (M+1).

### Example 42

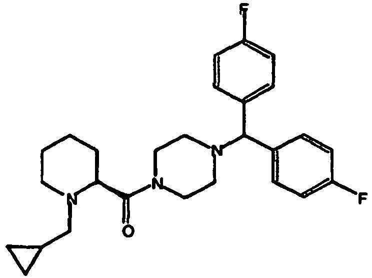

### {4-[Bis-(4-fluorophenyl)methyl]-piperazin-1-yl}-((2S)-1-cyclopropylmethyl-piperidin-2-yl)-methanone (Compound 63).

Compound **63** was prepared from {4-[Bis-(4-fluoro-phenyl)-methyl]-piperazin-1-yl}-(2S)-piperidin-2-yl-methanone and cyclopropylmethyl bromide as described for Compound 21 in Example 9 to afford 442mg (79%) as the dihydrochloride salt. ¹H NMR (CDCl₃, 500 MHz) δ 7.28 (m, 4H), 6.90 (m, 4H), 4.12 (s, 1H), 3.65 (m, 1H), 3.51 (m, 2H), 3.32 (m, 1H), 2.68 (m, 1H), 2.24 (m, 4H), 1.75-1.05 (m, 10H), 0.84 (m, 1H), 0.44 (m, 2H), 0.02 (m, 2H) ppm.

### Example 43

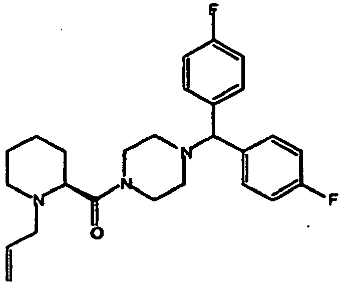

### ((2S)-1-Allylpiperidin-2-yl)-{4-[bis-(4-fluorophenyl)methyll-piperazin-1-yl}-methanone (Compound 64).

Compound **64** was prepared from {4-[Bis-(4-fluorophenyl)-methyl]-piperazin-1-yl}-(2S)-piperidin-2-yl-methanone and allyl bromide as described for Compound 21 in Example 9 to afford 355mg (65%) as the dihydrochloride salt. ¹H NMR (CDCl₃, 500 MHz) δ 7.31 (m, 4H), 6.96 (m, 4H), 5.81 (m, 1H), 5.09 (d, 2H), 4.17 (s, 1H), 3.87 (m, 1H), 3.66 (m, 1H), 3.57 (m, 1H), 3.50 (m, 1H), 3.22 (m, 1H), 3.06 (m, 1H), 2.78 (m, 1H), 2.26 (m, 4H), 1.95 (m, 1H), 1.84-1.34 (m, 6H), 1.22 (m, 1H) ppm. MS *m*/*z* 440.5 (M+1).

### Example 44

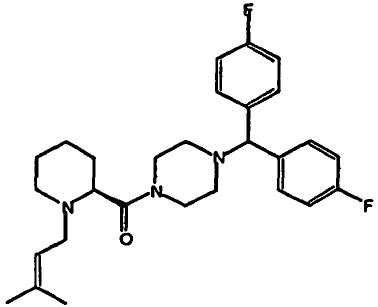

### {4-[Bis-(4-fluorophenyl)methyl]-piperazin-1-yl}-[(2S)-1-(3-methyl-but-2-enyl)-piperidin-2-yl]-methanone (Compound 65).

Compound **65** was prepared from {4-[Bis-(4-fluoro-phenyl)-methyl]-piperazin-1-yl}-(2S)-piperidin-2-yl-methanone and 3-methyl-2-butenyl bromide as described for Compound **21** in Example 9 to afford 290mg (51%) as the dihydrochloride salt. ¹H NMR (CDCl₃, 500 MHz) δ 7.50 (m, 4H), 7.13 (m, 4H), 5.38 (m, 1H), 4.34 (s, 1H), 3.88-3.60 (m, 3H), 3.54-2.98 (m, 3H), 2.46 (m, 4H), 1.95-1.00 (m, 9H), 1.85 (s, 3H), 1.70 (s, 3H) ppm. MS *m*/*z* 468.5 (M+1) .

### Example 45

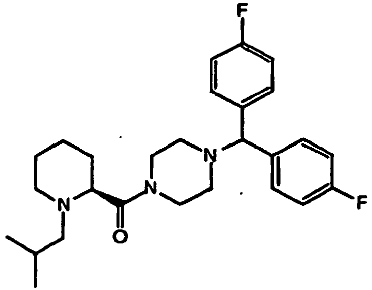

### [4-[Bis-(4-fluorophenyl)methyl]-piperazin-1-yl]-((2S)-1-(2-methylpropyl)-piperidin-2-yl)-methanone (Compound 66).

Compound **66** was prepared similarly to Compound 21 (Example 9) from {4-[Bis-(4-fluoro-phenyl)-methyl]-piperazin-1-yl}-piperidin-2-yl-methanone (500 mg, 1.06 mmol) and 1-bromo-2-methylpropane (164 mg, 1.22 mmol) to afford 590 mg (46% yield) after chromatography. ¹HNMR (CDCl₃, 500 MHz) δ 7.38-7.31, 4H m, 7.05-6.95, 4H m, 4.25-3.80, 2H m, 3.50-3.25 4H m, 3.20-2.75, 2H m, 2.42-2.25, 3H m, 2.25-1.70, 3H m, 1.62-1.40, 6H m, 1.38-1.00, 7H m ppm. MS: m/z 456.5 (M+1).

### Reference Example 46

### Preparation of A Key Intermediate for the Compounds Synthesized By Scheme 5

The compounds described in Examples 46-59 were prepared by Scheme 5.

### Step A:

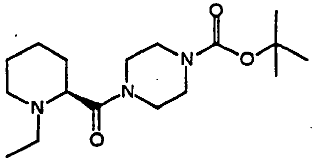

### 4-((2S)-1-Ethylpiperidine-2-carbonyl)-piperazine-1-carboxylic acid tert-butyl ester (Compound 67).

(2S)-1-Ethyl-piperidine-2-carboxylic acid (2.54g, 16.24 mmol) was taken into 20 ml of dichloromethane and 10.4 ml (30 mmol) of diisopropylethylamine. Pivaloyl chloride (2 ml, 16.24 mmol) was added to the solution dropwise. After stirring at room temperature for 1 hour, a solution of piperazine-1-carboxylic acid *tert*-butyl ester (2.76g, 14.6 mmol) was added dropwise and the reaction was stirred overnight. The reaction was washed with 1N sodium hydroxide, water, and brine. The organic layer was dried over anhydrous sodium sulfate, filtered and evaporated in vacuo to afford a yellow oil which was purified by flash chromatography (SiO₂) eluting with gradient of dichloromethane to 5% methanol to afford 4.7g (98%) of compound 67. ¹H NMR (CDCl₃, 500 MHz) δ 4.08 (m, 1H), 3.83 (m, 1H), 3.64 (m, 1H),3.56-3.40 (m, 6H), 3.13 (m, 2H), 2.68 (m, 1H), 2.24 (m, 1H), 1.94 (m, 1H), 1.80 (m, 2H), 1.66 (m, 2H), 1.48 (s, 9H), 1.32 (m, 1H), 1.09 (m, 3H) ppm.

### Step B.

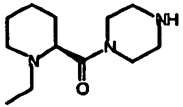

### ((2S)-1-Ethylpiperidin-2-yl)-piperazin-1-yl-methanone dihydrochloride (Compound 68).

4-((2S)-1-Ethyl-piperidine-2-carbonyl)-piperazine-1-carboxylic acid tert-butyl ester (3.1g, 9.5 mmol) was dissolved in 50 mL EtOAc and treated with HCl (g). After stirring for 1 hour, the resulting precipitate was filtered, washed with EtOAc, and dried in vacuo yielding 1.19g (55%) of compound **68**. MS: m/z (M+1) 299.

### Example 47

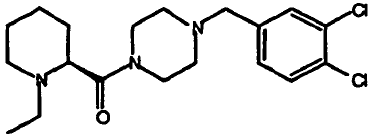

### [4-(3,4-Dichlorobenzyl)-piperazin-1-yl]-((2S)-1-ethylpiperidin-2-yl)-methanone dihydrochloride (Compound 69).

((S)-1-Ethyl-piperidin-2-yl)-piperazin-1-yl-methanone dihydrochloride (200mg (0.70 mmol, 1 equivalent), 139 mg (0.70 mmol, 1.0 equivalent) 3,4-dichlorobenzyl chloride, and 340 mg (2.5 mmol, 3 equivalents) potassium carbonate were suspended in 10 mL acetonitrile and stirred at 60°C for 5 hours. The reaction was filtered through Celite and evaporated in vacuo to afford an oil that was dissolved in DCM, washed with saturated sodium bicarbonate, and brine. The combined organic phases were washed with water, then brine. The washed organic phase was then dried over sodium sulfate, filtered, and evaporated. The resulting crude residue was purified via flash chromatography using a gradient from DCM to 6% MeOH in DCM. The product was then dissolved in Et₂O and HCl/Et₂O was added drop-wise until no more precipitate formed. The precipitate was removed by filtration and the filtrate was lyophilized to yield 35 mg (11%) of compound 69 as the dihydrochloride salt. ¹H NMR (CDCl₃, 500MHz) : δ 1.3 (t, 3H) ; 1.6 (t, 1H) ; 1.8 (m, 2H); 2.0 (dd, 2H) ; 2.2 (dd, 2H) ; 3.1 (m, 2H); 3.2 (m, 1H) ; 3.5 (bs, 4.5 H); 3.8 (d, 1H) ; 3.9 (bs, 3.5H); 4.4 (s, 2H); 4.5 (d, 1H) ; 7.5 (d, 1H) ; 7.70 (d, 1H) ; 7.75 (s, 1H) ppm. MS *m*/*z* 386 (M+1).

### Example 48

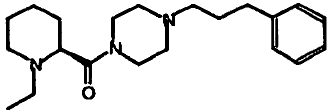

### ((2S)-1-Ethylpiperidin-2-yl)-[4-(3-phenylpropyl)-piperazin-1-yl)-methanone dihydrochloride (Compound 70).

Compound **70** was prepared as described in Example 47 employing (3-bromo-propyl)-benzene instead of 3,4-dichloro-benzyl chloride to yield 102 mg (37%) as the dihydrochloride salt. ¹H NMR (CDCl₃, 500MHz) : δ 1.3 (t, 3H); 1.6 (t, 1H) ; 1.8 (m, 2H); 2.0 (dd, 2H); 2.1 (m, 3H); 2.7 (t, 2H); 2.8-3.3 (m, 8H) ; 3.7 (m, 4H); 4.2 (bs, 1H) ; 4.4 (d, 1H) ; 4.6 (bs, 1H) ; 7.3 (m, 3H) ; 7.4 (m, 2H). MS m/z 417 (M+1).

### Example 49

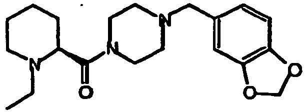

### (4-Benzo[1,3]dioxol-5-ylmethylpiperazin-1-yl)-((2S)-1-ethylpiperidin-2-yl)-methanone dihydrochloride (Compound 71).

Compound **71** was prepared as described in Example 47 employing 5-chloromethyl-benzo[1,3]dioxole instead of 3,4-dichloro-benzyl chloride to yield 196 mg (68%) as the dihydrochloride salt. ¹H NMR (CDCl₃, 500MHz) : δ 1.4 (t, 3H) ; 1.7 (t, 1H) ; 1.9 (m, 2H) ; 2.1 (dd, 2H) ; 2.3 (d, 1H) ; 3.1 (m, 2.5 H); 3.3 (m, 1.5H); 3.3-3.8 (m, 4H); 3.85 (d, 1.5H) ; 3.9-4.3 (m, 1.5H) ; 4.4 (s, 2H); 4.6 (m, 2H) ; 6.1-6 .3 (3 s, 2H); 7.0-7.3 (m, 3H) ppm. MS *m*/*z* 360 (M+1).

### Example 50

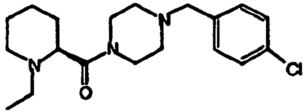

### [4-(4-Chlorobenzyl)-piperazin-1-yl]-((2S)-1-ethylpiperidin-2-yl)-methanone dihydrochloride (Compound 72).

Compound **72** was prepared as described in Example 47 employing 4-chloro-benzyl-bromide instead of 3,4-dichloro-benzyl chloride to yield 44 mg (16%) as the dihydrochloride salt. ¹H NMR (CDCl₃, 500MHz) : δ 1.3 (t, 3H) ; 1.6 (t, 1H) ; 1.8 (m, 2H) ; 2.0 (dd, 2H) ; 2.2 (dd, 2H); 3.1 (m, 2H); 3.2 (m, 1H); 3.5 (bs, 4.5 H); 3.8 (d, 1H); 3.9 (bs, 3.5H) ; 4.4 (s, 2H); 4.5 (d, 1H) ; 7.4 (d, 2H); 7.5 (d, 2H) ppm. MS *m*/*z* 423 (M+1).

### Reference Example 51

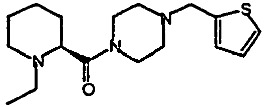

### ((2S)-1-Ethylpiperidin-2-yl)-(4-thiophen-2-ylmethylpiperazin-1-yl)-methanone dihydrochloride (Compound 73).

Compound **73** was prepared as described in Example 47 employing 2-chloromethyl-thiophene instead of 3,4-dichloro-benzyl chloride. 2-chloromethyl-thiophene was prepared as described in J. Janusz et al., J. Med. Chem., 41, pp. 3515-3529 (1998). This process yielded 93 mg (50%) of compound 73. ¹H NMR (CDCl₃, 500MHz) : δ 1.2 (t, 3H) ; 1.5 (t, 1H) ; 1.6 (q, 2H) ; 1.8 (dd, 2H) ; 2.0 (d, 1H) ; 2.9 (m, 2H), 3.1 (bs, 4H); 3.4-3.7 (m, 4H) ; 4.1 (bs, 1H), 4.3 (d, 1H) 4.5 (s, 4H) ; 7.0 (dd, 1H) ; 7.2 (dd, 1H) ; 7.5 (d, 1H) ppm. MS m/z 317 (M+1).

### Example 52

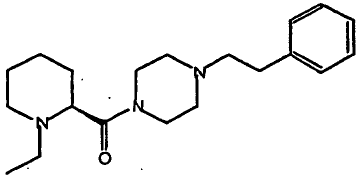

### ((2S)-1-Ethylpiperidin-2-yl)-(4-phenethylpiperazin-1-yl)-methanone dihydrochloride (Compound 74).

Compound **74** was prepared as described in Example 47 employing phenethyl bromide instead of 3,4-dichlorobenzyl chloride to yield 158 mg (50%). ¹H NMR (DMSO-d6) δ 12.2 (br s, 1H), 9.7 (br s, 1H), 7.51 (m, 2H), 7.41 (m, 3H), 4.95 (m, 0.5H), 4.76 (m, 0.5H), 4.61 (m, 1H), 4.32 (m, 1H), 4.22 (m, 2H), 3.86 (m, 1H), 3.80 (m, 1H), 3.71 (m, 1H), 3.47 (m, 3H), 3.31-2.98 (m, 6H), 2.22 (m, 1H), 1.93 (m, 3H), 1.72 (m, 2H), 1.38 (t, 3H) ppm. MS *m*/*z* 330.5 (M+1).

### Example 53

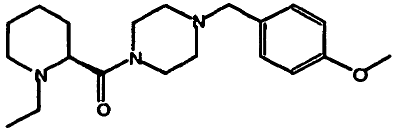

### ((2S)-1-Ethylpiperidin-2-yl)-[4-(4-methoxybenzyl)-piperazin-1-yl]-methanone (Compound 75).

Compound **75** was prepared as described in Example **47** employing 4-methoxybenzyl chloride instead of 3,4-dichlorobenzyl chloride to yield 133 mg (47%). ¹H NMR (CDCl₃, 500 MHz) δ 7.16 (d, 2H), 6.8 (d, 2H), 3.91 (m, 1H), 3.76 (s, 3H), 3.58 (m, 1H), 3.53 (m, 1H), 3.41 (s, 2H), 3.06 (m, 2H), 2.58 (m, 1H), 2.33 (m, 4H), 2.14 (m, 1H), 1.9-1.4 (m, 6H), 11.2 (m, 2H), 0.98 (m, 3H) ppm. MS m/z 346.4 (M+1).

### Example 54

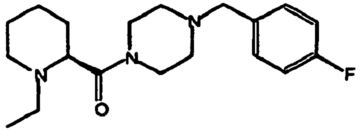

### ((2S)-1-Ethylpiperidin-2-yl)-[4-(4-fluorobenzyl)-piperazin-1-yl] methanone (Compound 76).

Compound **76** was prepared as described in Example 47 using 4-fluorobenzyl bromide instead of 3,4-dichloro-benzyl chloride to yield 134 mg (49%). ¹H NMR (CDCl₃, 500 MHz) δ 7.2 (m, 2H), 6.96 (m, 2H), 3.92 (m, 1H), 3.73 (m, 1H), 3.59 (m, 1H), 3.53 (m, 1H), 3.41 (s, 2H), 3.05 (m, 2H), 2.58 (m, 1H), 2.28 (m, 4H), 2.15 (m, 1H) , 1.82 (m, 1H), 1.75-1.38 (m, 5H), 1.22 (m, 1H), 0.95 (m, 3H) ppm. MS *m*/*z* 334.4 (M+1).

### Reference Example 55

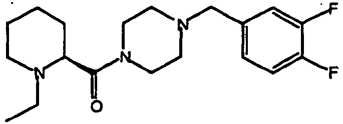

### [4-(3,4-Difluorobenzyl)-piperazin-1-yl]- ((2S) -1-ethylpiperidin-2-yl)-methanone (Compound 77).

Compound **77** was prepared as described in Example 47 using 3,4-difluorobenzyl bromide instead of 3,4-dichloro-benzyl chloride to yield 185 mg (65%). ¹H NMR (CDCl₃, 500 MHz) δ 7.28 (m, 1H), 7.18 (m, 1H), 7.10 (m, 1H), 4.06 (m, 1H), 3.88 (m, 1H), 3.75 (m, 1H), 3.66 (m, 1H), 3.52 (s, 2H), 3.18 (m, 2H), 2.72 (m, 1H), 2.45 (m, 4H), 2.25 (m, 1H), 1.94 (m, 1H), 1.88-1.55 (m, 5H), 1.34 (m, 1H), 1.08 (m, 3H) ppm. MS *m*/*z* 352.5 (M+1)

### Example 56

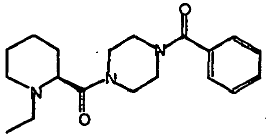

### [4-((2S)-1-Ethylpiperidine-2-carbonyl)-piperazin-1-yl]-phenyl methanone (Compound 78).

((2S)-1-Ethyl-piperidin-2-yl)-piperazin-1-yl-methanone dihydrochloride (221mg, 0.74 mmol) was suspended in 5mL anhydrous DCM. N,N-diisopropylethylamine (0.45ml, 2.6mmol) was added to the solution followed by the dropwise addition of benzoyl chloride (0.095ml, 0.81 mmol). After stirring at room temperature for 16 hours, the reaction was diluted with 5mL of dichloromethane and washed with saturated sodium bicarbonate, water, and brine. The organic layer was dried over anhydrous sodium sulfate, filtered, and evaporated in vacuo. The crude residue was purified by flash chromatography (SiO₂) using a gradient from 100% dichloromethane to 6% methanol in dichloromethane to afford 110mg (45%) of the compound **78**. ¹H NMR (CDCl₃, 500MHz): δ 1.0 (t, 3H); 1.1-1.9 (m, 7H); 2.1 (bs, 1H) ; 2.7 (bs, 1H) ; 3.0 (bs, 2H) ; 3.2-3.9 (m, 7H); 4.1 (bs, 1H); 7.4 (m, 5H) ppm. MS *m*/*z* 330 (M+1).

### Example 57

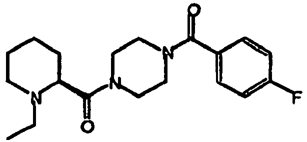

### ((2S)-1-Ethylpiperidin-2-yl)-[4-(4-fluorobenzoyl)-piperazin-1-yl] methanone hydrochloride (Compound 79).

Compound **79** was prepared as described in Example 56 using 4-flourobenzoyl chloride instead of benzoyl chloride to yield 148mg (54%) as the hydrochloride salt. ¹H NMR (CDCl₃, 500MHz) : δ 1.2 (t, 3H); 1.5 (m, 1H) ; 1.65 (broad t, 2H); 1.85 (m, 2H); 2.1 (m, 1H) ; 2.9 (m, 2H); 3.1 (m, 1H) ; 3.5 (m, 4H) ; 3.7 (m, 5H) ; 4.3 (m, 1H) ; 7.1 (t, 2H); 7.4 (m, 2H). %) ppm. MS m/z 348 (M+1).

### Example 58

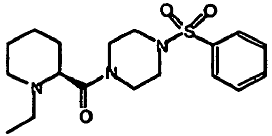

### (4-Benzenesulfonylpiperazin-1-yl)-((2S)-1-ethylpiperidin-2-yl)-methanone hydrochloride (Compound 80).

Compound **80** was prepared as described in Example 56 using benzenesulfonyl chloride instead of 4-flourobenzoyl chloride to yield 117 mg (45%) as the HCl salt. ¹H NMR (CDCl₃, 500MHz): δ 0.85 (t, 3H) ; 1.1-1.2 (m, 1.5H); 1.4-1.55 (m, 2.5H); 1.6 (d, 1H); 1.7 (d, 1H); 1.8 (t, 1H); 2.0 (m, 1H); 2.4 (m, 1H) ; 2.9 (bs, 2H); 3.0 (d, 4H); 3.5-3.8 (broad dd, 2H); 3.9 (bs, 1H); 4.1 (bs, 1H); 7.5 (t, 2H); 7.6 (t, 1H); 7.7 (d, 2H) ppm. MS *m*/*z* 366 (M+1).

### Example 59

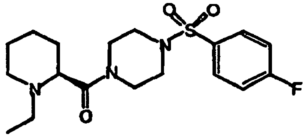

### ((2S)-1-Ethylpiperidin-2-yl)-[4-(4-fluorobenzenesulfonyl)-piperazin-1-yl]-methanone hydrochloride (Compound 81).

Compound **81** was prepared as described in Example 56 using 4-flourobenzenesulfonyl chloride instead of 4-flourobenzoyl chloride to yield 181 mg (67%) as the HCl salt. ¹H NMR (CDCl₃, 500NHz): δ1.0 (t, 3H) ; 1.2-1.5 (m, 3H); 1.6 (d, 1H); 1.7-1.8 (m, 2H); 2.7 (m, 2H); 2.85 (m, 3H); 2.95 (m, 2H); 3.4-3.6 (m, 5H); 4.1 (m, 1H); 7.2 (t, 2H); 7.7 (m, 2H) ppm. MS *m*/*z* 384 (M+1).

The compounds described in Examples 60-64 were prepared using the synthetic scheme depicted in Scheme 6.

### Example 60

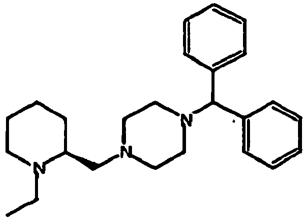

### 1-Benzhydryl-4-((2S)-1-ethylpiperidin-2-ylmethyl)-piperazine (Compound 100).

10ml (10mmol) of 1M Borane-tetrahydrofuran complex was added to a solution of 150 mg (0.36 mmol) of 1-(4-(1,1-Diphenylmethyl)piperazin-1-yl)-1-((S)-1-ethylpiperidin-2-yl)methanone (Compound 1) in 10ml of anhydrous THF at room temperature. The reaction was stirred for 4 days then quenched with the dropwise addition of methanol. The mixture was evaporated in vacuo to give a clear viscous oil. The crude product was dissolved in 10 ml of 1N HCl and 1ml of acetone was added and the solution stirred for 30 mins. The mixture was basified with saturated sodium bicarbonate and then extracted with dichloromethane (2x). The combined extracts were washed with brine, dried over anhydrous sodium sulfate, filtered and evaporated to afford a clear oil that was purified by flash chromatography (SiO₂) eluting with 100:5 dichloromethane/methanol to afford 72 mg of the title compound.
¹H NMR (CDCl₃, 500 MHz) δ 7.31 (m, 4H), 7.18 (m, 4H), 7.11 (m, 2H), 4.10 (s, 1H), 3.15-2.60 (m, 5H), 2.58-2.08 (m, 10H), 1.8 (m, 2H), 1.72 (m, 3H), 1.29 (m, 1H), 1.13 (m, 3H) ppm. MS: *m*/*z* (M+1) 378.5

### Example 61

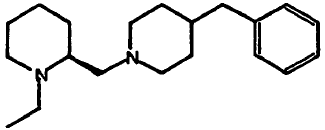

### 4-Benzyl-1-((2S)-1-ethylpiperidin-2-ylmethyl)-piperidine (Compound 101).

Compound **101** was prepared by the reduction of compound **26** as described in Example 60 to yield 141 mg. ¹H NMR (DMSO-d6, 500 MHz) δ 7.45 (m, 2H), 7.36 (m, 3H), 4.23 (m, 3H), 3.99 (m, 1H), 3.88-3.68 (m, 2H), 3.64 (m, 1H), 3.53-3.22 (m, 2H), 3.10 (m, 2H), 2.64 (m, 3H), 2.44 (m, 0.5H), 2.22 (m, 0.5H), 2.07-1.61 (m, 9H),1.43 (t, 3H) ppm. MS *m*/*z* (M+1) 301.5

### Example 62

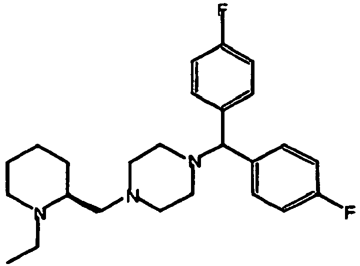

### 1-[Bis-(4-fluorophenyl)methyl]-4-((2S)-1-ethylpiperidin-2-ylmethyl)-piperazine. (Compound 102).

Compound **102** was prepared by the reduction of Compound **25** as described in Example 60 to yield 369 mg. ¹H NMR (CD₃OD, 500 MHz) δ 7.72 (m, 4H), 7.12 (m, 4H), 5.48 (d, 1H), 3.63 (br s, 0.5H), 3.43 (m, 1H), 3.34 (m, 1.5H), 3.22-2.75 (m, 11H), 2.62 (m, 1H), 1.95 (m, 0.5H), 1.86 (m, 0.5H), 1.72-1.58 (m, 3H), 1.48 (m, 2H), 1.25 (m, 3H) ppm. MS: *m*/*z* (M+1) 414.6

### Reference Example 63

### Synthesis of ((2S,4R)-1-Benzyl-4-methoxypyrrolidin-2-yl)-(4-benzylpiperidin-1-yl)methanone (Compound 153)

### Step A.

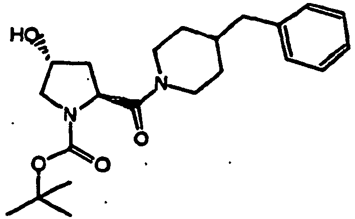

### (2S,4R)-2-(4-benzylpiperdine-1-carbonyl)-4-hydroxypyrrolidine-1-carboxylic acid tert -butyl ester (Compound 151).

To Boc-4-hydroxyproline (5.0g, 21.6 mmol) in 20 mL of dichloromethane was added diisopropyl carbodiimide (3.0g, 23.9 mmol) and 1-Hydroxylbenzotriazole (3.2g, 23.8 mmol). After stirring for 1h, 4-benzylpiperdine (4.2g, 23.8 mmol) was added neat. The solution was stirred for 12 hours. The reaction was diluted with 50 ml of dichloromethane and washed with 1M HCl, NaHCO₁ (sat.), brine, dried (MgSO₄) and concentrated. The product was purified by flash chromatography to give 6.67g (80 % yield) as a white foam. ¹H NMR (500 MHz, CDCl₃) δ 7.45-7.20 (m, 5H), 5.40 (s, 1 H), 4.90-4.45 (m, 2H), 4.10-3.55 (m, 3H), 3.30-3.00 (m, 1H), 2.75-2.55 (m, 2H), 2.35-1.70 (m, 7H), 1.60&1.50 (s,s 9H (rotomers)), 1.40-1.10 (m, 2H) ppm. MS: *m*/*z* 389.5 (M+1).

### Step B.

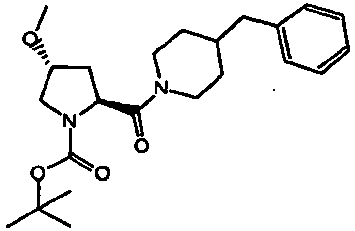

### (2S,4R)-2-(4-benzylpiperdine-1-carbonyl)-4-methoxypyrrolidine-1-carboxylic acid tert -butyl eater (Compound 152).

2-(4-benzylpiperdine-1-carbonyl)-4-hydroxy-pyrrolidine-1-carboxylic acid tert-butyl ester in THF (5 mL) was added dropwise to hexane-washed NaH (113 mg, 2.83 mmol) suspended in THF (5 mL). After stirring for 0.5 h, methyl iodide (402 mg, 2.83 mmol) was added neat and the solution was refluxed for 4 hours. The reaction was poured into NaHCO₃ (sat.), extracted with ethyl acetate, washed with brine, dried (MgSO₄) and concentrated. Flash chromatography afforded 720 mg (70% yield) of a amber oil. ¹H NMR (500 MHz, CDCl₃) δ 7.20-6.90 (m, 5H), 4.90-4.45 (m, 2H), 4.15-3.50 (m, 3H), 3.35&3.31 (s,s, 3H (rotomers)), 3.20-2.90 (m, 1H), 2.60-2.50 (m, 2H), 2.30-1.65 (m, 6H), 1.60&1.50 (s,s 9H (rotomers)), 1.40-1.10 (m, 2H) ppm. MS: *m*/*z* 403.5 (M+1).

### Step C.

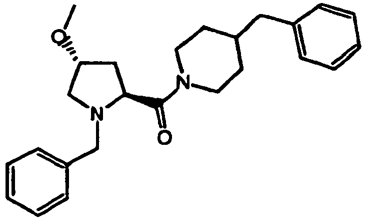

### ((2S,4R)-1-Benzyl-4-methoxypyrrolidin-2-yl)-(4-benzylpiperdin-1-yl)-methanone (Compound 153).

2-(4-benzylpiperdine-1-carbonyl)-4-methoxy-pyrrolidine-1-carboxylic acid tert -butyl ester (720 mg, 1.79 mmol) was treated with HCl(g) in ethyl acetate. After 1 hour the solution was evaporated and used without further purification. Alkylation was performed as described above from (4-Benzyl-piperidin-1-yl)-(4-methoxy-pyrrolidin-2-yl)-methanone and Benzyl bromide (459 mg, 2.68 mmol) to afford 400mg after flash chromatography. The final compound 153 was converted to a citrate salt (592 mg, 57 % yield). ¹HNMR (500 MHz, CDCl₃) δ 7.20-6.90 (m, 10H), 4.50-4.40 (d, 2H), 4.90-3.10 (m, 5H), 3.05 (s, 3H), 2.70-2.60 (m, 1H), 2.00-1.80 (m, 1H), 1.60-1.35 (m, 4H), 1.30-1.10 (m, 2H) ppm. MS: *m*/*z* 393.5 (M+1).

### Reference Example 64

### Synthesis of [(2S, 4R)-1-Benzyl-5-(4-benzylpiperidine-1-carbonyl)-pyrrolidin-3-yloxyl]-acetic acid methyl ester (Compound 155).

### Step A.

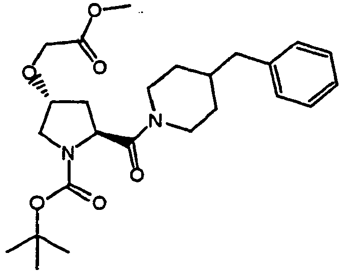

### (2S, 4R)-2-(4-benzylpiperdine-1-carbonyl)-4-methoxycarbonylmethoxypyrrolidine-1-carboxylic acid tert-butyl ester (Compound 154).

This was prepared via the procedure reported for Example 63 (Step B) where the reaction of Compound 151 (1.0 g, 2.57 mmol) and methyl bromo acetate (488 mg, 5.14 mmol) afforded 581 mg (49% yield) of the desired product. ¹H NMR (500 MHz, CDCl₃) δ 7.40-7.30 (m, 5H), 4.95-4.65 (m, 2H), 4.45-3.65 (m, 8H), 3.20-2.95 (m, 2H), 2.70-2.20 (m, 5H), 1.90-1.70 (m, 3H), 1.60-1.45 (s,s, 9H(rotomers)), 1.45-1.15 (m, 2H) ppm. MS: *m*/*z* 461.5 (M+1).

### Step B.

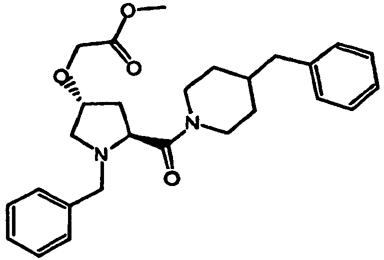

### [(2S, 4R)-1-Benzyl-5-(4-benzylpiperidine-1-carbonyl)-pyrrolidin-3-yloxyl]-acetic acid methyl ester (Compound 155).

Compound **155** was prepared as described in Example 63, Step C from 2-(4-benzyl-piperdine-1-carbonyl)-4-methoxy-carbonylmethoxy-pyrroline-1-carboxylic acid tert-butyl ester (581 mg, 1.26 mmol) and Benzyl bromide (324 mg, 1.89 mmol) to afford 270 mg (48 % yield ) after flash chromatography. ¹H NMR (500 MHz, CDCl₃) δ 7.40-6.95 (m, 10H), 4.50-4.40 (m, 1H), 4.10-3.65 (m, 5H), 3.70-3.20 (m, 5H), 2.71-2.62 (m, 1H), 2.40-2.35 (m, 3H), 2.20-1.90 (m, 2H), 1.60-1.40 (m, 4H), 1.20-1.00 (m, 3H) ppm. MS: *m*/*z* 451.5 (M+1).

### Example 65

### Combinatorial Synthesis of Compounds Via Scheme 7

Compounds of this invention were also made via the synthetic scheme set forth in Scheme 7. The coupling of the appropriate Boc-Amino Acid (150 µmol) with amines (300 µmole) was accomplished using N-cyclohexanecarbodiimide-N'-propyloxymethyl polystyrene resin (300 µmol) as described in Example 12. The resulting Boc-protected amino amides were treated with a saturated solution of HCl in ethyl acetate (5 mL). After shaking for 3 hours, filtration and evaporation afforded the pure products as hydrogen chloride salts.

The above products were taken up in methanol (1 mL) and transferred to the reaction block wells containing K₂CO₃ (excess) suspended in CH₃CN (5 mL). The reactions were treated with the appropriate alkyl halide (300 µmol) and the reaction block was shaken for 24 hours at ambient temperature or at 50°C, depending upon the alkyl halide. Filtration and evaporation gave the crude compounds. Purification was performed using reverse phase HPLC (H₂O/CH₃CN/0.1% TFA) to afford the desired products as determined by LC/MS.

Table 2 sets forth compounds that were prepared by this method or via Scheme 3 (see Example 11) and their mass spectrometry values.

**Table 2. Compounds prepared by Scheme 3 (N-methyl derivatives) Scheme 7 (N-ethyl or N-benzyl derivatives).**

| # | Structure | MS (M/Z) | # | Structure | MS (m/z) |
|---|---|---|---|---|---|
| 202 | 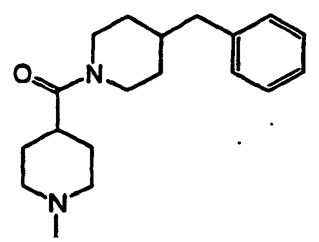 | 301.57 | | | |
| 204 | 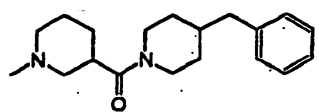 | 301.57 | | | |
| 218 | 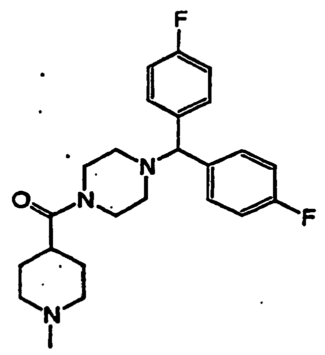 | 414.51 | | | |
| 220 | 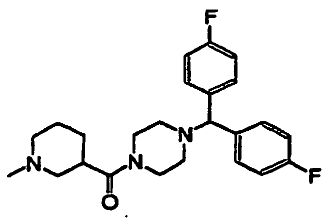 | 414.52 | | | |
| 226 | 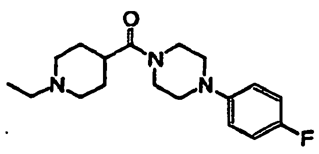 | 320.1 | | | |
| | | | 255 | 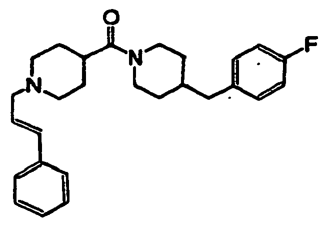 | 420.1 |
| 256 | 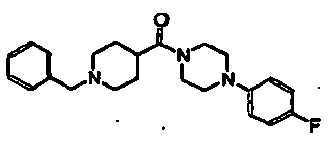 | 382.1 | 257 | 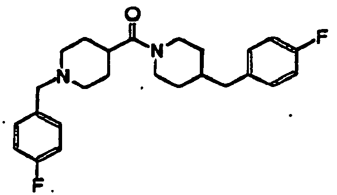 | 413.1 |
| 258 | 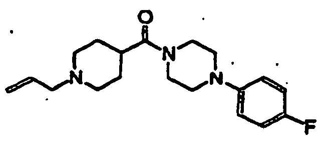 | 332.0 | | | |
| 260 | 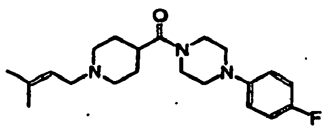 | 360.2 | | | |
| 262 | 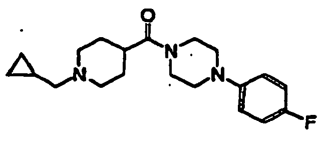 | 346.1 | | | |
| 264 | 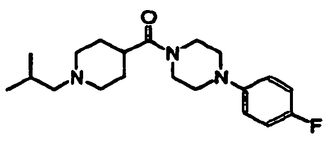 | 346.1 | | | |
| 266 | 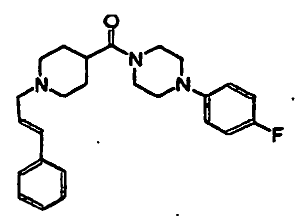 | 408.1 | | | |
| 268 | 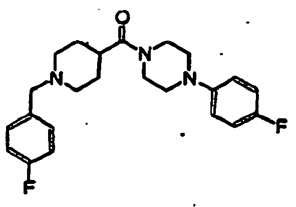 | 410.1 | | | |
| 270 | 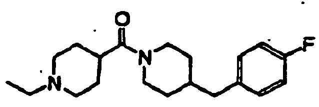 | 333.1 | | | |
| 272 | 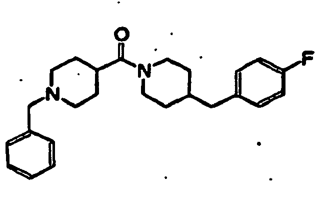 | 3952 | | | |
| 274 | 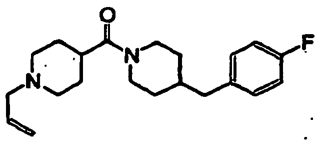 | 345.1 | | | |
| 276 | 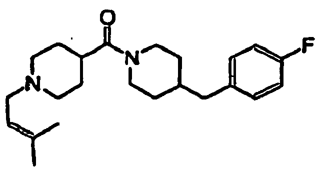 | 373.2 | | | |
| 278 | 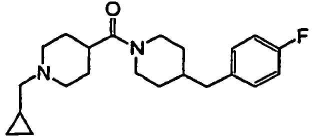 | 3592 | | | |

### Example 66

### Neuroprotection Assay

The ventral mesencephalic region was dissected out of embryonic day 15 Sprague-Dawley rat embryos (Harlan), dissociated into single cell suspension by a combination of trypsinization and trituration (Costantini et al., Neurobiol Dis., pp. 97-106 (1998). Dissociated VM cells were plated into poly-L-ornithine-coated 96-well plates at a density of 85,000 cells/well in 100 uL of DMEM supplemented with 18% heat-inactivated horse serum, 0.24% glucose, 2 mM glutamine and 50 u/ml pernicillin/streptomycin and incubated in a 5% CO₂ incubator. After one day in culture (DIV1), the medium was replaced with 100 µL of a defined medium (DMEM supplemented with 1x N2 cocktail (Gibco-BRL), 0.12% glucose, 2 mM glutamine, and 50 units/ml penicillin/streptomycin) containing DMSO or various concentrations of the compounds of this invention. On DIV5, neuroexcitotoxic injury was induced by the addition of various concentrations of the glutamate receptor agonist NMDA (100-400 µM). Cultures were incubated with the neurotoxin for 20 hours and the effects of neurophilin compounds were assessed using high affinity ³H-dopamine uptake according to a procedure published by Park and Mytilineou [Brain Res., 599, pp. 83-97 (1992)].

The table below shows the results of this assay for various compounds of this invention.

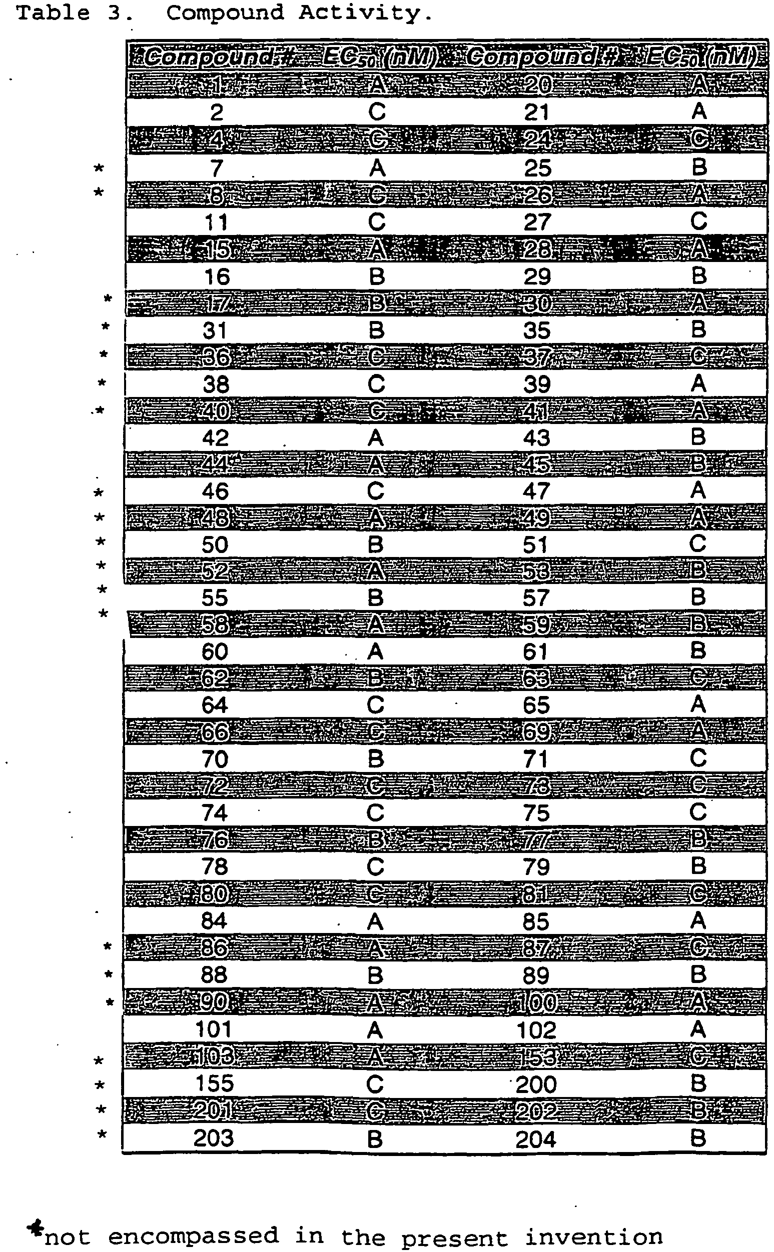

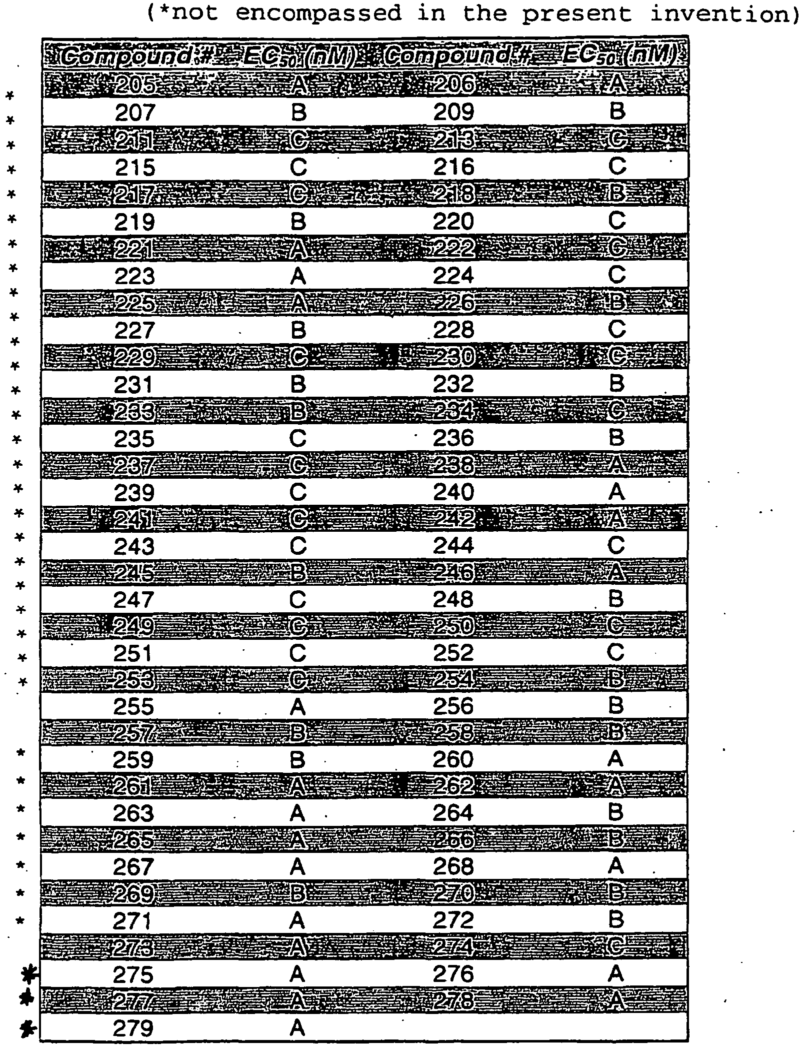

In the table above, "A" designates an EC₅₀ of less than 100 nM; "B" designates an EC₅₀ of between 100 and 500 nM; and "C" designates an EC₅₀ of greater than 500 nM. All of the compounds tested above had EC₅₀ values of less than 1250 nM. It is expected that all compounds of this invention will show detectable activity in this assay.

### Example 67

### Preparation of ((2S)-1-Ethylpiperidin-2-yl)-[4-(4-fluoro benzylidene)piperidin-1-yl] methanone hydrochloride (Compound 84)

### Step A.

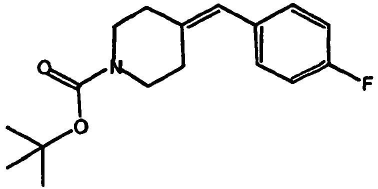

### 4-(4-Fluorobenzylidene)piperidine-1-carboxylic acid tert-butyl ester (Compound 82).

4-Fluorobenzyl triphenylphosphonium chloride (54.2g, 133.2 mmol) was suspended in 400 ml of anhydrous THF. Sodium hydride (60% dispersion in mineral oil; 5.35g, 133.2 mmol) was added to the suspension and stirred at room temperature for 3 hours. A solution of tert-butyl 4-oxo-1-piperidinecarboxylate (25 g, 125.5 mmol) in 150 ml of anhydrous THF was added dropwise over 1 hour. The reaction was heated to reflux for 8 hours and then cooled to room temperature, filtered, and the filtrate evaporated in vacuo to afford the crude product as a yellow viscous oil. The crude product was purified by flash chromatography (SiO₂) eluted with a gradient of hexane to hexane-ethyl acetate (7:3). The pure fractions were combined and evaporated to afford 25.83 g (70% yield) of Compound 82 as a white crystalline solid.

### Step B.

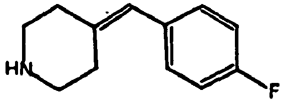

### 4-(4-Fluorobenzylidene)piperidine hydrochloride (Compound 83).

4-(4-Fluorobenzylidene)piperidine-1-carboxylic acid tert-butyl ester (Compound **82**; 695mg, 2.38 mmol) was dissolved in 25 ml of ethyl acetate and anhydrous HCl gas was bubbled into the solution at room temperature until warm. The reaction was stirred for 1 hour, then evaporated *in vacuo* to afford 521 mg (96% yield) of the desired product as a white crystalline solid.

### Step C.

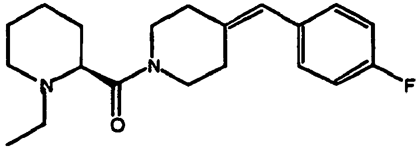

### ((2S)-1-Ethylpiperidin-2-yl)-[4-(4-fluorobenzylidene)piperidin-1-yl]-methanone hydrochloride (Compound 84).

Compound **84** was prepared from (2S)-1-ethylpiperidin-2-yl carboxylic acid and 4-(4-Fluorobenzylidene)piperidine hydrochloride (Compound 83) as described in Example 1 to yield 234 mg (70%) as the hydrochloride salt.
¹H NMR (CD₃OD, 500 MHz) δ 7.23 (m, 2H), 7.05 (m, 2H), 6.48 (s, 1H), 4.56 (m, 1H), 3.84 (m, 0.5H), 3.72 (m, 2H), 3.65 (m,2H), 3.55 (m, 0.5H), 3.23 (m, 1H), 3.04 (m, 2H), 2.61 (m, 1H), 2.53 (m, 2H), 2.44 (m, 1H), 2.18 (m, 1H), 1.96 (m, 2H), 1.88-1.68 (m, 3H), 1.38 (t, 3H). MS *m*/*z* 331.04 (M+1)

### Reference Example 68

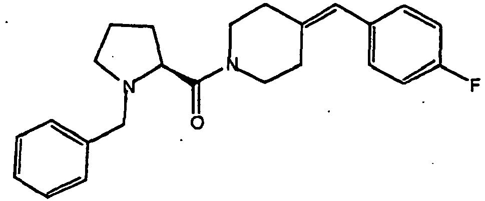

### ((2S)-1-Benzylpyrrolidin-2-yl)-[4-(4-fluorobenzylidene) piperidin-1-yl] methanone hydrochloride (Compound 85).

Compound 85 was prepared from (2S)-1-benzyl-pyrrolidin-2-yl carboxylic acid and 4-(4-Fluorobenzylidene)piperidine hydrochloride (Compound **83**) as described in Example 1 to yield 310 mg (79%) as the hydrochloride salt.
¹H NMR (CD₃OD, 500 MHz) δ 7.57 (m, 2H), 7.48 (m, 3H), 7.22 (m, 2H), 7.08 (m, 2H), 6.46 (m, 1H), 4.79 (m, 1H), 4.50 (d, 1H), 4.32 (d, 1H), 3.71 (m, 1.5H), 3.62 (m, 0.5H), 3.48-3.21 (m, 3.5H), 2.65 (m, 1H), 2.52 (m, 1H), 2.42-2.22 (m, 3H), 2.12 (m, 1H), 2.05 (m, 1H), 1.95 (m, 1H). MS *m*/*z* 379.12 (M+1)

### Reference Example 69

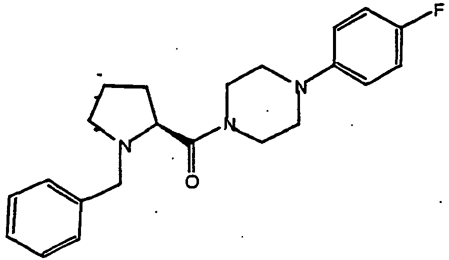

### ((2S)-1-Benzylpyrrolidin-2-yl)-[4-(4-fluorophenyl) piperazin-1-yl] methanone hydrochloride (Compound 86).

Compound **86** was prepared from (2S)-1-benzylpyrrolidin-2-yl carboxylic acid and 4-(4-fluorophenyl)piperazine as described in Example 1 to yield 620 mg (72%) as the dihydrochloride salt.
¹H NMR (CDCl3, 500 MHz) δ 7.34 (m, 5H), 7.02 (m, 2H), 6.84 (m, 2H), 4.02 (m, 1H), 3.96-3.68 (m, 4H), 3.55 (m, 2H), 3.26-2.95 (m, 5H), 2.38 (m, 1H), 2.21 (m, 1H), 1.92 (m, 3H). MS *m*/*z* 368.3 (M+1)

### Reference Example 70

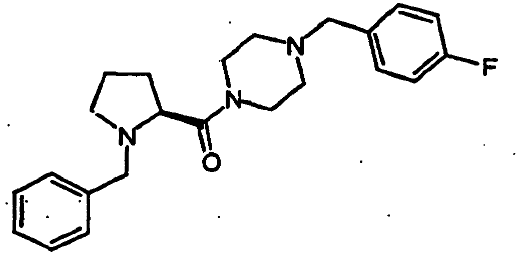

### ((2S)-1-Benzyl-pyrrolidin-2-yl)-[4-(4-fluoro-benzyl)-piperazin-1-yl] methanone (Compound 87).

Compound **87** was prepared from (2S)-1-benzylpyrrolidin-2-yl carboxylic acid and 4-(4-fluorobenzyl)piperazine as described in Example 1 to yield 210mg (36% yield) as the dihydrochloride salt. ¹H NMR (CDCl₃, 500 MHz) 7.25 (m, 7H), 6.95 (m, 2H), 3.90 (d, 1H), 3.65-3.49 (m, 4H), 3.41 (s, 2H), 3.31 (m, 1H), 2.97 (m, 1H), 2.25 (m, 6H), 2.13 (m, 1H), 1.81 (m, 2H), 1.72 (m, 1H). MS *m*/*z* 382.16 (M+1).

### Reference Example 71

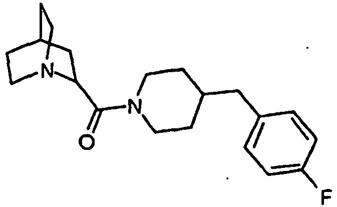

### (1-Aza-bicyclo[2.2.2]oct-2-yl)-[4-(4-fluoro-benzyl)-piperidin-1-yl] methanone hydrochloride (Compound 88).

Compound **88** was prepared from 1-azabicyclo[2.2.2]octane-2-carboxylic acid and 4-(4-fluorobenzyl)piperidine as described in Example 1 to yield 30 mg (19%) as the hydrochloride salt.
¹H NMR (CDCl₃, 500 MHz) 7.09 (m, 2H), 6.95 (m, 2H), 4.61 (d, 1H), 4.01-3.88 (m, 2H), 3.49 (s, 1H), 3.41 (s, 1H), 3.21-3.03 (m, 2H), 2.92 (m, 1H), 2.55 (m, 3H), 2.25 (m, 1H), 2.05 (d, 1H), 1.80-1.55 (m, 7H), 1.39 (m, 1H), 1.15 (m, 2H). MS *m*/*z* 331.08 (M+1).

### Example 72

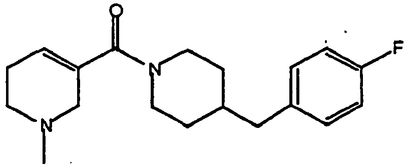

### [4-(4-Fluorobenzyl)piperidin-1-yl]-(1-methyl-1,2,5,6-tetrahydropyridin-3-yl) methanone hydrochloride (Compound 89).

Compound **89** was prepared from arecaidine hydrochloride and 4-(4-fluorobenzyl)piperidine as described in Example 33 to yield 1.26 g (91%) as the hydrochloride salt.
¹H NMR (CD3OD, 500 MHz) δ 7.08 (m, 2H), 6.88 (m, 2H), 6.04 (s, 1H), 4.28 (m, 1H), 4.02 (m, 1H), 3.93 (d, 1H), 3.67 (d, 1H), 3.48 (m, 1H), 3.12 (m, 1H), 2.95 (m, 1H), 2.86 (s, 3H), 2.56 (m, 2H), 2.47 (m, 3H), 1.73 (m, 1H), 1.62 (m, 2H), 1.06 (m, 2H). MS m/z 317.2 (M+1).

### Example 73

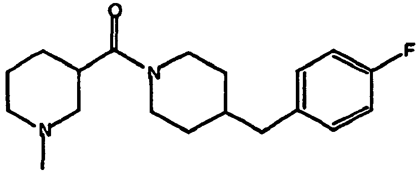

### [4-(4-Fluorobenzyl)piperidin-1-yl]-(1-methylpiperidin-3-yl) methanone hydrochloride (Compound 90).

Compound **89** (200 mg) was dissolved in 10 ml of ethanol. To the solution was added 50 mg of 10% palladium on carbon and the flask charged with an atmosphere of hydrogen (1 atm.). After 3 hours, the reaction was filtered through Celite and evaporated to afford compound **90** as a clear viscous oil which was converted to the hydrochloride salt (132mg).
¹H NMR (CD3OD, 500 MHz) δ 7.07 (m, 2H), 6.88 (m, 2H), 4.43 (d, 0.5H), 4.38 (d, 0.5H), 3.88 (d, 0.5H), 3.76 (d, 0.5H), 3.50-3.22 (m, 3H), 3.18 (s, 0.5H), 3.10 (m, 0.5H), 2.98 (m, 2H), 2.85 (m, 1H), 2.78 (m, 1H), 2.53 (m, 1H), 2.48 (m, 2H), 1.94-1.34 (m, 7H), 1.3-0.92 (m, 3H). MS *m*/*z* 319.3 (M+1).

### Example 74

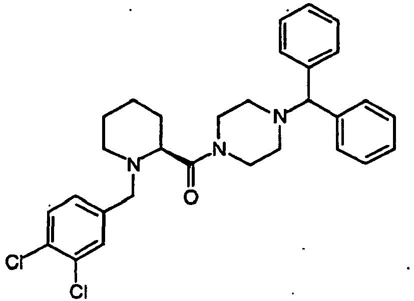

### (4-Benzhydryl-piperazin-1-yl)-[(2S)-1-(3,4-dichloro-benzyl)-piperidin-2-yl] methanone dihydrochloride (Compound 91).

Compound **91** was prepared from 1-[4-(1,1-diphenylmethyl)piperazin-1-yl]-(2S)-piperidin-2-yl methanone dihydrochloride and 3,4-dichlorobenzyl chloride as described for Compound 21 in Example 9 to afford 56 mg (56%) as the dihydrochloride salt.
¹H NMR (CDCl₃, 500 MHz) 7.48-7.25 (m, 10H), 7.21 (d, 2H), 7.15 (m, 1H), 4.21 (s, 1H), 3.81 (d, 2H), 3.65 (s, 2H), 3.24 (m, 2H), 2.91 (s, 1H), 2.38 (s, 4H), 1.98 (s, 1H), 1.75 (s, 3H), 1.51 (s, 2H), 1.29 (s, 2H). MS *m*/*z* 523.01 (M+1).

### Reference Example 75

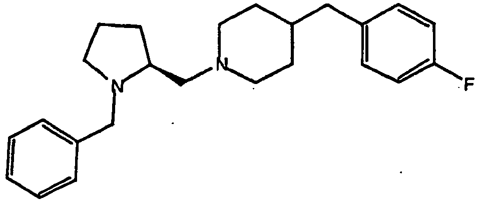

### 1-((2S)-1-Benzylpyrrolidin-2-ylmethyl)-4-(4-fluorobenzyl)piperidine dihydrochloride (Compound 103).

Compound **103** was prepared by the reduction of Compound **49** as described in Example 60 to yield 241 mg (89%) of the title compound as the dihydrochloride salt. ¹H NMR (CDCl₃, 500 MHz) δ 7.38-7.30 (m, 5H), 7.09 (m, 2H), 6.98 (m, 2H), 4.3 (d, 1H), 3.33 (m, 1H), 2.97 (br s, 3H), 2.66 (m, 2H), 2.52 (d, 2H), 2.37 (br s, 1H), 2.18 (br s, 1H), 1.98 (m, 3H), 1.85-1.55 (m, 5H), 1.51 (m, 1H), 1.32 (m, 2H). MS *m*/*z* 367.4 (M+1).

## Claims

1. A compound selected from any one of compounds:

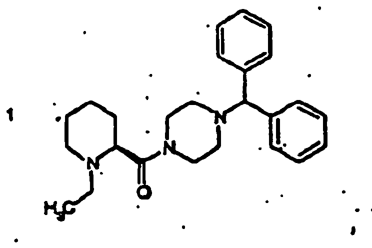

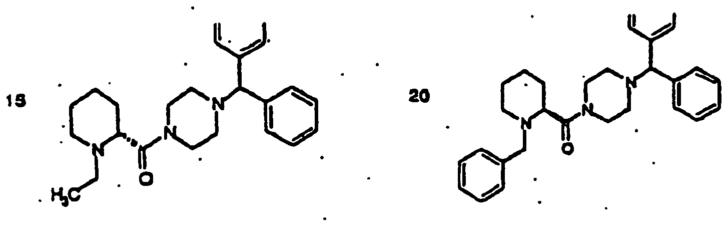

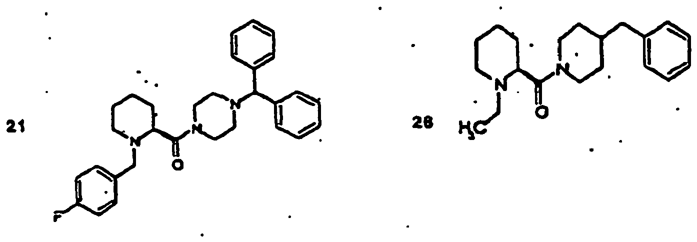

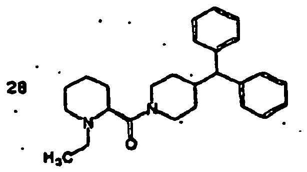

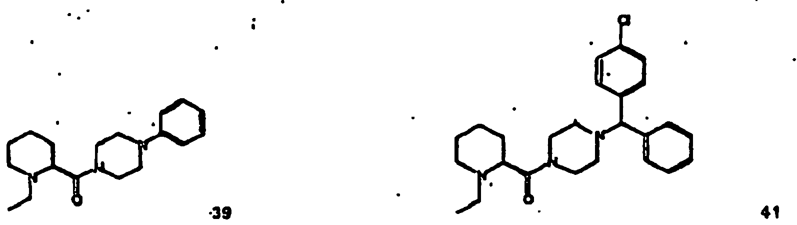

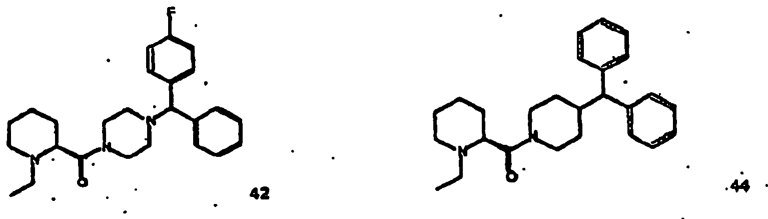

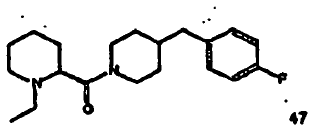

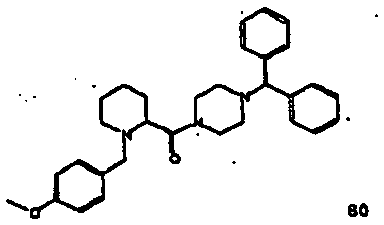

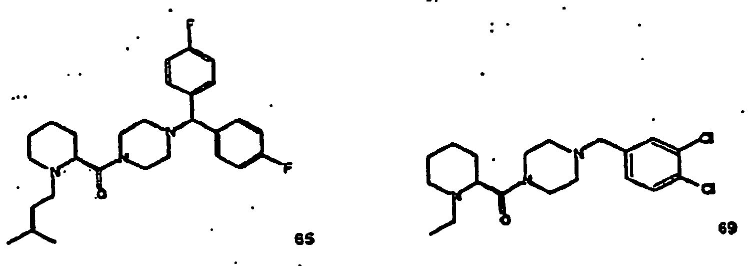

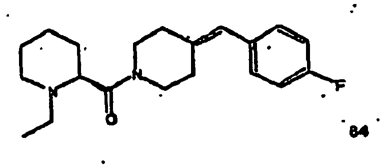

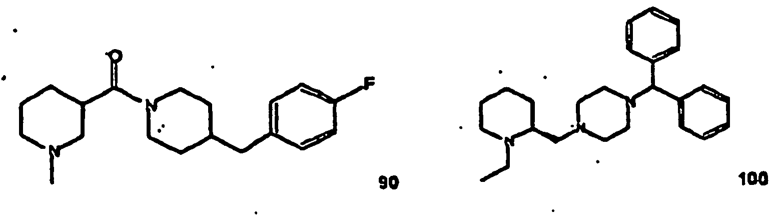

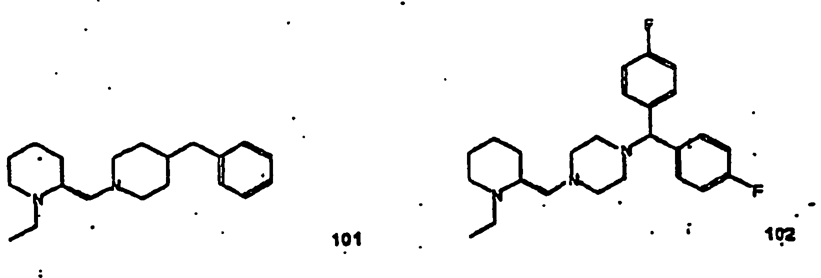

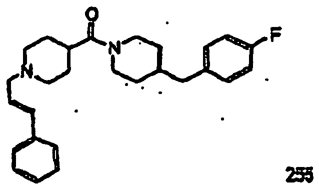

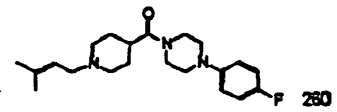

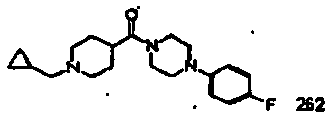

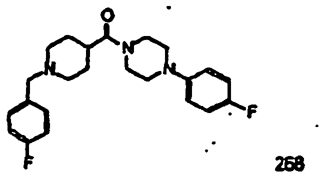

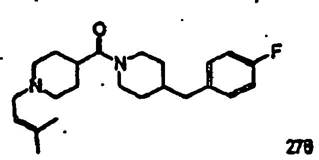

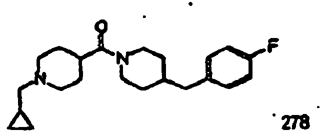

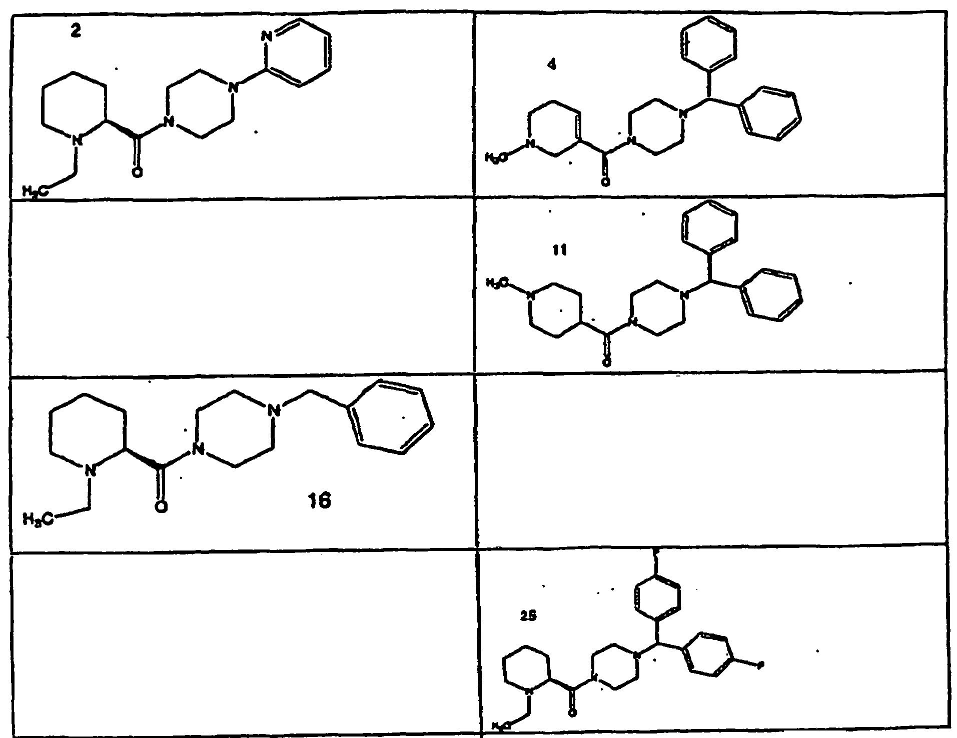

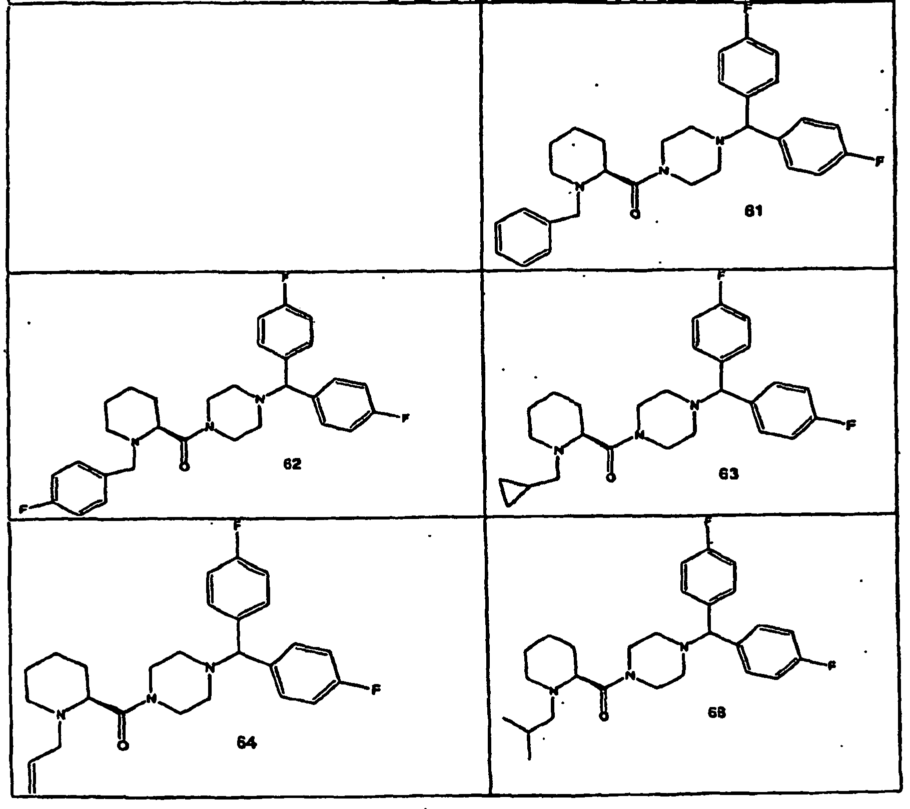

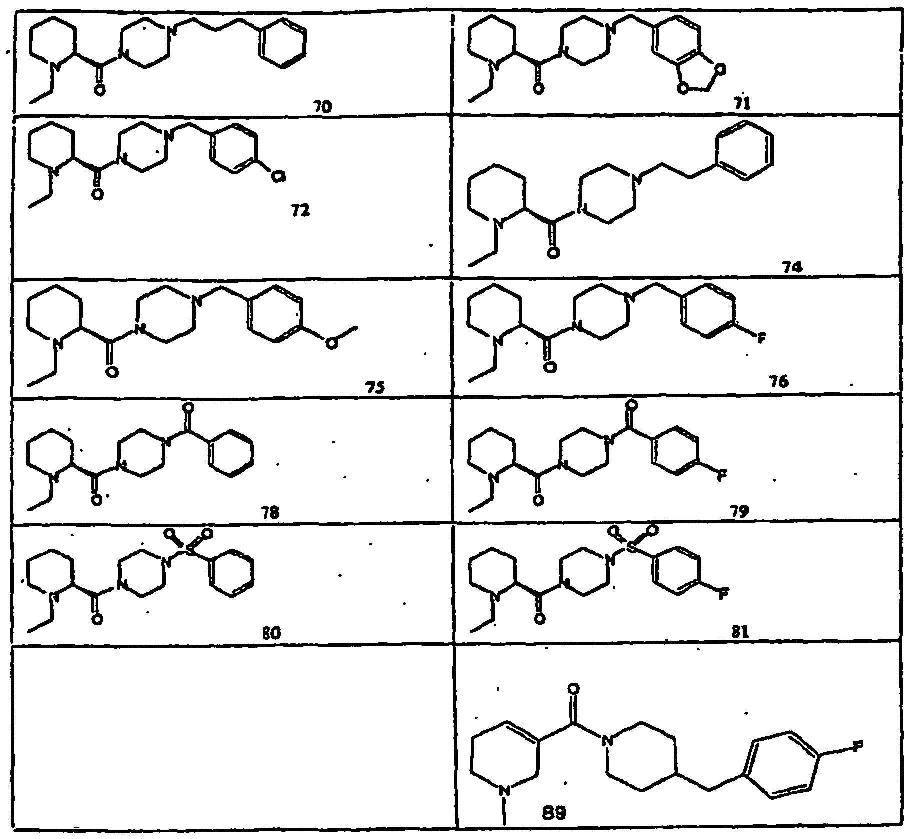

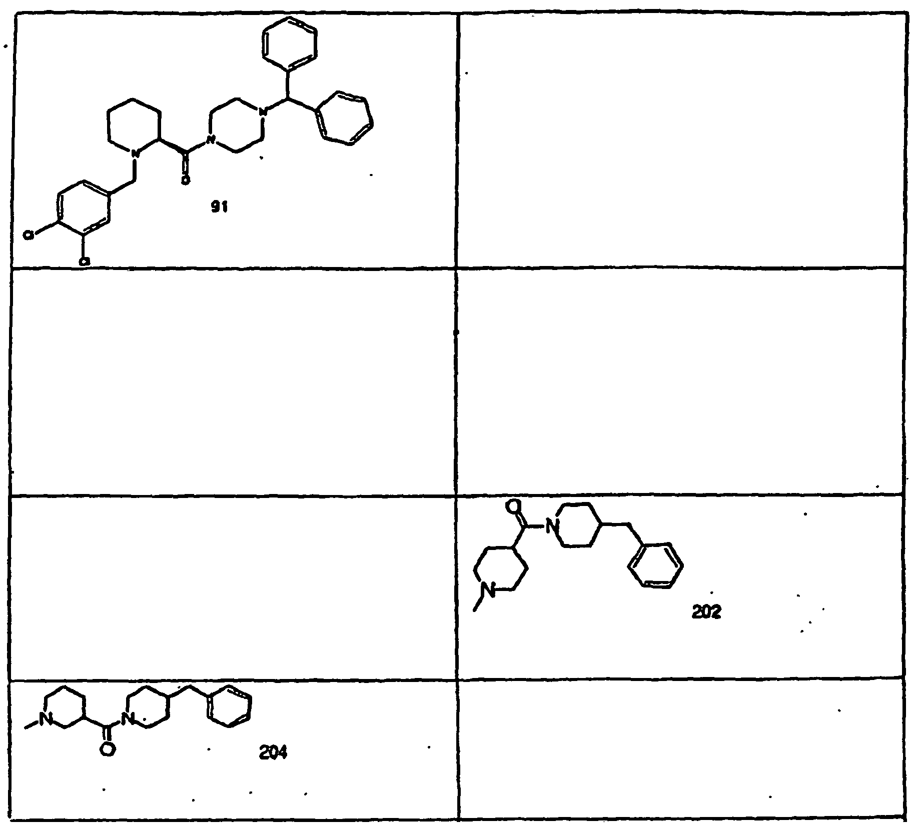

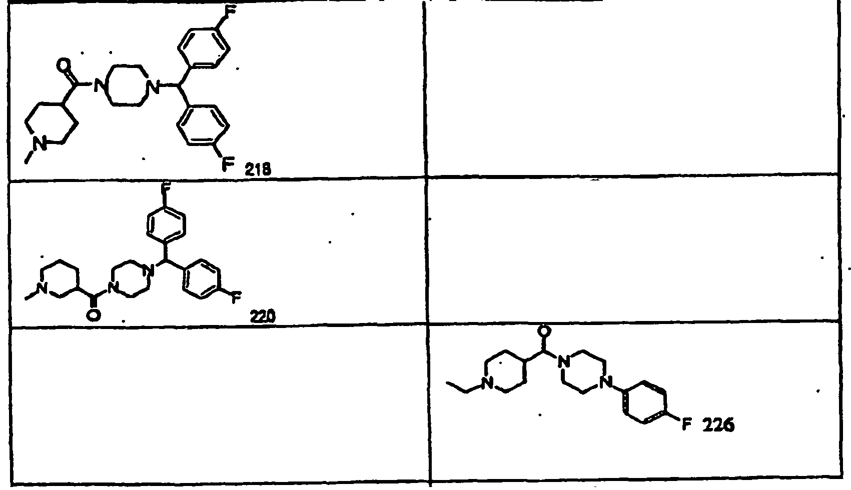

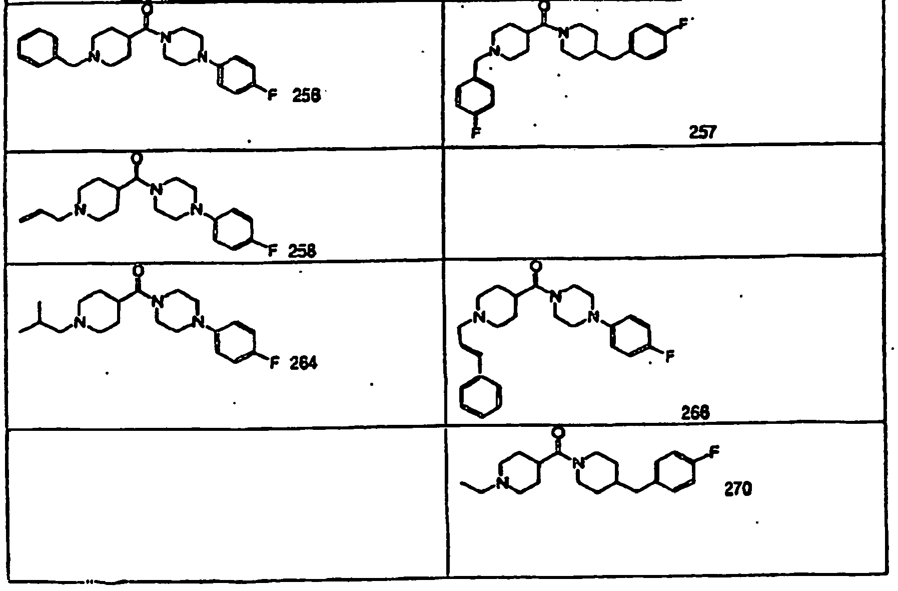

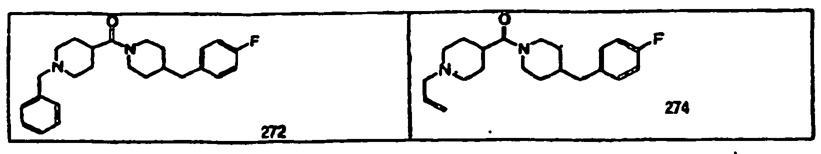

2. A composition comprising a compound according to claim 1 and a pharmaceutically acceptable carrier.

3. The composition according to claim 2, additionally comprising a neurotrophic factor.

4. The composition according to claim 3, wherein said neurotrophic factor is selected from nerve growth factor (NGF), insulin-like growth factor (IGF-1), gIGF-1, Des(1-3)IGF-I, acidic and basic fibroblast growth factor (aFGF and bFGF, respectively), platelet-derived growth factors (PDGF), brain-derived neurotrophic factor (BDNF), ciliary neurotrophic factors (CNTF), glial cell line-derived neurotrophic factor (GDNF), neurotrophin-3 (NT-3)and neurotrophin 4/5 (NT-4/5).

5. The composition according to claim 2, wherein said composition is formulated for oral or parenteral administration to a patient.

6. The composition according to claim 3, wherein said composition is formulated for oral or parenteral administration to a patient.

7. A use of a compound according to claim 1 for the manufacture of a medicament for promoting neuronal repair or preventing neuronal damage in a patient or in an ex *vivo* nerve cell.

8. The use according to claim 7 for promoting neuronal repair or preventing neuronal damage in a patient or in an ex vivo nerve cell, glial cell, chromaffin cell or stem cell.

9. The use according to claim 8, wherein said medicament additionally comprises a neurotrophic factor.

10. The use according to claim 9, wherein said neurotrophic factor is selected from nerve growth factor (NGF), insulin-like growth factor (IGF-1), gIGF-1, Des(1-3)IGF-I, acidic and basic fibroblast growth factor (aFGF and bFGF, respectively), platelet-derived growth factors (PDGF), brain-derived neurotrophic factor (BDNF), ciliary neurotrophic factors (CNTF), glial cell line-derived neurotrophic factor (GDNF), neurotrophin-3 (NT-3) and neurotrophin 4/5 (NT-4/5).

11. The use according to claim 7, wherein said medicament is used to treat a patient suffering from a disease selected from trigeminal neuralgia, glossopharyngeal neuralgia, Bell's Palsy, myasthenia gravis, muscular dystrophy, muscle injury, progressive muscular atrophy, progressive bulbar inherited muscular atrophy, herniated, ruptured, or prolapsed intervertebrae disk syndromes, cervical spondylosis, plexus disorders, thoracic outlet destruction syndromes, peripheral neuropathies, such as those caused by lead, dapsone, ticks, or porphyria, other peripheral myelin disorders, Alzheimer's disease, Guillain-Barre syndrome, Parkinson's disease and other Parkinsonian disorders, ALS, Tourette's syndrome, multiple sclerosis, other central myelin disorders, stroke and ischemia associated with stroke, neural paropathy, other neural degenerative diseases, motor neuron diseases, sciatic injury, neuropathy associated with diabetes, spinal cord injuries, facial nerve injury and other trauma, chemotherapy- and other medication-induced neuropathies, Huntington's disease, and protein fibrillization diseases, such as Diffuse Lewy Body disease, Alzheimer's disease-Lewy Body variant, Famillal British Dementia, and Frontotemporal Dementia.

## Patentansprüche

1. Verbindung, ausgewählt aus einer der Verbindungen:

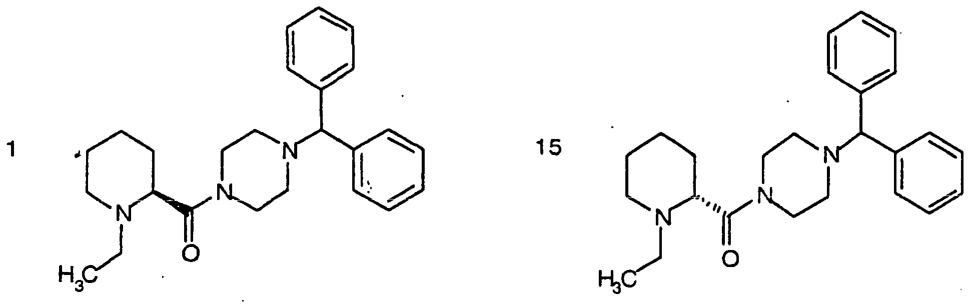

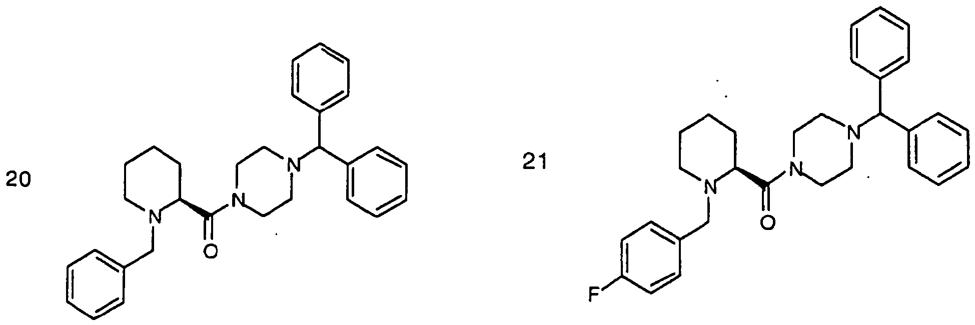

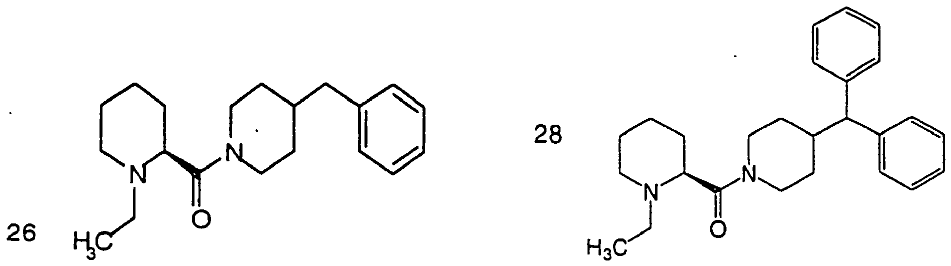

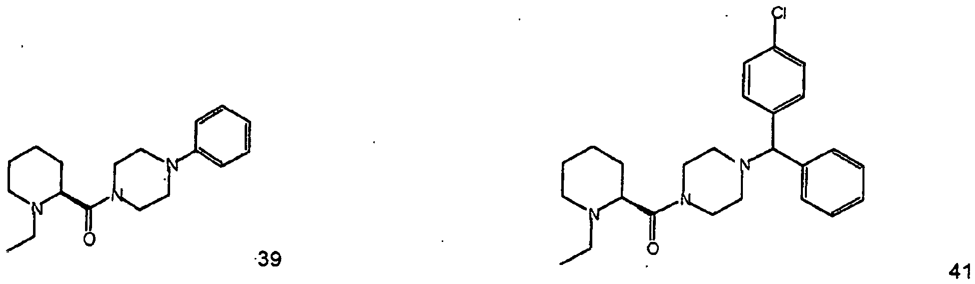

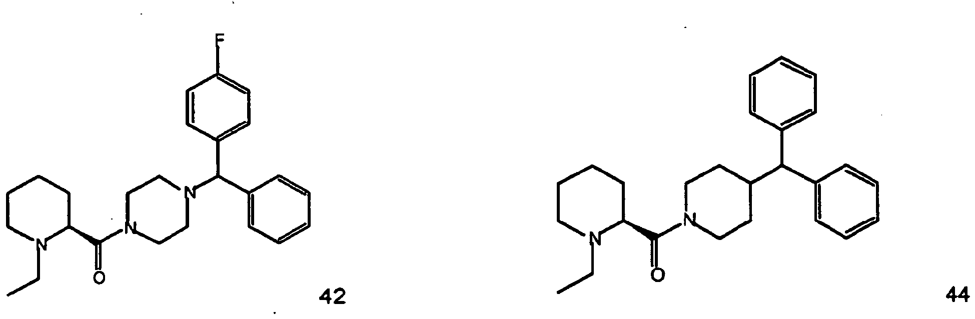

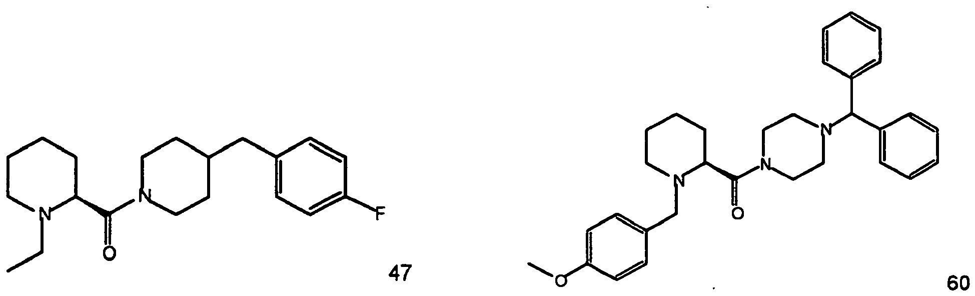

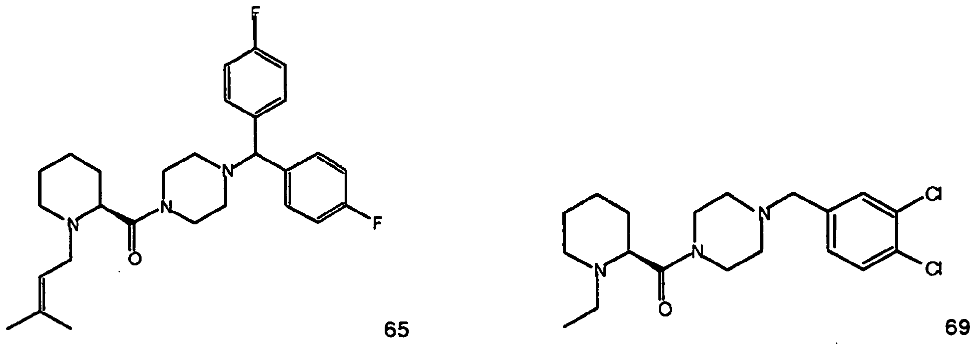

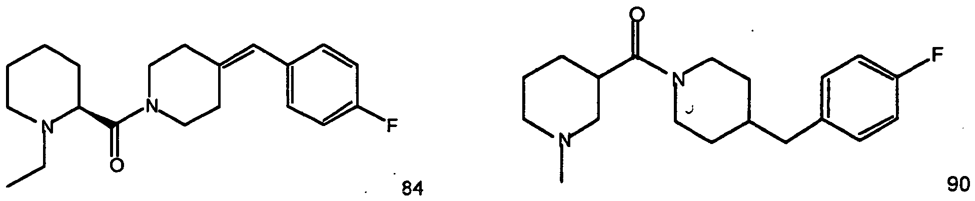

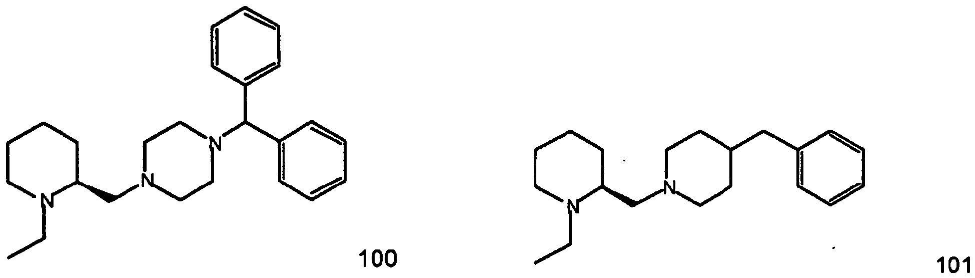

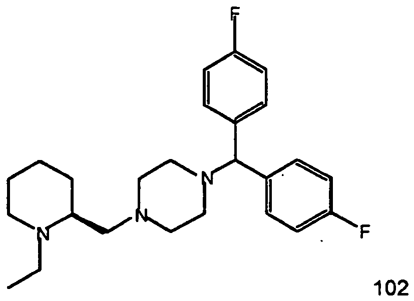

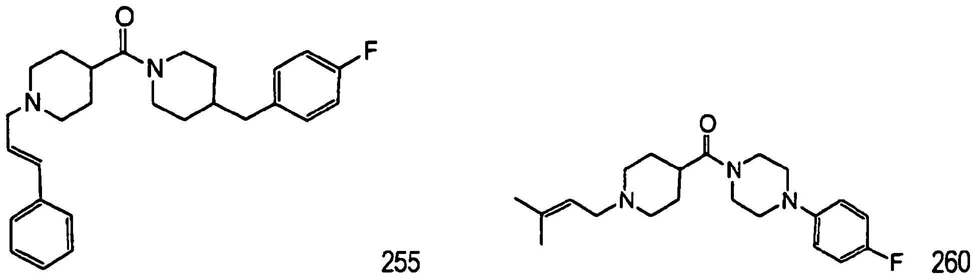

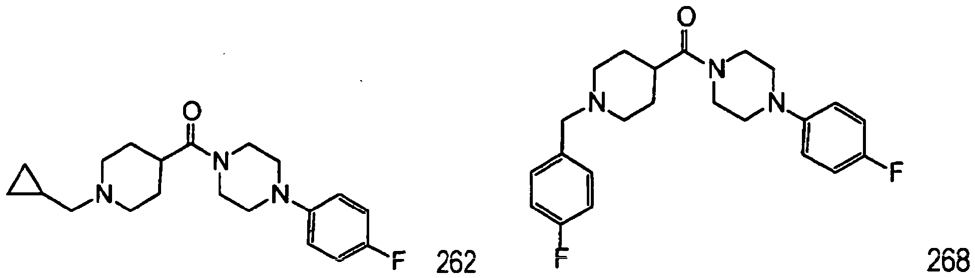

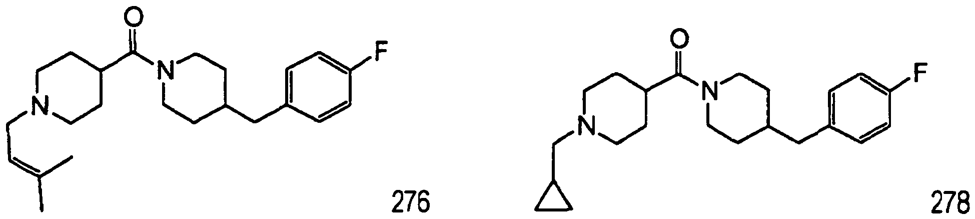

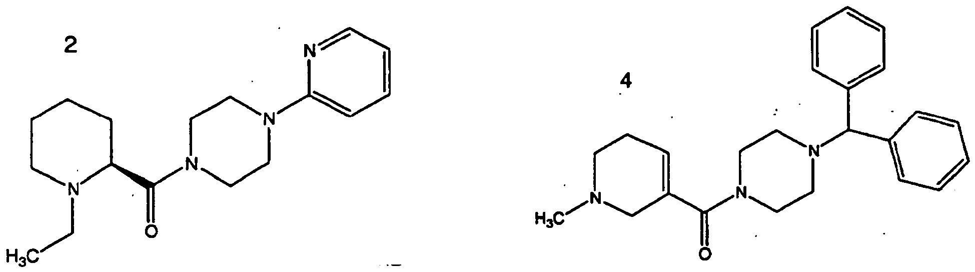

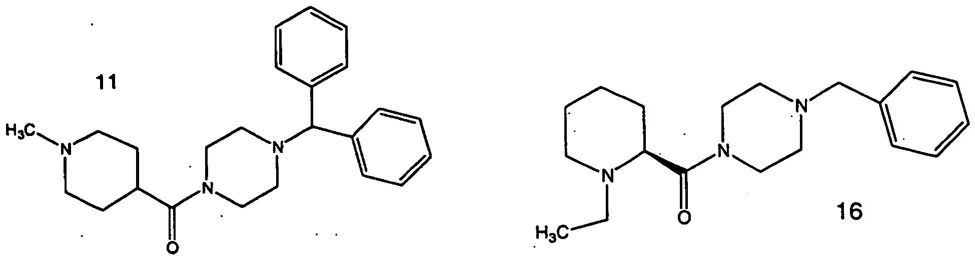

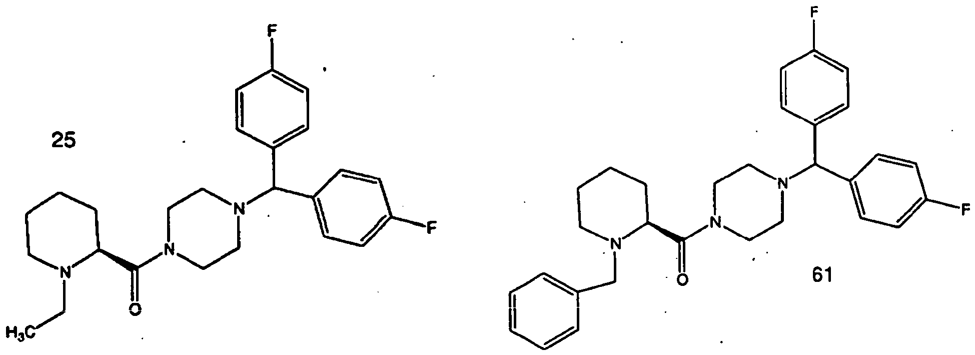

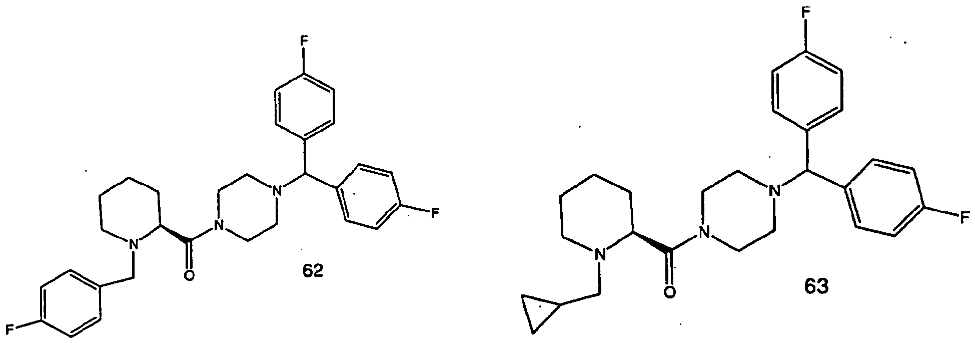

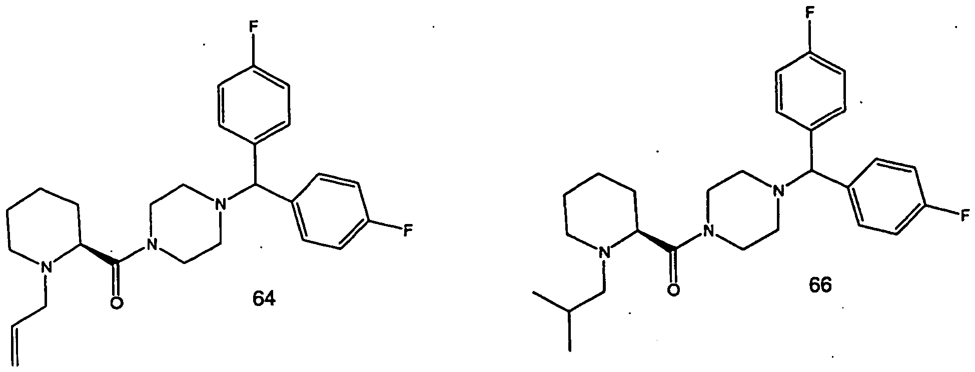

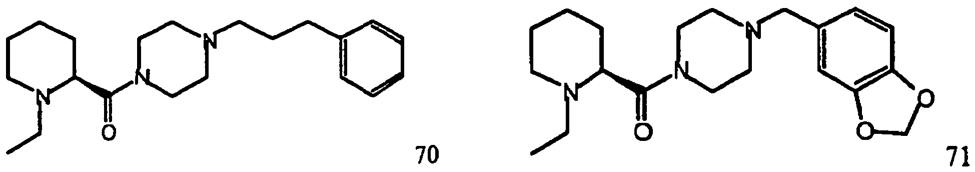

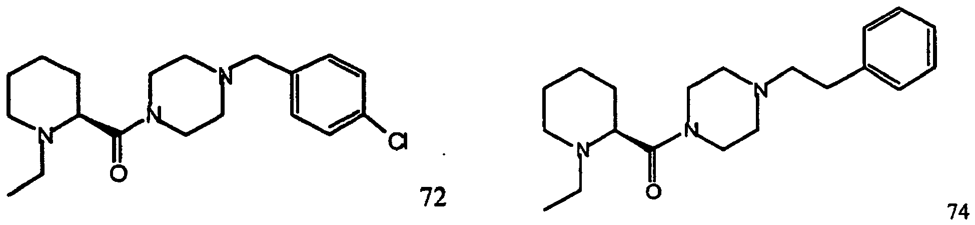

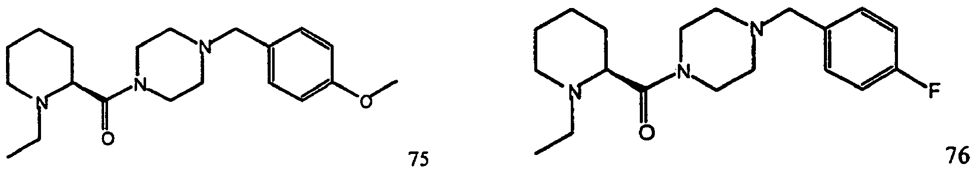

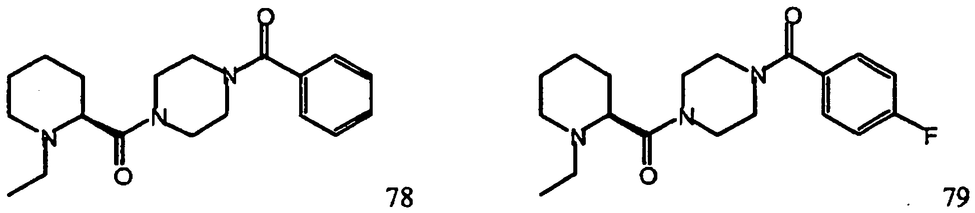

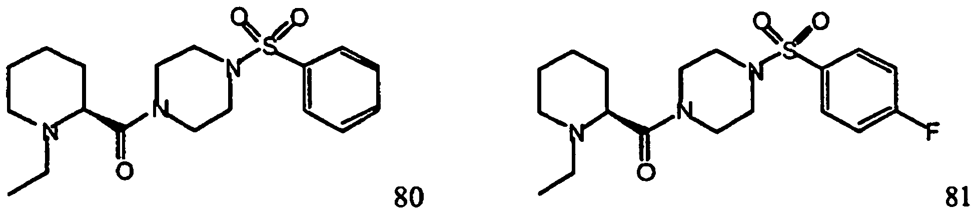

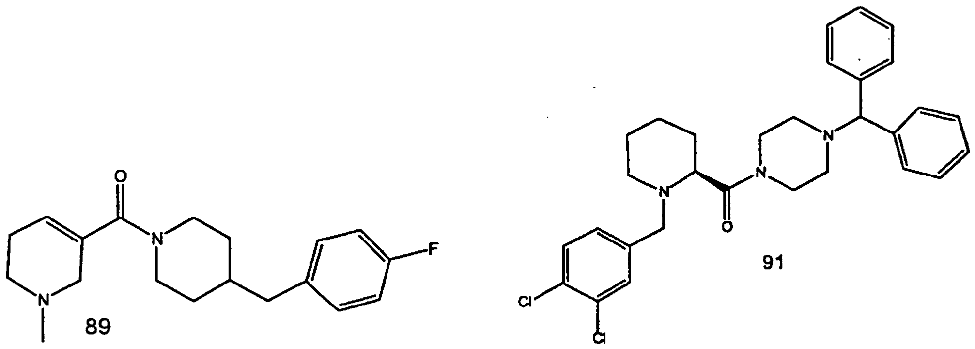

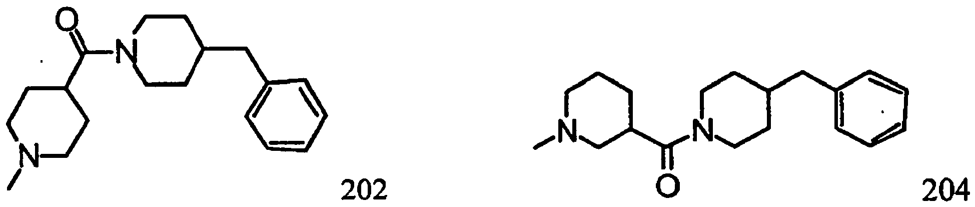

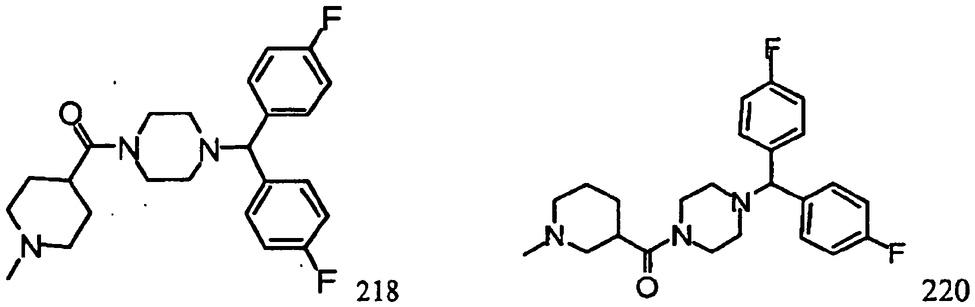

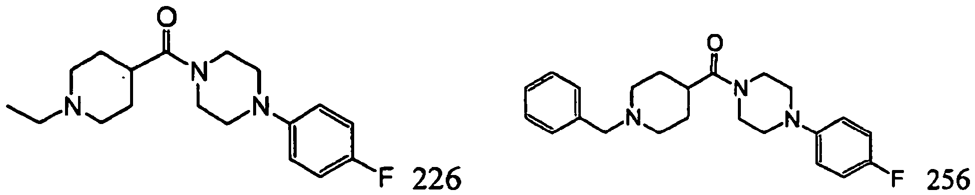

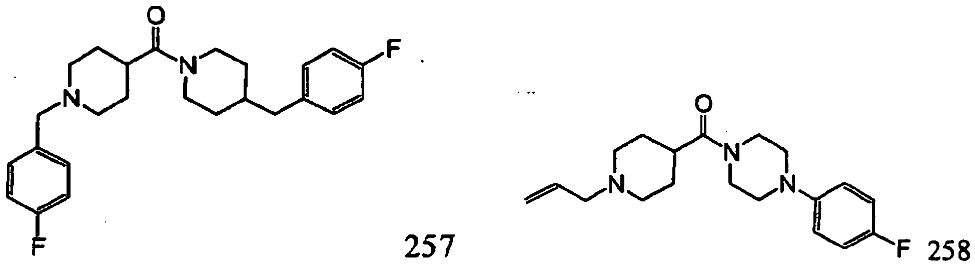

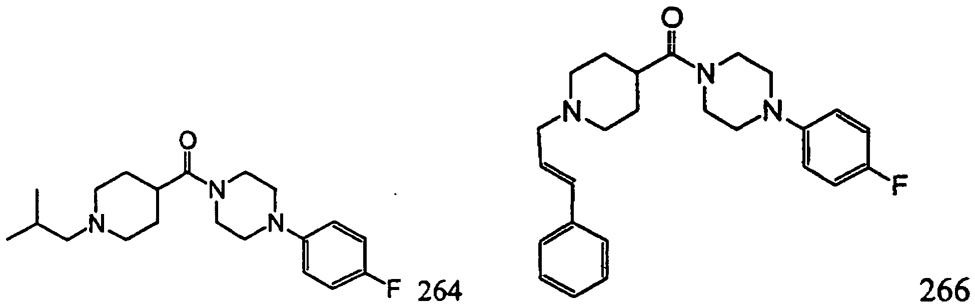

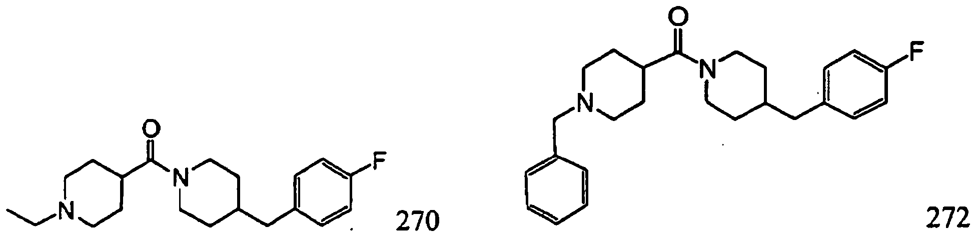

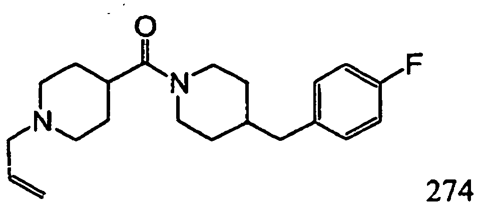

2. Zusammensetzung, die eine Verbindung gemäß Anspruch 1 und einen pharmazeutisch verträglichen Träger umfasst.

3. Zusammensetzung gemäß Anspruch 2, die ferner einen neurotrophen Faktor umfasst.

4. Zusammensetzung gemäß Anspruch 3, wobei der neurotrophe Faktor ausgewählt ist aus Nervenwachstumsfaktor (NGF), insulinartigem Wachstumsfaktor (IGF-1), gIGF-1, Des(1-3)IGF-I, saurem und basischem Fibroblasten-Wachstumsfaktor (aFGF bzw. bFGF), Plättchenwachstumsfaktoren (PDGF), aus Gehirn stammendem neurotrophem Faktor (BDNF), ciliaren neurotrophen Faktoren (CNTF), aus Glia-Zelllinie stammendem neurotrophem Faktor (GDNF), Neurotrophin-3 (NT-3) und Neurotrophin 4/5 (NT-4/5).

5. Zusammensetzung gemäß Anspruch 2, wobei die Zusammensetzung für orale oder parenterale Verabreichung an einen Patienten formuliert ist.

6. Zusammensetzung gemäß Anspruch 3, wobei die Zusammensetzung für orale oder parenterale Verabreichung an einen Patienten formuliert ist.

7. Verwendung einer Verbindung gemäß Anspruch 1 für die Herstellung eines Medikaments zur Förderung neuronaler Regeneration oder Vorbeugung neuronaler Schäden bei einem Patienten oder bei einer ex vivo-Nervenzelle.

8. Verwendung gemäß Anspruch 7 zur Förderung neuronaler Regeneration oder Vorbeugung neuronaler Schäden bei einem Patienten oder bei einer ex vivo-Nervenzelle, Gliazelle, Chromaffinzelle oder Stammzelle.

9. Verwendung gemäß Anspruch 8, wobei das Medikament ferner einen neurotrophen Faktor umfasst.

10. Verwendung gemäß Anspruch 9, wobei der neurotrophe Faktor ausgewählt ist aus Nervenwachstumsfaktor (NGF), insulinartigem Wachstumsfaktor (IGF-1), gIGF-1, Des(1-3)IGF-I, saurem und basischem Fibroblasten-Wachstumsfaktor (aFGF bzw. bFGF), Plättchenwachstumsfaktoren (PDGF), aus Gehirn stammendem neurotrophem Faktor (BDNF), ciliaren neurotrophen Faktoren (CNTF), aus Glia-Zelllinie stammendem neurotrophem Faktor (GDNF), Neurotrophin-3 (NT-3) und Neurotrophin 4/5 (NT-4/5).

11. Verwendung gemäß Anspruch 7, wobei das Medikament zur Behandlung eines Patienten verwendet wird, der an einer Krankheit leidet, ausgewählt aus Trigeminusneuralgie, Glossopharyngeusneuralgie, Bell-Lähmung, Myasthenia gravis, Muskeldystrophie, Muskelverletzung, progressivem Muskelschwund, progressivem bulbärem vererbtem Muskelschwund, bruchbildenden, gerissenen oder prolabierten Zwischenwirbelscheibensyndromen, cervicaler Spondylose, Plexusstörungen, Thoracicoutlet-Zerstörungssyndromen, peripheren Neuropathien wie denjenigen, die durch Blei, Dapson, Zecken oder Porphyrie verursacht werden, anderen peripheren Myelinstörungen, Alzheimer-Krankheit, Guillain-Barre-Syndrom, Parkinson-Krankheit und anderen Parkinson'schen Störungen, ALS, Tourette-Syndrom, Multipler Sklerose, anderen Zentralmyelinstörungen, Schlaganfall und Ischemie im Zusammenhang mit Schlaganfall, neuraler Paropathie, anderen neuralen degenerativen Krankheiten, Motoneuron-Krankheiten, Ischiasverletzung, Neuropathie im Zusammenhang mit Diabetes, Rückenmarksverletzungen, Gesichtsnervverletzung und anderem Trauma, Chemotherapie- und anderen Medikamenten-induzierten Neuropathien, Chorea Huntington und Proteinfibrillisierungskrankheiten wie diffuser Lewy-Körperchen-Krankheit, Alzheimer-Krankheit vom Lewy-Körperchen-Typ, Familiärer Britischer Demenz und Frontotemporaler Demenz.

## Revendications

1. Composé choisi parmi l'un quelconque des composés :

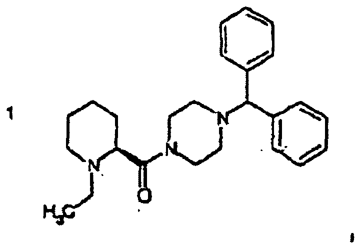

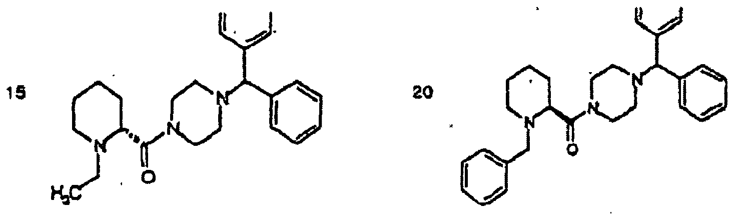

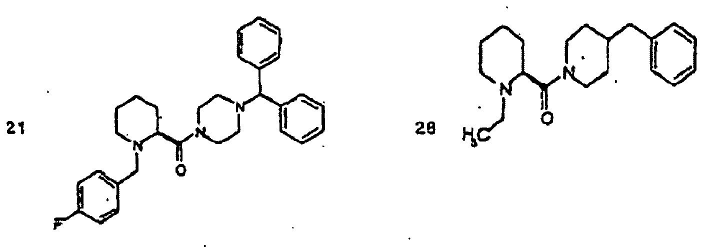

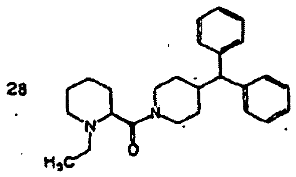

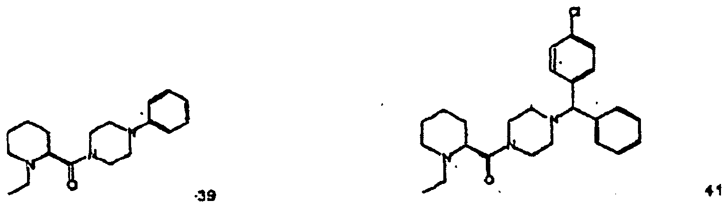

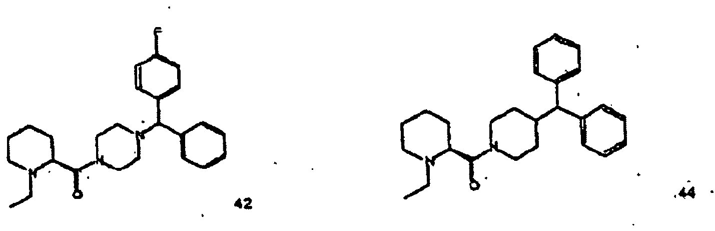

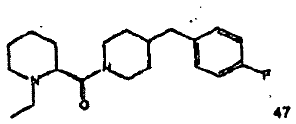

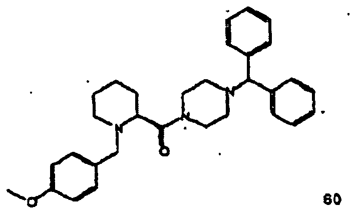

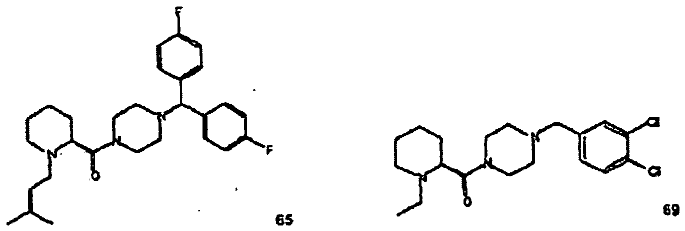

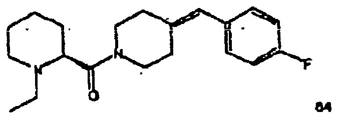

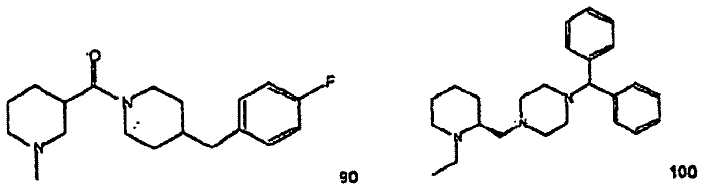

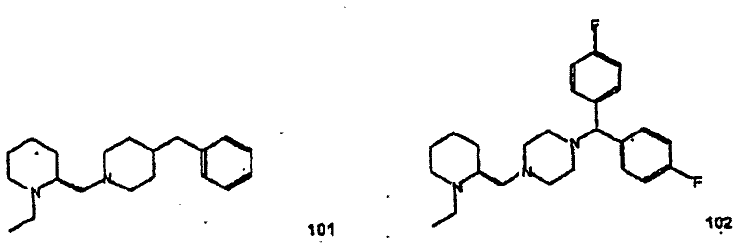

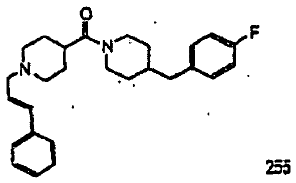

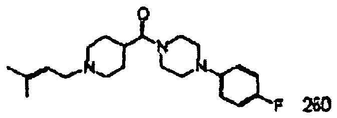

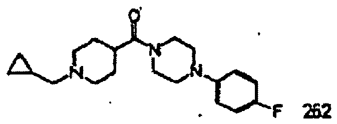

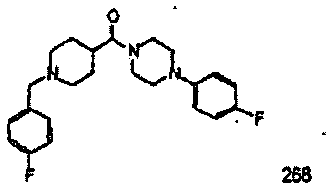

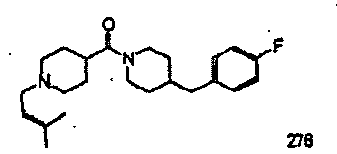

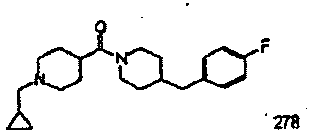

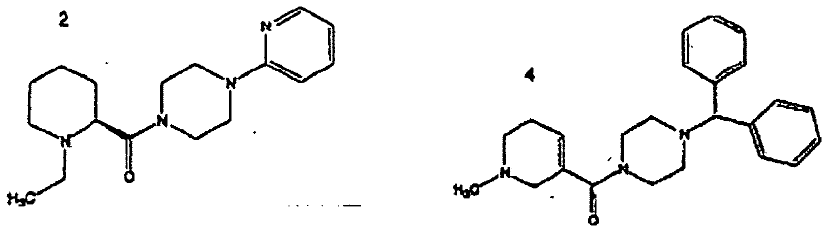

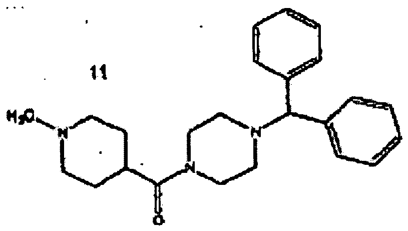

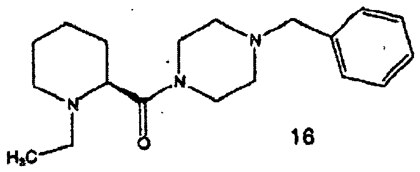

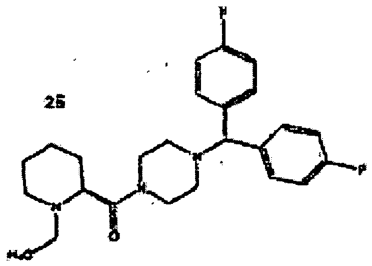

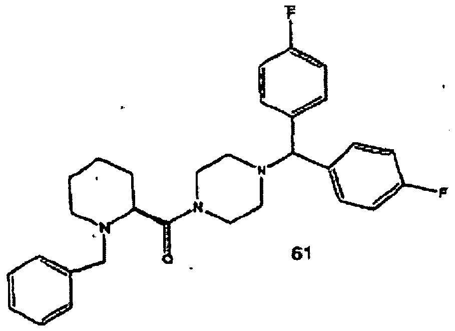

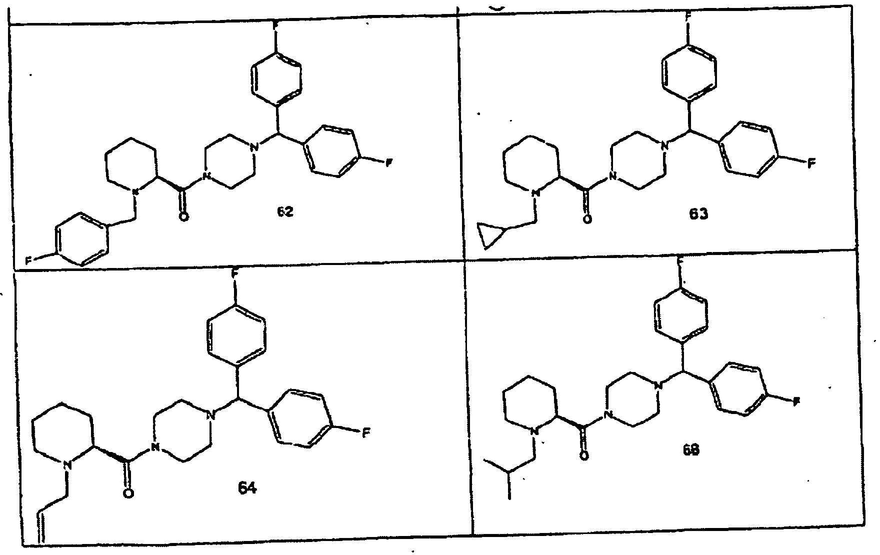

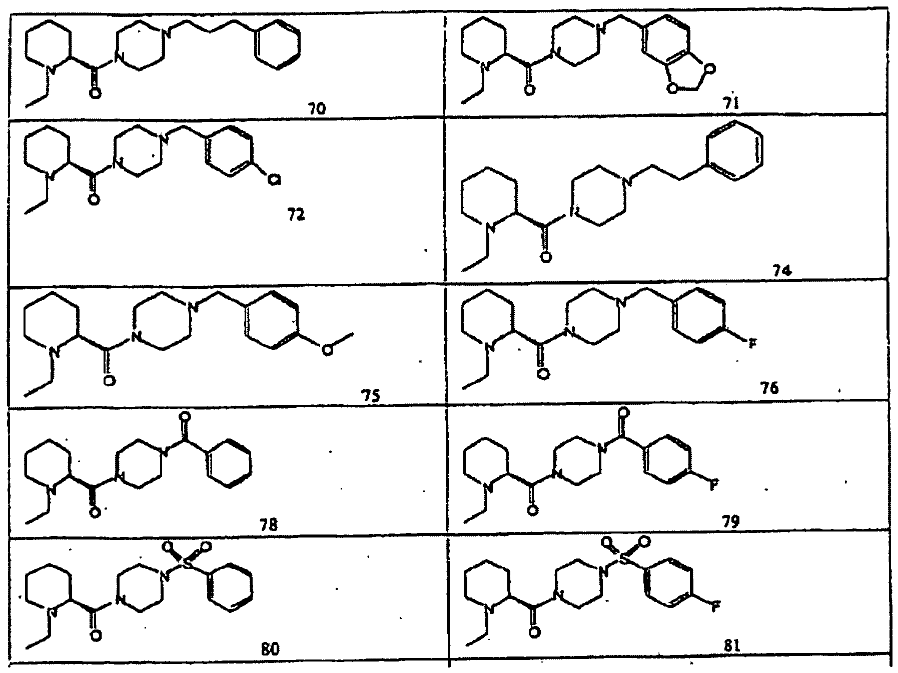

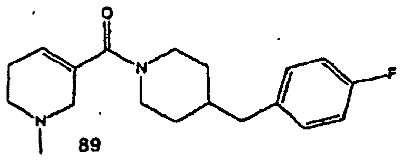

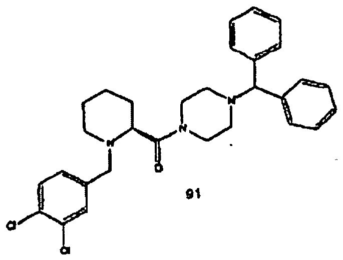

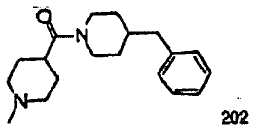

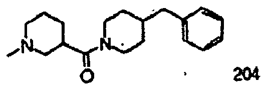

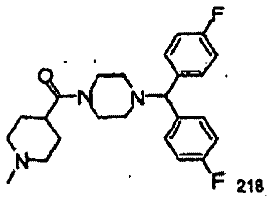

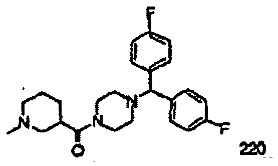

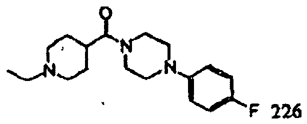

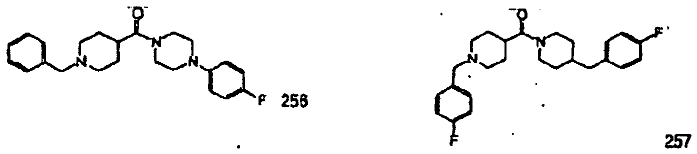

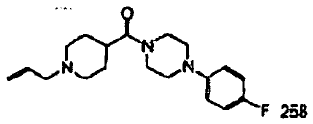

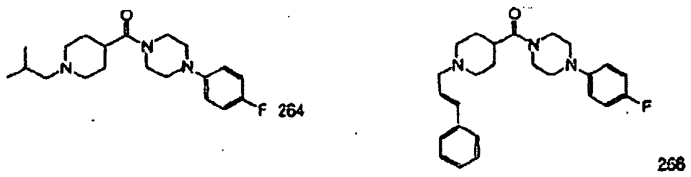

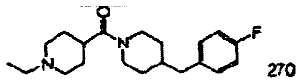

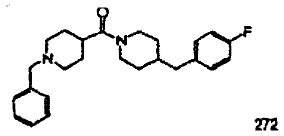

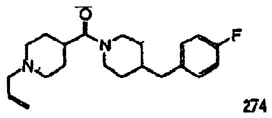

2. Composition comprenant un composé selon la revendication 1 et un véhicule pharmaceutiquement acceptable.

3. Composition selon la revendication 2 comprenant, en plus, un facteur neurotrophique.

4. Composition selon la revendication 3, dans laquelle ledit facteur neurotrophique est choisi parmi le facteur de croissance nerveuse (NGF), l'hormone de croissance semblable à l'insuline (IGF-1), gIGF-1, Des(1-3)IGF-I, le facteur de croissance fibroblastique acide et basique (aFGF et bFGF, respectivement), les facteurs de croissance dérivés des plaquettes (PDGF), le facteur neurotrophique dérivé du cerveau (BDNF), les facteurs neurotrophiques ciliaires (CNTF), le facteur neurotrophique dérivé de la lignée cellulaire gliale (GDNF), la neurotrophine-3 (NT-3) et la neurotrophine 4/5 (NT-4/5).

5. Composition selon la revendication 2, dans laquelle ladite composition est formulée pour une administration par voie orale ou parentérale à un patient.

6. Composition selon la revendication 3, dans laquelle ladite composition est formulée pour une administration par voie orale ou parentérale à un patient.

7. Utilisation d'un composé selon la revendication 1 pour la fabrication d'un médicament destiné à promouvoir la réparation neuronale ou à prévenir les lésions neuronales chez un patient ou dans une cellule nerveuse *ex vivo.*

8. Utilisation selon la revendication 7 pour promouvoir la réparation neuronale ou prévenir les lésions neuronales chez un patient ou dans une cellule nerveuse, une cellule gliale, une cellule chromaffine ou une cellule souche *ex vivo.*

9. Utilisation selon la revendication 8, dans laquelle ledit médicament comprend, en plus, un facteur neurotrophique.

10. Utilisation selon la revendication 9, dans laquelle ledit facteur neurotrophique est choisi parmi le facteur de croissance nerveuse (NGF), le facteur de croissance semblable à l'insuline (IGF-1), gIGF-1, Des(1-3)IGF-I, le facteur de croissance fibroblastique acide et basique (aFGF et bFGF, respectivement), les facteurs de croissance dérivés des plaquettes (PDGF), le facteur neurotrophique dérivé du cerveau (BDNF), les facteurs neurotrophiques ciliaires (CNTF), le facteur neurotrophique dérivé de la lignée cellulaire gliale (GDNF), la neurotrophine-3 (NT-3) et la neurotrophine 4/5 (NT-4/5).

11. Utilisation selon la revendication 7, dans laquelle ledit médicament est utilisé pour traiter un patient souffrant d'une maladie choisie parmi la névralgie du trijumeau, la névralgie du glosso-pharyngien, la paralysie de Bell, la myasthénie grave, la dystrophie musculaire, la lésion musculaire, l'atrophie musculaire progressive, l'atrophie musculaire bulbaire progressive héréditaire, les syndromes des disques intervertébraux herniés, rompus, ou prolabés, la spondylose cervicale, les atteintes du plexus, les syndromes de la traversée thoraco-brachiale, les neuropathies périphériques, telles que celles provoquées par le plomb, la dapsone, les tiques, ou la porphyrie, ou autres atteintes myéliniques périphériques, la maladie d'Alzheimer, le syndrome de Guillain-Barre, la maladie de Parkinson et autres atteintes parkinsoniennes, la SLA, le syndrome de Tourette, la sclérose en plaques, autres atteintes myéliniques centrales, l'attaque et l'ischémie associée à l'attaque, la paropathie neurale ou autres maladies dégénératives neurales, les maladies des neurones moteurs, la lésion sciatique, la neuropathie associée au diabète, les lésions de la moelle épinière, la lésion du nerf facial et autres traumatismes, les neuropathies induites par la chimiothérapie et autres médications, la maladie de Huntington, et les maladies **caractérisées par** une fibrillation protéique, telles que la maladie diffuse à corps de Lewy, la variante de la maladie d'Alzheimer à corps de Lewy, la démence britannique familiale, et la démence fronto-temporale.
